## Europäisches Patentamt

## European Patent Office

## Office européen des brevets

(11) Veröffentlichungsnummer: **0 564 801 A1**

## EUROPÄISCHE PATENTANMELDUNG

(21) Anmeldenummer: **93103159.5**

(22) Anmeldetag: **27.02.93**

(51) Int. Cl.5: **C12Q 1/70**, C07K 15/00, C12N 9/50

(30) Priorität: **04.03.92 DE 4206769**
**30.05.92 DE 4217929**

(43) Veröffentlichungstag der Anmeldung:
**13.10.93 Patentblatt 93/41**

(84) Benannte Vertragsstaaten:
**AT BE CH DE DK ES FR GB GR IE IT LI LU NL PT SE**

(71) Anmelder: **Boehringer Ingelheim International GmbH**

**D-55216 Ingelheim(DE)**

(72) Erfinder: **Sommergruber, Wolfgang, Dr.**
**Stoesselgasse 6**
**A-1130 Wien(AT)**
Erfinder: **Auer, Herbert**
**Währinger Gürtel 82/83**
**A-1090 Wien(AT)**
Erfinder: **Blaas, Dieter, Dr.**
**Liechtensteinstrasse 119/13**
**A-1090 Wien(AT)**
Erfinder: **Frasel, Lela**
**Obere Donaustrasse 5**
**A-1020 Wien(AT)**
Erfinder: **Hartmuth, Klaus, Dr.**
**Treustrasse 5/29**
**A-1200 Wien(AT)**
Erfinder: **Küchler, Ernst, Prof. Dr.**
**Görgengasse 23/6/26**
**A-1190 Wien(AT)**
Erfinder: **Kowalski, Heinrich**
**Andergasse 12-22/14/12**
**A-1140 Wien(AT)**
Erfinder: **Liebig, Hans-Dieter, Dr.**
**Dörfelgasse 3A/21**
**A-1120 Wien(AT)**
Erfinder: **Skern, Timothy, Dr.**
**Burgenlandstrasse 6**
**A-2410 Hainburg an der Donau(AT)**
Erfinder: **Ziegler, geb. Stadler, Elisabeth**
**Hans Sachs Gasse 4/7**
**A-1180 Wien(AT)**

(54) **Analyse des "host cell shut-offs".**

(57) Die Erfindung umfaßt ein Verfahren zur Analyse des durch Picornaviren verursachten "host cell shut-offs" sowie die Verwendung dieses Verfahrens zur Isolierung von Inhibitoren gegen die am "host cell shut-off" beteiligten zellulären und viralen Proteine, insbesondere gegen die 2A Proteinasen.

Eine Infektion durch die meisten Vertreter der Picornaviren führt sehr bald zu einer drastischen Reduktion der zellulären Proteinbiosynthese trotz Anwesenheit intakter, endogener messenger RNA (mRNA). Dieses als "host cell shut-off" bekannte Phänomen ermöglicht dem Virus eine effiziente Synthese der genomischen RNA und der viralen Proteine (Rueckert R. R. (1990) "Picornaviridae and their Replication", in Fields, B. N. (Hrsg.): "Virology", 2. Auflage, Bd. 1-2, 507 - 548).

Der molekulare Mechanismus, der zum "host cell shut-off" führt, ist noch nicht genau bekannt. Im Verlauf der Infektion wird eine Spaltung des Proteins p220 (eIF-4γ) beobachtet, einer Komponente des eukaryotischen Initiationsfaktors eIF-4F, die für die Ausbildung eines Cap-abhängigen Initiationskomplexes notwendig erscheint (Sonenberg, N. (1987) Adv. Virus Res. 33, 175-204). Da die zellulären mRNAs eine charakteristische Cap-Struktur besitzen, wird dadurch deren Translation unterbunden. Picornaviren enthalten hingegen kein Cap, sondern verfügen über eine funktionell konservierte Region in der 5' nicht translatierten Region, die eine Cap-unabhängige Translation ermöglicht (Pelletier, J. und Sonenberg, N. (1988), Nature 334, 320 - 325; Kühn et al. (1990), J. Virol. 64, 4625-4631).

Die Spaltung von p220 wird bei Entero- und Rhinoviren möglicherweise indirekt durch die virale Proteinase 2A und bei Aphtoviren durch das Leaderprotease-Paar L/L' hervorgerufen (Kräusslich, H.-G., Nicklin, M. J. H., Toyoda, H., Etchinson, D. und Wimmer, E. (1987) J. Virol. 61, 2711 - 2718; Lloyd, R. E., Toyoda, H., Etchinson, E., Wimmer, E. und Ehrenfeld, E. (1986) Virology 150, 299 - 303; Belsham, G. J. und Brangwyn, J. K. (1990) J. Virol. 64, 5389 - 5395; Devaney et al. (1989) J. Virol. 62, 4407-4409). Eine Reihe experimenteller Ergebnisse spricht dafür, daß durch diese äußerst spezifischen viralen Proteinasen eine latent in der infizierten Zelle vorliegende, endogene Proteinase proteolytisch aktiviert wird, die ihrerseits die Spaltung von p220 bewirkt (Kräusslich et al., loc. cit.; Lloyd et al., loc. cit.; Hellen, C. U. T., Kräusslich, H.-G. und Wimmer, E. (1989) Biochem. 28, 9881 - 9890). Diese Aktivierung ist jedoch noch nicht bewiesen, da es bislang nicht gelungen ist, die zelluläre Proteinase zu identifizieren. Es konnte jedoch gezeigt werden, daß der eukaryotische Initiationsfaktor eIF-3 ein notwendiger Bestandteil dieser p220 Spaltungsaktivität ist (Wyckoff, E., Hershey, J.W.B. und Ehrenfeld, E. (1990) Proc. Natl. Acad. Sci. U.S.A. 87, 9529 - 9523).

Beobachtungen anderer Arbeitsgruppen zeigen jedoch keinen zeitlich kausalen Zusammenhang zwischen der Spaltung von p220 und dem Auftreten des "host cell shut-offs", was dessen Relevanz als auslösende Ursache dieses Phänomens zur Zeit noch kontrovers erscheinen läßt (Urzainqui and Carrasco (1989) J. Virol. 63, 4729-4735).

Die vorliegende Erfindung stellt nun ein in vitro Verfahren zur Verfügung, das erlaubt, den molekularen Mechanismus des durch Picornaviren verursachten "host cell shut-off" zu analysieren.

Das erfindungsgemäße Verfahren ist dadurch gekennzeichnet, daß eine RNA abgeleitet von einem Gen mit mindestens einem internen Translationsstart mit einer Cap-Struktur versehen wird. Diese RNA wird mittels einer Translationsmischung, die mit einer picornaviralen Proteinase vorinkubiert ist, in vitro translatiert. Durch Vergleich des erhaltenen Proteinmusters mit entsprechenden Proteinmustern der Translationsprodukte der entsprechenden RNAs ohne Cap-Struktur und/oder ohne Vorinkubation der Translationsmischung mit einer picornaviralen Proteinase kann der "host cell shut-off" analysiert werden.

Die vorliegende Erfindung umfaßt weiterhin ein Verfahren zur Herstellung der für das Analyseverfahren benötigten picornaviralen Proteinasen sowie Verfahren zu deren Reinigung.

Neben der Charakterisierung der am "host cell shut-off" beteiligten Substanzen kann das erfindungsgemäße Verfahren zur Identifizierung von Inhibitoren des "host cell shut-offs" verwendet werden.

Im folgenden wird die Erfindung detailliert erläutert.

Überraschenderweise wurde gefunden, daß die Einwirkung von picornaviralen 2A Proteinasen auf in vitro Translationsmischungen zu einer Erhöhung der Translationsinitiation an internen Startcodons (AUG) von RNA-Molekülen mit Cap-Struktur führt.

Ein Aspekt der vorliegenden Erfindung umfaßt deshalb ein Verfahren zur Analyse des "host cell shut-offs" mit folgenden Teilschritten:

(a) In vitro Herstellung einer RNA mit Cap-Struktur abgeleitet von einem Gen mit mindestens einem internen Translationsstart;

(b) Inkubation einer in vitro Translationsmischung mit einer den "host cell shut-off" auslösenden picornaviralen Proteinase, insbesondere einer rhinoviralen 2A Proteinase;

(c) Inkubation der unter Punkt (a) hergestellten RNA mit der unter Punkt (b) hergestellten Translationsmischung;

(d) Analyse der Translationsprodukte.

Für das erfindungsgemäße Verfahren kann zur Herstellung der entsprechenden RNA durch in vitro Transkription jedes Gen verwendet werden, das einen Leserahmen mit Start- und Stopcodon besitzt und dessen RNA zusätzlich mindestens ein internes Translationsstartcodon (AUG) aufweist. Dabei bezeichnet der Begriff "Gen" ein beliebiges DNA-Fragment, das Informationen zur Synthese eines Proteins enthält bzw. für ein Protein codiert. Zweckmäßigerweise sind ein oder mehrere zusätzliche Translationsinitiationsstellen so angeordnet, daß sowohl das erste Startcodon als auch alle zusätzlichen Startcodons solche Proteine bzw. Peptide liefern, die z.B. durch ihr unterschiedliches Molekulargewicht einzeln nachgewiesen werden können. In Beispiel 1 ist die Konstruktion eines geeigneten Gens anhand eines picornaviralen 2C Gens (HRV2 2C Gen) ausführlich beschrieben.

Die Sequenzen picornaviraler Gene sowie die Methoden zu ihrer Herstellung sind bekannt und z.B. von Stanway et al. (1984) Nucl. Acids Res. 12, 7859-7875 für das humane Rhinovirus HRV14 und von Skern et al. (1985) Nucl. Acids Res. 13, 2111-2126 für das humane Rhinovirus HRV2 beschrieben. Die entsprechenden Viren als Ausgangsstoffe zur Isolierung der Gene sind z.B. von der American Type Culture Collection (Rockville, Maryland, USA) erhältlich. Die benötigten Genabschnitte können auf Grundlage der bekannten Sequenzen natürlich auch synthetisch hergestellt werden (z. B. nach Edge et al. (1981) Nature 292, 756-762).

Alle für die Konstruktion eines geeigneten Gens notwendigen Methoden wie z.B. der die Insertion von Start- und Stopcodons sowie interner Translationsinitiationsstellen sind dem Fachmann bekannt und z.B. im Sambrook et al., loc. cit. beschrieben. Dabei kann die Sequenz vor dem ersten Start-AUG vorteilhafterweise so gewählt werden, daß es die höchstmögliche Übereinstimmung mit der Konsensussequenz von Kozak besitzt (Kozak, M. (1989) J. Cell. Biol. 108, 229-241).

Für das in Beispiel 1 verwandte 2C Gen müssen Start- und Stopcodon ergänzt werden. Im HRV2 2C-Gen, das nach Skern et al. (1985 loc. cit.) isoliert werden kann, liegen interne Translationsinitiationsstellen bereits vor.

Zur in vitro Transkription, d. h. zur Herstellung der entsprechenden RNA, wird das wie oben beschrieben vorbereitete Gen in einen Transkriptionsvektor kloniert. Damit wird das Gen unter die Kontrolle eines RNA-Polymerase Promotors gebracht. Nach in vitro Transkription kann die entsprechende RNA im Mikrogrammbereich erhalten werden. Bevorzugte Vektorsysteme arbeiten mit SP6-, T3- und T7-Promotoren. Zum Beispiel kann das Plasmid pGEM2 (Promega) eingesetzt werden.

Die in vitro Transkription der entsprechenden DNA erfolgt nach Standardmethoden, wie z.B. bei Duechler et al. (1989) Virology 168, 159 - 161, Sambrook et al. (1989) loc. cit., Krieg, P. A. und Melton, D. A. (1987) Meth. Enzymol., 155, 397 - 415 oder Bujard, H. et al. (1987) Meth. Enzymol. 155, 416 - 433 beschrieben.

Ein in vitro Transkriptionsansatz kann z.B. enthalten: 20 mM Natriumphosphat, pH 7,7; 8 mM $MgCl_2$, 10 mM DTT, 4 mM Spermidin-HCl, 50 mM NaCl und 0,4 bis 1 mM ATP, CTP, GTP und UTP. Die Template-DNA (20 $\mu$g/ml) wird mit einem geeigneten Restriktionsenzym linearisiert, gefolgt von Phenolextraktion und Ethanolpräzipitation. Die Reaktion wird durch Zugabe der entsprechenden RNA Polymerase gestartet. Es wird für 30 min. bei 37°C inkubiert.

Nach der Transkriptionsreaktion kann die DNA durch Zugabe von DNase I (RNAse-frei, Boehringer Mannheim) verdaut und die RNA durch Phenolextraktion und Ethanolfällungen gereinigt werden.

Falls für die nachfolgende in vitro Translation die RNA mit einer Cap-Struktur versehen werden soll, kann zur Transkriptionsreaktion [7]mGpppG und GTP zugesetzt werden. Zum Beispiel kann der von Duechler et al. (1989) loc. cit., beschriebene Reaktionsansatz 1 mM [7]mGpppG und 250 $\mu$M GTP enthalten.

Zur in vitro Translation werden Proteinextrakte, die kommerziell bezogen oder mit Standardmethoden hergestellt werden können, eingesetzt. Zum Beispiel können Kaninchenretikulozytenlysate oder HeLa-Zellextrakte verwendet werden. Außerdem kann die Translationsmischung durch bekannte Verfahren fraktioniert werden, um die Wirkung der einzelnen Proteine bzw. Proteinmischungen auf die Translationsreaktion zu überprüfen (Sambrook, J., Fritsch, E. F., Maniatis, T. (1989): "Molecular cloning: a laboratory manual", Cold Spring Harbor Laboratory Press; Beispiel 1).

Wie in Beispiel 1 gezeigt, wird jeweils 1 $\mu$g einer RNA pro Reaktionsansatz eingesetzt. Der Reaktionsansatz enthält außerdem in 20 $\mu$l 90 mM KCl, 0,3 mM $MgCl_2$, 10 mM Kreatinphosphat, 2 $\mu$l $^{35}$S-Trans-Label (ICN; 10 $\mu$Ci/$\mu$l, 1000 Ci/mmol) und 11,5 $\mu$l Kaninchenreticulocytenlysat, das mit Micrococcal Nuclease (Sigma) behandelt worden war. Das Kaninchenreticulocytenlysat kann z.B. nach der Methode von Jackson, R. und Hunt, T. (Meth. Enzymol. (1983) 96, 50 - 74) durch Behandlung mit Micrococcal Nuclease hergestellt werden. Nach 1 Stunde bei 30°C wird die Reaktion durch Zugabe von 1 $\mu$l 200 mM Methionin gestoppt und ein Aliquot (1 $\mu$l) auf einem SDS-Polyacrylamidgel aufgetragen. Das Gel wird nach Beendigung des Laufes fluorographiert. Beispiel 4 zeigt die Verwendung eines HeLa-Zellextraktes zur in vitro Translation.

Für die Markierung der Translationsprodukte kann eine Vielzahl von Methoden verwandt werden, die sämtlich aus dem Stand der Technik bekannt sind, z.B. Einbau radioaktiver Aminosäuren, elektrophoretische Auftrennung und Fluorographie oder Detektion der aufgetrennten Translationsprodukte durch immunologische Methoden, z.B. durch Nachweis der entsprechenden Proteine bzw. seiner Fragmente durch gegen sie gerichtete monoklonale oder polyklonale Antikörper, die nach bekannten Methoden herstellbar sind (Sambrook et al. (1989) loc. cit.).

Gemäß der Erfindung wird die Translationsmischung mit dem den "host cell shut-off" auslösenden Agens, der entsprechenden picornaviralen Proteinase, vorinkubiert. Dazu kann die Proteinase in reiner oder partiell reiner Form sowie als Rohextrakt der Translationsmischung zugesetzt werden.

Herstellungsmethoden für picornavirale Proteinasen, insbesondere der rhinoviralen 2A Proteinasen sind bekannt. Zum Beispiel sind für die HRV2 2A Proteinase die Expressionsvektoren pEx2A (Skern et al. (1991) Virology 181, 46-54) und pPROK-2A (Sommergruber et al. (1992) J. Biol. Chem. 267, 22639-22644) beschrieben. Allerdings fällt nach Expression mittels pEx2A der größte Teil der Proteinase als unlösliches Material an. Die Expression mittels pPROK-2A liefert ein Gemisch aus reifer Proteinase und ein um ein zusätzliches N-terminales Methionin verlängertes Protein.

Für die Herstellung löslicher picornaviraler Proteinasen, insbesondere in größerer Menge, kann vorteilhafterweise das in den Beispielen 3 und 4 beschriebene Herstellungsverfahren genutzt werden. Hier wird zur Präparation einer löslichen, maturen, rhinoviralen Proteinase das Genfragment für den C-terminalen Anteil des VP1 Proteins sowie das vollständige Gen für die 2A Proteinase in den Vektor pET8c (Novagen, Madison, USA) oder pET3b (Novagen, Madison, USA) kloniert und in dem E. coli Stamm BL21(DE3)LysS bzw. -LysE (Novagen, Madison, USA) zur Expression gebracht.

Der VP1-Anteil kann bevorzugt die 3 C-terminalen bis 40 C-terminalen Aminosäuren umfassen, so daß nach Expression ein lösliches Fusionsprotein anfällt, das durch die autokatalytische Aktivität des picornaviralen 2A Proteinaseanteils das lösliche mature Protein freisetzt. Insbesondere eignen sich die 28 C-terminalen VP1-Aminosäuren für die Bildung des Fusionsproteins. Beispielhaft ist die Expression für die Herstellung der löslichen, maturen HRV2 2A Proteinase mittels der Plasmide pET/2A und pET8c/2A in den Beispielen 2 und 3 beschrieben. Die cDNA-Sequenzen für den C-terminalen Anteil des VP1-Proteins sowie des 2A Proteins sind bekannt und z.B. durch Stanway et al., loc. cit. und Skern et al., loc. cit. beschrieben. Sie können direkt aus den Viren isoliert oder aber auch synthetisch hergestellt werden (Edge et al., loc. cit.). Die entsprechenden Viren sind z.B. von der American Type Culture Collection (Rockville, Maryland, USA) erhältlich.

Ein Aspekt der Erfindung ist deshalb auch ein Verfahren zur rekombinanten Herstellung einer löslichen, maturen, picornaviralen 2A Proteinase, bevorzugt einer rhinoviralen 2A Protease, das durch die Klonierung des C-terminalen Anteils des picornaviralen VP1 Proteins und nachfolgend des vollständigen picornaviralen 2A Proteins in die oben genannten Vektoren gekennzeichnet ist. Nach Expression können die Zellen wie in Beispiel 3 beschrieben aufgearbeitet werden. Zum Beispiel können die Zellen durch Ultraschall lysiert und die Proteinase aus dem nach Zentrifugation erhaltenen Zellüberstand durch fraktionierte Ammoniumsulfatfällung konzentriert werden. Anschließend kann die Proteinase durch Ionenaustausch- und Gelchromatographie weiter gereinigt werden (Beispiel 3).

Daneben kann die 2A Proteinase auch direkt nach fraktionierter Ammoniumsulfatfällung eingesetzt werden (Beispiel 2).

Nach Vorinkubation der Translationsmischung mit der entsprechenden Proteinase findet die in vitro Translation unter Zusatz eines markierenden Reagenzes, zum Beispiel einer radioaktiven markierten Aminosäure wie $^{35}$S-Methionin statt. Nach Translation können die erhaltenen Proteine mit bekannten Methoden detektiert werden (Sambrook et al. (1989) loc. cit.).

Im folgenden wird das Verfahren anhand der Translation des rhinoviralen 2C Gens erläutert:

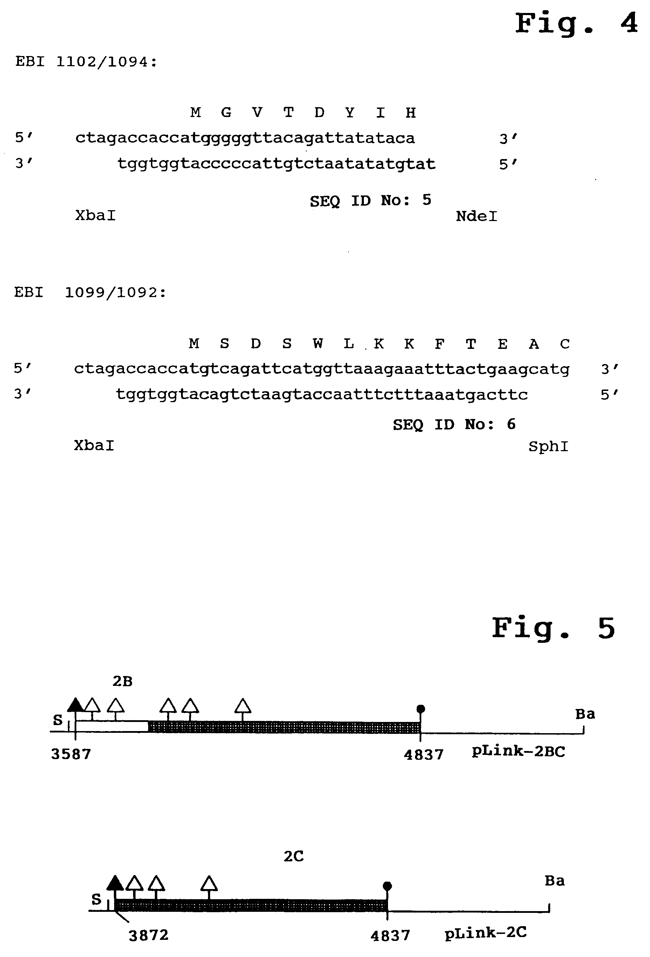

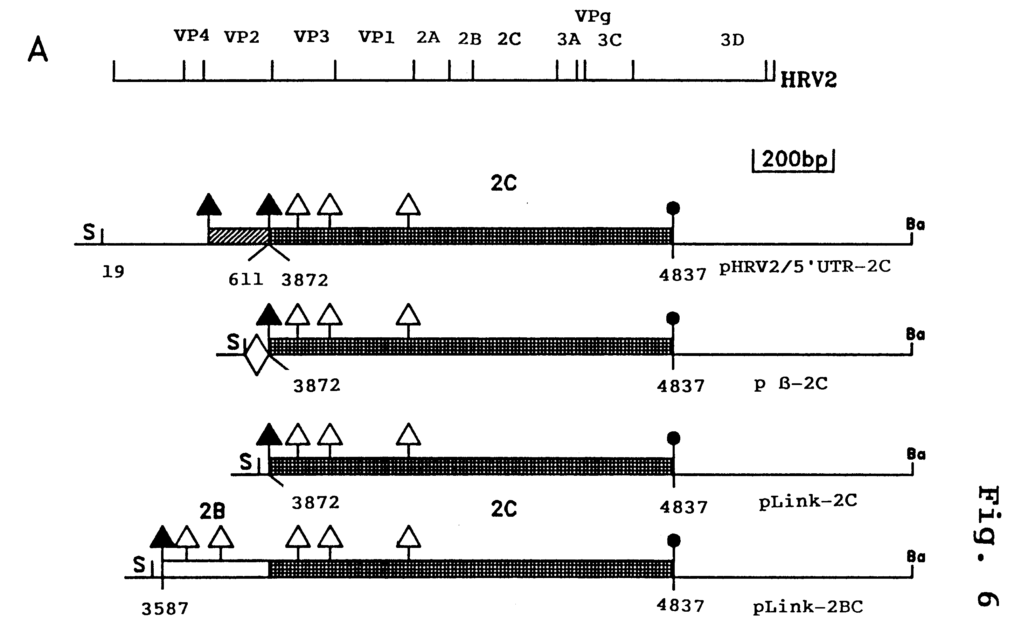

Das gemäß Beispiel 1 vorbereitete HRV2 2C-Gen (Skern et al., loc. cit.) in Plasmid pLink-2C (Fig. 5 und 6) wird nach Linearisierung mit BamH1 in vitro transkribiert. Die RNA umfaßt vom Transkriptionsstart bis zum AUG Codon 46 Nukleotide, gefolgt von den Nukleotiden 3872 bis 5219 der HRV2 Sequenz. Zur Kontrolle kann das Plasmid pLink-BC (Fig. 5 und 6, Beispiel 1) dienen, wobei nach Linearisierung mit BamH1 und der in vitro Transkription eine RNA entsteht, die von Transkriptionsbeginn bis zum Translationsstart der HRV2 Sequenz 48 Nukleotide umfaßt, gefolgt von den Nukleotiden 3587 bis 5219 der HRV2 Sequenz. Zum weiteren Vergleich kann das 2C Gen hinter die 5' UTR des HRV2 und hinter die 5' UTR Region des Kaninchen-Globingens kloniert werden. Es resultieren die Plasmide pHRV2/5'UTR-2C bzw. pβ-2C (Fig. 6, Beispiel 1).

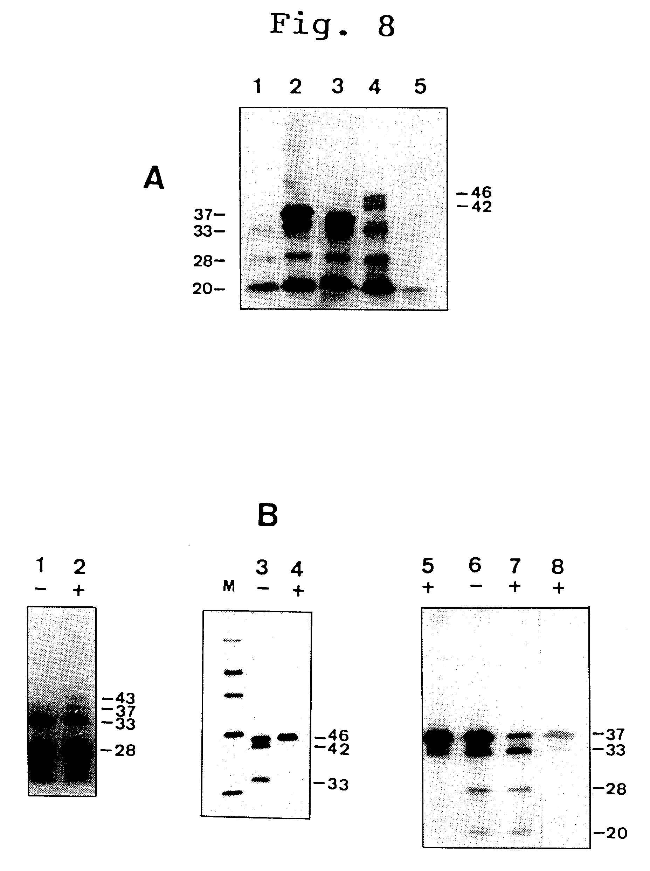

Fig. 8A zeigt die Translationsprodukte der RNAs, die nach in vitro Transkription von den Plasmiden pHRV2/5'UTR-2C, pβ-2C, pLink-2C und pLink-2BC erhalten werden. Überraschenderweise werden von jeder RNA mehrere Proteine translatiert.

Unter den Produkten der RNAs der Plasmide pLink-2C und pβ-2C tritt eine Bande von 37 kD auf; diese Bande entspricht der Größe des reifen 2C Proteins. Dieses Produkt ist offensichtlich durch Initiation am ersten AUG der beiden Konstruktionen entstanden (in Figur 6 durch schwarze Pfeile gekennzeichnet).

Die anderen Banden (33 kD, 28 kD und 20 kD) sind Produkte der Initiation an den in Fig. 6 mit offenen Pfeilen gekennzeichneten AUGs.

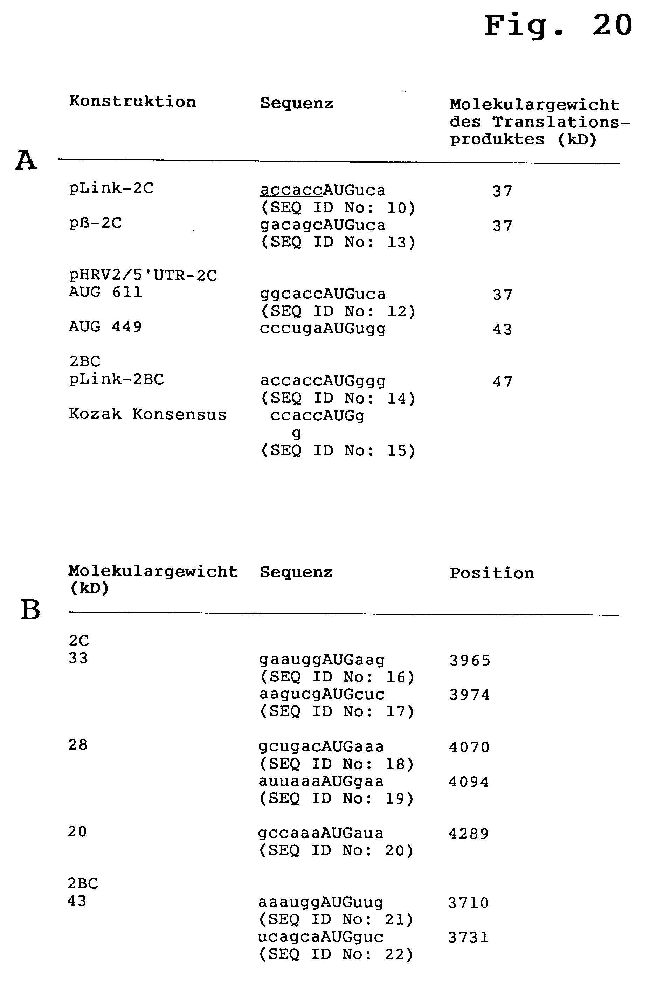

Überraschenderweise tritt bei Translation der RNA der Konstruktion pHRV2/5'UTR-2C keine 37 kD Bande auf; es folgt daher, daß keine Initiation bei AUG 611 stattgefunden hat. Die Identität der 37 kD Bande mit dem maturen 2C Protein wird dadurch bestätigt, daß sie in den Translationsprodukten der RNA des Plasmids pLink-2BC nicht vorhanden ist. Hier sind zusätzlich zu den 33 kD, 28 kD und 20 kD Banden zwei weitere Banden von 47 kD und 42 kD zu finden. Die 37 kD Bande fehlt, da sie von dem synthetisch eingeführten AUG vor dem 2C Gen stammt. Die 47 kD Bande entstammt der Initiation an dem ersten AUG des 2BC Gens (in Figur 6 schwarz gekennzeichnet) und die 42 kD Bande der Initiation an einem AUG im 2BC Gen. In Fig. 20 sind die Sequenzen im Bereich jener AUGs angegeben, die für die internen Initiationen verantwortlich sind.

Das unerwartete Phänomen der internen Initiation wurde ebenfalls mit Transkripten überprüft, die eine 5' Cap-Struktur besitzen und somit der Struktur der zellulären RNAs der infizierten Wirtszellen entsprechen. In Figur 8B sind die Translationsprodukte der mit einer Cap-Struktur versehenen RNAs, die von den Plasmiden pHRV2/5'UTR-2C, pLink-2C und pLink-2BC transkribiert werden, nach einer elektrophoretischen Auftrennung gezeigt. Von den RNAs der Plasmide pLink-2C und pLink-2BC wurde hier fast ausschließlich an jenen AUGs initiiert, die in Figur 6 mit schwarzen Pfeilen gekennzeichnet sind; es ist nahezu ausschließlich die 37 kD bzw. die 47 kD Bande zu erkennen. Die Anwesenheit der Cap-Struktur führt also zur Zurückdrängung der internen Initiation. Bei den Translationsprodukten der mit einer Cap-Struktur versehenen RNA der Konstruktion pHRV2/5'UTR gibt es kaum Unterschiede zu jenen mit der nicht-gecappten RNA. Die schwachen Banden bei 37 kD und 43 kD deuten auf eine Initiation bei den AUG Codons 449 und 611 hin. Die 5'UTR- Region von HRV2 kann daher, auch wenn gecappt, die Eigenschaft der 2C RNA, eine interne Translation zu initiieren, nicht unterdrücken.

Für die Analyse des "host cell shut-offs" können nun die Translationsmischungen mit der den "host cell shut-off" auslösenden picornaviralen Proteinase vorinkubiert werden. Dabei stellt sich überraschenderweise heraus, daß zum Beispiel nach Vorinkubation der Translationsmischung mit der HRV2 2A Proteinase die Translation des mit einer Cap-Struktur versehenen pLink-2C Transkriptes das gleiche Proteinmuster wie das entsprechende Transkript ohne Cap-Struktur liefert.

In Figur 8B, Spur 7, sind die erhaltenen Translationsprodukte gezeigt. Obwohl eine RNA mit Cap-Struktur eingesetzt wurde, beobachtet man das Translationsmuster einer RNA ohne Cap-Struktur; die Anwesenheit von 2A verändert offensichtlich die Eigenschaften des Kaninchenreticulocytenlysates.

Daß die 2A Proteinase für die Änderung verantwortlich ist, wird vom Ergebnis in Figur 8B, Spur 8 unterstützt. In diesem Experiment (Beispiel 3) wurde die 2A Präparation zuerst mit Elastatinal versetzt (ein Inhibitor der 2A Aktivität). Die Translation einer RNA ohne Cap-Struktur läuft in diesem Fall in Kaninchenretikulocyten normal ab; die Inhibition der 2A verhindert die Änderung der Translationseigenschaften.

Durch das unerwartete Auftreten unterschiedlicher Proteinmuster in Abhängigkeit der Cap-Struktur und der Zugabe einer viralen Proteinase ergibt sich ein einfaches Verfahren zur Analyse des durch Picornaviren verursachten "host cell shut-offs".

Das Verfahren kann insbesondere zur Charakterisierung und Isolierung von Substanzen verwendet werden, die am "host cell shut-off" beteiligt sind. Virale wie zelluläre Substanzen können auf die Beteiligung am "host cell shut-off" untersucht werden. Dazu können z.B. die Translationsmischungen fraktioniert eingesetzt werden. Außerdem ist es möglich mit Hilfe dieses Verfahrens Inhibitoren des "host cell shut-off" zu finden. Beispiel 2 zeigt, daß der Zusatz von Elastatinal zur Translationsmischung die Wirkung der zugefügten HRV2 Proteinase spezifisch aufhebt. Eine Inhibition kann eindeutig am Translationsmuster abgelesen werden.

Neben dem Kaninchenreticulozytenlysat können natürlich auch andere Translationssysteme verwendet werden, z.B. HeLa-Zellextrakte. Beispiel 4 beschreibt ein in vitro System zur Detektion des Einflusses einer picornaviralen Proteinase auf die Translation einer cap-versehenen RNA in einem HeLa-Zellextrakt. Dieses Translationssystem ist an sich bekannt (Lawson, M. A., und Semler, B. L. (1991) Proc. Natl. Acad. Sci. USA, 88, 9919-9923 und Molla, A., Paul, A.V., und Wimmer, E. (1991) Science 254, 1647-1651).

Beispiel 5 zeigt eine Untersuchung des unerwarteten Phänomens der internen Initiation im Kaninchenreticulozytenlysat, das im Beispiel 3 mit RNA der Konstruktion pLink-2C und PB-2C beschrieben wurde.

Im folgenden wird die Verwendung des Verfahrens zur Analyse des "host cell shut-offs" am Beispiel der Wechselwirkung der rhinoviralen HRV2 2A Proteinase mit dem entsprechenden zellulären Ziel erläutert. Das Verfahren erlaubt gemäß der Erfindung die Überprüfung der Aktivität von Inhibitoren des "host cell shut-offs".

Die Aminosäuren um eine Proteinase Spaltungsstelle werden im allgemeinen folgendermaßen bezeichnet:

...P4-P3-P2-P1-P1'-P2'-P3'-P4'......,

wobei die Spaltung zwischen P1 und P1' stattfindet. Aufgrund bekannter Studien mit einer rekombinanten Proteinase 2A von HRV2 (HRV2 2A) konnte innerhalb der natürlichen Spaltregion P8 bis P8' (SEQ ID NO. 23) gezeigt werden, daß besonders die Position P2, überraschenderweise aber nicht Position P1, entscheidend für die Spaltbarkeit eines Peptidsubstrates ist. Die Verwendung von C- und N-terminal verkürzten Peptidsubstraten wies auf weitere zusätzliche wichtige Positionen in der P-Region ("upstream" von der Spaltstelle) und der P1'- und P2'-Position hin (Sommergruber et al. (1992) J. Biol. Chem. 267, 22639-22644).

Zur genaueren Charakterisierung der Substratspezifität bzw. Erkennungssequenz von HRV2 2A (insbesondere der P1'-Position und der P8-P3-Region), können, wie in den Beispielen 7 und 8 durchgeführt, 15-mer Oligopeptide synthetisiert werden, die jeweils einen Aminosäureaustausch in den Positionen P8 bis P6' aufwiesen. Dabei wurde in den einzelnen Positionen jeweils gegen eine saure, basische, hydrophobe und polare Aminosäure ausgetauscht. Im Fall der Positionen P2, P1 und P1' wurden mehrere Aminosäuresubstitutionen vorgenommen.

Basierend auf diesen Daten sowie den Daten für die Peptidsubstrate aus Beispiel 7 und den Ergebnissen der Deletionsstudien in Peptidsubstraten (Sommergruber et al., loc cit) kann folgende potentielle Erkennungssequenz für HRV2 2A in einem Peptidsubstrat abgeleitet werden:


```
P7(ARG)-P6(X)-P5(X)-P4(Ile/Leu)-P3(X)-P2(Thr)-P1(X)*P1'
(Gly)-P2'(Pro)
```


Damit ergibt sich die Möglichkeit, kompetitive Inhibitoren der rhinoviralen HRV2 2A Proteinase durch Aminosäuresubstitution aus der natürlichen Spaltsequenz TRPIITTAGPSDMYVH (SEQ ID NO. 23) oder aber aus der Spaltsequenz des zellulären Ziels der HRV2 2A Proteinase zu bilden. Die so erhaltenen Inhibitoren können mit dem oben beschriebenen Verfahren auf ihre Wirksamkeit überprüft werden.

Überraschenderweise liefert der Sequenzvergleich des Aminosäuremotivs R-X-X-I/L-X-T-X-G-P mit der Aminosäuresequenz des humanen p220 Proteins (eIF-4γ; Yan et al. (1992) J. Biol. Chem. 267, 23226) zwei Peptidsequenzen, p220-3 (SEQ ID NO. 124) und p220-4 (SEQ ID NO: 125), einerseits als Substrat, andererseits aber auch als kompetitive Inhibitoren der HRV2 2A Proteinase dienen können.

Die kompetitive Wirkung konnte durch Vergleich mit der Spaltung der entsprechenden natürlichen Erkennungssequenz der HRV2 2A Proteinase Peptid P8-P8' (SEQ ID NO. 23) bestimmt werden. Die Durchführung der Kompetitionsexperimente ist in Beispiel 7 erläutert.

Die Peptide P8-P8' (SEQ ID NO. 23), p220-3 (SEQ ID NO. 124) und p220-4 (SEQ ID NO. 125) sind in der Lage, den "host cell shut-off" zu inhibieren.

Damit umfaßt die Erfindung außerdem Inhibitoren des "host cell shut-offs", der durch die rhinovirale 2A Proteinase verursacht wird. Die Inhibitoren sind dadurch gekennzeichnet, daß sie von der natürlichen Spaltungssequenz TRPIITTAGPSDMYVH (SEQ ID NO. 23) abgeleitet sind, wobei das Aminosäuremotiv R-X-X-I/L-X-T-X-G-P erhalten bleibt (X stellt eine beliebige Aminosäure dar), wobei Aminosäuren, die keine Seitengruppen mit sauren Resten (Glutaminsäure, Asparaginsäure) enthalten, bevorzugt sind.

Bevorzugt sind die Inhibitoren, die an Position P1 Methionin enthalten.

Insbesondere umfaßt die Erfindung die Peptide P8F (SEQ ID NO. 26), P8K (SEQ ID NO. 28), P3K (SEQ ID NO. 48), PC1 (SEQ ID NO. 82), P4'K (SEQ ID NO. 110), P4'F (SEQ ID NO. 111, P4'T (SEQ ID NO. 112), P5'K (SEQ ID NO. 116), MMP-1 (SEQ ID NO. 127) und MMP-2 (SEQ ID NO. 128).

Der Begriff Inhibitor umfaßt die oben genanten Peptide als auch solche, die zusätzliche chemische Gruppen besitzen, die normalerweise nicht Teil dieses Moleküls sind. Diese chemischen Derivate enthalten Gruppen, die die Molekülöslichkeit, die Absorption, die biologische Halbwertszeit usw. verbessern oder alternativ die Toxizität oder unerwünschte Nebeneffekte verringern können. Gruppen, die solche Effekte vermitteln, sind bekannt (Remington's Pharmazeutical Sciences (1980)):

Cysteinyl-Reste werden gewöhnlich mit α-Haloacetaten (sowie den korrespondierenden Aminen), wie Chloressigsäure oder Chloracetamid zu Carboxymethyl- oder Carboxyamidomethylderivaten umgesetzt. Cysteinyl-Reste können auch mit Bromtrifluoraceton, Chloracetylphosphat, N-Alkylmaleinimid, 3-Nitro-2-pyridylsufid, Methyl-2-pyridyldisulfid, p-Chlormercuribenzoat, 2-Chlormercuri-4-nitrophenol oder Chlor-7-nitrobenzo-2-oxa-1,3-diazol umgesetzt werden.

Histidylreste können z.B. durch Reaktion mit Diethylpyrocarbonat bei pH 5,5 bis 7,0 derivatisiert werden. Lysinyl- und aminoterminale Reste werden mit Bernsteinsäure- oder anderen carboxylischen Säureanhydriden umgesetzt. Diese Reaktionen bewirken eine Ladungsumkehr an Lysinylresten. Andere geeignete Reagentien zur Derivatisierung von Resten, die α-Aminogruppen enthalten schließen Imidoester wie Methylpicolinimidate, Pyridoxalphosphate, Pyridoxal, Chlorborhydrid, Trinitrobenzoesulfonsäure, O-Methylharnstoff; 2,4-Pentandion sowie Transaminasekatalysierte Reaktionen mit Glyoxylat ein.

Arginylreste werden durch Reaktion mit konventionellen Reagenzien wie Phenylglyoxal, 2,3-Butandion, 1,2-Cyclohexandion und Ninhydrin modifiziert. Die Derivatisierung der Arginin-Reste setzt alkalische Reaktionsbedingungen voraus. Außerdem können diese Reagentien mit Aminogruppen aus Lysinresten und mit der Arginin δ-Aminogruppe reagieren.

Die spezifischen Modifikationen der Tyrosylreste sind ausführlich beschrieben. Insbesondere die Einführung photometrisch detektierbarer Reste durch Reaktion mit aromatischen Diazoniumgruppen oder mit Tetranitromethan führt zur Bildung von O-Acetyltyrosylspezies bzw. 3-Nitroderivaten. Tyrosyl-Reste können mit $^{125}$I oder $^{131}$I iodiert werden. Die markierten Peptide können dann in Radioimmunoassays eingesetzt werden.

Carboxylreste z.B. in Asparaginsäure und Glutaminsäure können selektiv mit Carbodiimiden (R'-N-C-N-R') wie 1-Cyclohexyl-3-(2-morpholinyl-(4-ethyl)carbodiimid umgesetzt werden. Daneben können Aspartyl- und Glutamylreste durch Reaktion mit Ammoniumionen in Asparginyl- und Glutaminylreste umgewandelt werden.

Glutaminyl- und Asparaginylreste werden häufig deamidiert, um die entsprechenden Glutamyl- und Aspartylreste zu erhalten.

Die Erfindung umfaßt ebenfalls Arzneimittel zur Inhibierung der HRV2A Proteinase, die die oben beschriebenen Inhibitoren enthalten, gegebenenfalls mit an sich bekannten Hilfs- und/oder Trägerstoffen und/oder Stabilisatoren.

Weiterhin umfaßt die Erfindung die Verwendung der oben beschriebenen Inhibitoren zur Herstellung von Arzneimitteln zur Inhibierung der HRV2A Proteinase sowie ein Mittel enthaltend eines der oben genannten Peptide, gegebenenfalls mit an sich bekannten Hilfs- und/oder Trägerstoffen zur Inhibierung der HRV2A Proteinase. Die Erfindung umfaßt weiterhin die Verwendung der oben beschriebenen Peptide zur Inhibierung der HRV2A Proteinase sowie ein Verfahren zur Herstellung der Peptide mittels Fmoc-Strategie mit aktivierten Estern bzw. über HOBt/DIPCDI-Aktivierung sowie nach Analogieverfahren.

Die erfindungsgemäßen Polypeptide sowie ihre pharmakologisch unbedenklichen Säureadditionssalze können in bekannter Weise in die üblichen Formulierungen - wie Tabletten, Dragees, Pillen, Granulate, Aerosole, Siruppe, Emulsionen, Suspensionen und Lösungen unter Verwendung inerter pharmazeutisch geeigneter Trägerstoffe oder Lösungsmittel überführt werden. Hierbei soll der Anteil der pharmazeutisch wirksamen Verbindung(en) jeweils im Bereich von 0,5 bis 90 Gew.-% der Gesamtzusammensetzung liegen, d.h. in Mengen, die ausreichend sind, um den unten angegebenen Dosierungsbereich zu erreichen.

Die Formulierungen werden zum Beispiel durch Verstrecken der Wirkstoffe mit Lösungsmitteln und/oder Trägerstoffen, gegebenenfalls unter Verwendung von Emulgiermitteln und/oder Dispergiermitteln hergestellt, wobei beispielsweise bei der Verwendung von Wasser als Verdünnungsmittel gegebenenfalls organische Lösungsmittel als Lösungsvermittler bzw. Hilfslösungsmittel eingesetzt werden können.

Als Hilfsstoffe seien beispielsweise Wasser, pharmazeutisch unbedenkliche organische Lösungsmittel, wie Paraffine, Öle pflanzlichen Ursprungs, mono- oder polyfunktionelle Alkohole, Trägerstoffe, wie z.B. natürliche Gesteinsmehle, synthetische Gesteinsmehle, Zucker, Emulgiermittel und Gleitmittel erwähnt.

Die Applikation erfolgt in üblicher Weise, vorzugsweise oral oder parenteral insbesondere perlingual oder intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Die Tabletten können auch aus mehreren Schichten bestehen. Entsprechend können Dragées durch Überziehen von analog den Tabletten hergestellten Kernen mit den üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmackverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethyl-cellulose Netzmittel, beispielsweise Kondensationsprodukte von Fettalkoholen mit Ethylenoxid oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise in der Weise hergestellt werden, daß man die Wirkstoffe mit inerten Trägern, wie Milchzucker oder Sorbit, mischt und in Gelatinekapseln einkapselt.

Für den Fall der parenteralen Anwendung können Lösungen der Wirkstoffe unter Verwendung geeigneter flüssiger Trägermaterialien eingesetzt werden.

Die Dosierung für die orale Anwendung liegt bei 1-300 mg, vorzugsweise zwischen 5 und 150 mg.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen. Ferner können die erfindungsgemäßen Polypeptide bzw. deren Säureadditionssalze auch mit andersartigen Wirkstoffen kombiniert werden.

Im einzelnen umfaßt die Erfindung

- ein Verfahren zur Analyse des durch Picornaviren verursachten "host cell shut-offs", das dadurch gekennzeichnet ist, daß

  eine RNA abgeleitet von einem Gen mit mindestens einem internen Translationsstart mit einer Cap-Struktur versehen wird, in einer Translationsmischung, die mit einer picornaviralen Proteinase, die vorinkubiert ist, in vitro translatiert wird und die Translationsprodukte analysiert werden.

- Die in vitro translatierten Proteine sind markiert.

- Die erhaltenen Proteine werden mit Translationsprodukten der RNA ohne Cap-Struktur und/oder ohne Vorinkubation der Translationsmischung mit einer picornaviralen Proteinase verglichen.

- Die RNA wird durch in vitro Transkription eines picornaviralen 2C Gens, bevorzugt eines rhinoviralen 2C Gens, erhalten.

- Insbesondere kann die RNA durch in vitro Transkription des Plasmids pLink-2C erhalten werden. Zum Vergleich können RNAs der Plasmide pHRV2/5'UTR-2C, p$\beta$-2C oder pLink 2BC verwendet werden.

- Für die Vorinkubation wird eine lösliche, mature picornavirale 2A Proteinase, rhinovirale 2A Proteinase, bevorzugt die HRV2 2A Proteinase verwendet. Die jeweilige Proteinase kann dazu in Form eines angereicherten Rohextraktes oder in reiner Form verwendet werden.

- Die Translationsmischung besteht aus einem Reticulozytenlysat oder einem HeLa-Zellextrakt oder fraktionierten Anteilen dieser Translationssysteme.

- In einer bevorzugten Ausführungsform ist das erfindungsgemäße Verfahren, dadurch gekennzeichnet, daß das HRV2 2C-Gen mit Start- und Stoppcodons ausgestattet in vitro transkribiert, das Transkript mit einer Cap-Struktur versehen wird und in einer Translationsmischung, die mit einer rhinoviralen 2A Proteinase vorinkubiert ist, translatiert und die translatierten Proteine mit Translationsprodukten der RNA ohne Cap-Struktur bzw. unter Zusatz von Inhibitoren mittels radioaktiver Markierung, Elektrophorese und Autoradiographie verglichen werden.

- Das Verfahren kann zum Nachweis und zur Isolierung der zellulären und der viralen Proteine, die am "host cell shut-off" beteiligt sind, sowie zur Isolierung von Inhibitoren des "host cell shut-offs" verwendet werden. Speziell können Inhibitoren der rhinoviralen Proteinasen isoliert werden.

- Außerdem beinhaltet die Erfindung die Plasmide pHRV2/5'UTR-2C, pLink-2C, pLink-2BC, pSVL-2BC, pSVL-2C und p$\beta$-2C.

- Die Erfindung umfaßt weiterhin ein Verfahren zur rekombinanten Herstellung einer löslichen, maturen, picornaviralen 2A Proteinase, das dadurch gekennzeichnet ist, daß ein Genfragment für das C-terminale Ende des VP1 Proteins und darauf folgend das vollständige Gen für die 2A Proteinase in den Expressionsvektor pET8c oder pET3b kloniert und zur Expression gebracht wird.

- Die 2A Proteinase kann insbesondere in dem E. coli Stamm BL21(DE3)LysS oder -LysE als Fusionsprotein exprimiert und durch autokatalytische Aktivität die mature 2A Proteinase freigesetzt werden.

- Für das C-terminale Ende des VP1 Proteins und das vollständige Gen für die 2A Proteinase kann eine DNA-Sequenz codierend für folgende Aminosäuresequenz ausgewählt werden:

↓VP1

Asn Pro Val Glu Asn Tyr Ile Asp Glu Val Leu Asn Glu Val Leu Val
1                   5                 10                      15

Val Pro Asn Ile Asn Ser Ser Asn Pro Thr Thr Ser Asn Ser Ala Pro
              20                  25                      30

```
Ala Leu Asp Ala Ala Glu Thr Gly His Thr Ser Ser Val Gln Pro Glu
        35              40              45

Asp Val Ile Glu Thr Arg Tyr Val Gln Thr Ser Gln Thr Arg Asp Glu
        50              55              60

Met Ser Leu Glu Ser Phe Leu Gly Arg Ser Gly Cys Ile His Glu Ser
65              70              75              80

Lys Leu Glu Val Thr Leu Ala Asn Tyr Asn Lys Glu Asn Phe Thr Val
            85              90              95

Trp Ala Ile Asn Leu Gln Glu Met Ala Gln Ile Arg Arg Lys Phe Glu
            100             105             110

Leu Phe Thr Tyr Thr Arg Phe Asp Ser Glu Ile Thr Leu Val Pro Cys
        115             120             125

Ile Ser Ala Leu Ser Gln Asp Ile Gly His Ile Thr Met Gln Tyr Met
        130             135             140

Tyr Val Pro Pro Gly Ala Pro Val Pro Asn Ser Arg Asp Asp Tyr Ala
145             150             155             160

Trp Gln Ser Gly Thr Asn Ala Ser Val Phe Trp Gln His Gly Gln Ala
            165             170             175

Tyr Pro Arg Phe Ser Leu Pro Phe Leu Ser Val Ala Ser Ala Tyr Tyr
            180             185             190

Met Phe Tyr Asp Gly Tyr Asp Glu Gln Asp Gln Asn Tyr Gly Thr Ala
        195             200             205

Asn Thr Asn Asn Met Gly Ser Leu Cys Ser Arg Ile Val Thr Glu Lys
        210             215             220

His Ile His Lys Val His Ile Met Thr Arg Ile Tyr His Lys Ala Lys
225             230             235             240

His Val Lys Ala Trp Cys Pro Arg Pro Arg Ala Leu Glu Tyr Thr
            245             250             255

Arg Ala His Arg Thr Asn Phe Lys Ile Glu Asp Arg Ser Ile Gln Thr
            260             265        ↓ P2A    270

Ala Ile Val Thr Arg Pro Ile Ile Thr Thr Ala Gly Pro Ser Asp Met
        275             280             285

Tyr Val His Val Gly Asn Leu Ile Tyr Arg Asn Leu His Leu Phe Asn
        290             295             300

Ser Glu Met His Glu Ser Ile Leu Val Ser Tyr Ser Ser Asp Leu Ile
305             310             315             320

Ile Tyr Arg Thr Asn Thr Val Gly Asp Asp Tyr Ile Pro Ser Cys Asp
            325             330             335
```

```
Cys Thr Gln Ala Thr Tyr Tyr Cys Lys His Lys Asn Arg Tyr Phe Pro
            340                 345                 350

Ile Thr Val Thr Ser His Asp Trp Tyr Glu Ile Gln Glu Ser Glu Tyr
            355                 360                 365

Tyr Pro Lys His Ile Gln Tyr Asn Leu Leu Ile Gly Glu Gly Pro Cys
    370                 375                 380

Glu Pro Gly Asp Cys Gly Gly Lys Leu Leu Cys Lys His Gly Val Ile
385                 390                 395                 400

Gly Ile Val Thr Ala Gly Gly Asp Asn His Val Ala Phe Ile Asp Leu
            405                 410                 415

Arg His Phe His Cys Ala Glu Glu Gln
            420                 425
```

- Weiterhin umfaßt die Erfindung die Plasmide pET8c/2A und pET/2A und ein
- Verfahren zur Reinigung einer löslichen, maturen rhinoviralen Proteinase 2A, das dadurch gekennzeichnet ist, daß
  a) die Proteinase in einem heterologen Wirt exprimiert,
  b) die die Proteinase enthaltenden Zellen lysiert,
  c) die Proteinase aus dem Zellüberstand durch fraktionierte Ammoniumsulfatfällung konzentriert und
  d) durch Ionenaustausch- und Gelchromatographie gereinigt wird.
- Inhibitoren der rhinoviralen HRV2 2A Proteinase abgeleitet aus der natürlichen Spaltungssequenz TRPIITTAGSPSDMYVH (SEQ ID NO. 23), die das Aminosäuremotiv R-X-X-I/L-X-T-X-G-P enthalten und mindestens 10, höchstens aber 16 Aminosäuren umfassen, wobei X eine beliebige natürliche Aminosäure ist, bevorzugt eine, die keine sauren Seitengruppen enthält. Bevorzugt sind Inhibitoren die an Position P1 Methionin enthalten oder die Peptide P8F (SEQ ID NO. 26), P8K (SEQ ID NO. 28), P3K (SEQ ID NO. 48), P1C (SEQ ID NO. 82), P4'K (SEQ ID NO. 106), P4'F (SEQ ID NO. 111), P4'T (SEQ ID NO. 108), P5'K (SEQ ID NO. 116), MMP-1 (SEQ ID NO. 123) oder MMP-2 (SEQ ID NO. 128) sind.
- Inhibitoren der rhinoviralen HRV2 2A Proteinase, abgeleitet aus der p220 Sequenz, dadurch gekennzeichnet, daß es das Aminosäuremotiv R-X-X-I/L-X-T-X-G-P enthält, wobei X eine beliebige Aminosäure ist und/oder es sich um die Peptide p220-3 (SEQ ID NO. 120) oder p220-4 (SEQ ID NO. 121) handelt.
- Die Verwendung eines der oben genannten Inhibitoren zur Herstellung eines Arzneimittels zur Inhibierung der HRV2 2A Proteinase.
- Mittel enthaltend einem oben genannten Inhibitor gegebenenfalls mit Hilfs- und/oder Trägerstoffen und/oder Stabilisatoren zur Inhibierung der HRV2 2A Proteinase.
- Verfahren zur Herstellung der Inhibitoren nach der Fmoc-Strategie mit aktivierten Estern oder über HOBI-DIPCDI Aktivierung sowie nach Analogieverfahren.

Anhand der folgenden Beispiele wird die Erfindung erläutert.

**Beispiel 1: In vitro Translation von RNAs abgeleitet von rhinoviralen cDNA Fragmenten des 2C Gens.**

**A) Einbau der Stopcodons**

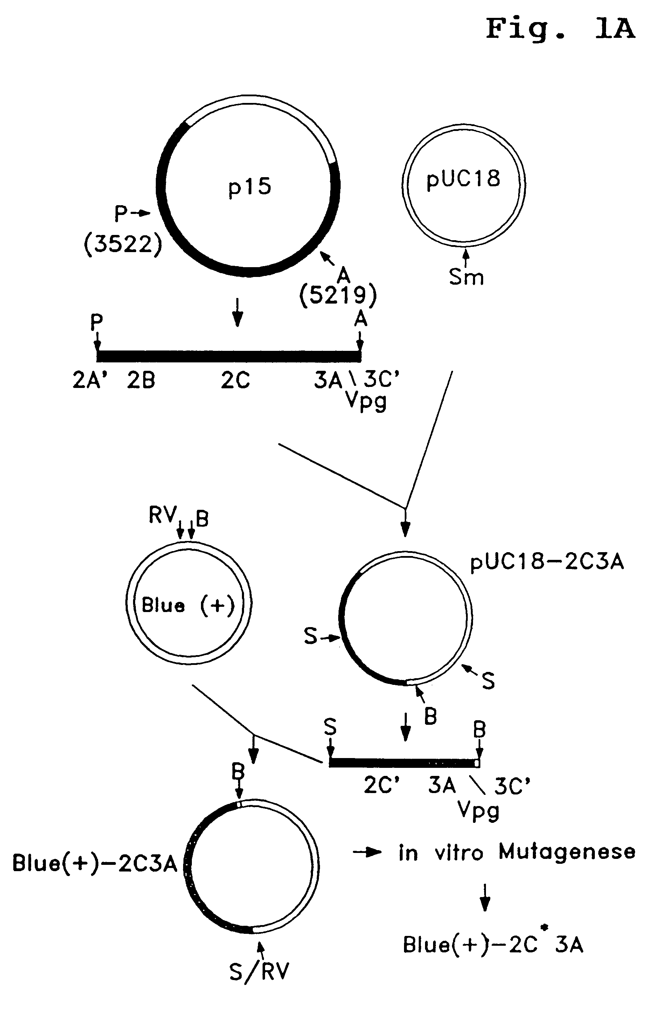

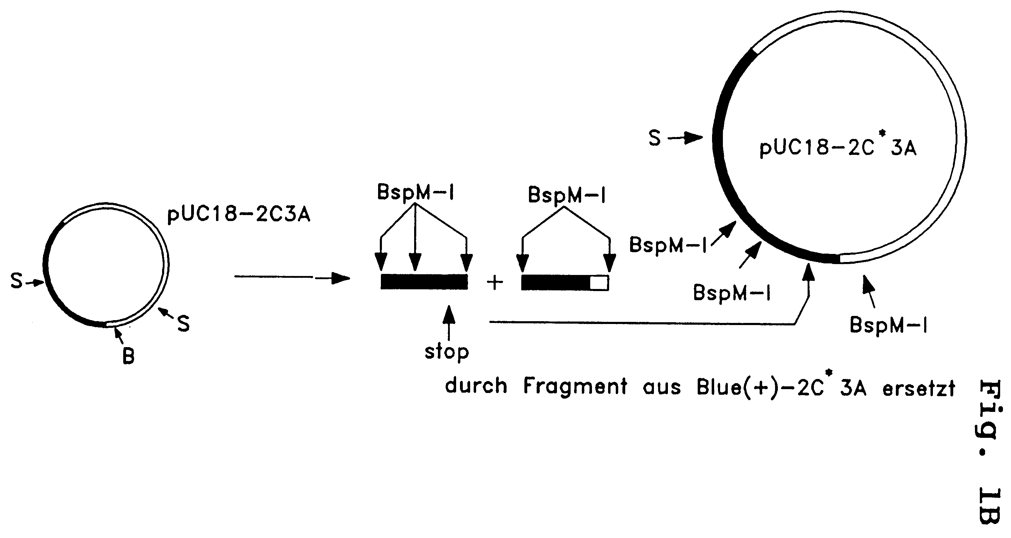

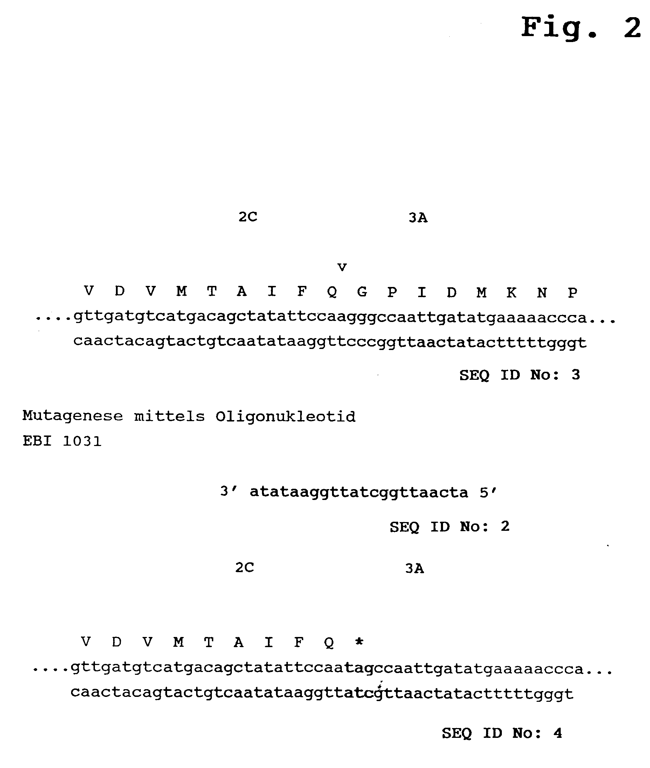

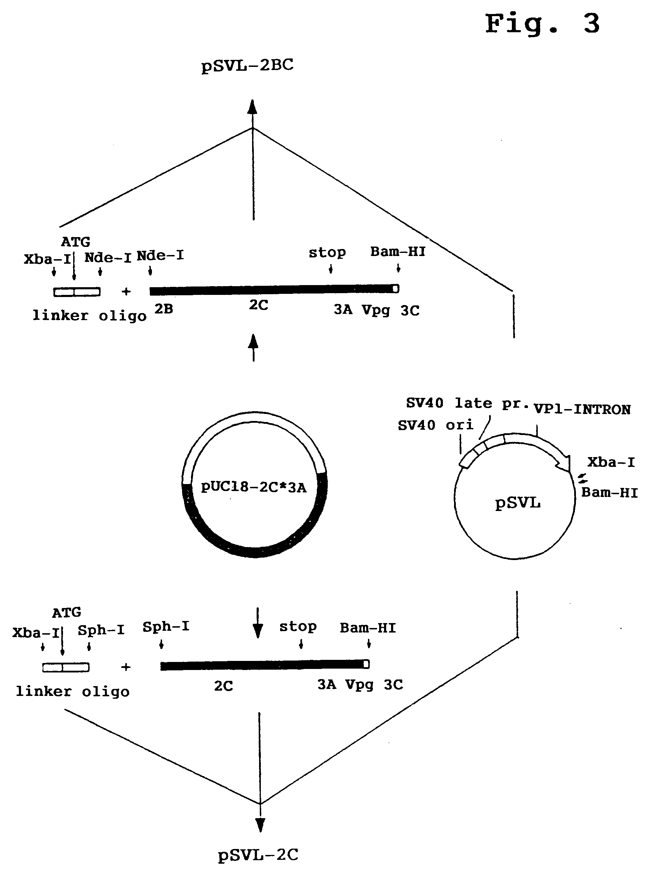

Das Plasmid p15 (Duechler et al. (1989), loc. cit.), das die cDNA von HRV2 von Nukleotid 2125 bis 7102 enthält (Skern et al. (1985) Nucl. Acids Res. 13, 2111-2126), wurde mit Acc I geschnitten und anschließend mit Mung Bean Nuklease behandelt, um die überhängenden 5'-Enden der resultierenden Fragmente zu entfernen. Danach wurde mit Pvu II weiterverdaut. Das resultierende 1.7 kb Fragment (Nukleotid 3522 bis 5219; Figur 2), das einen Teil der 2A Sequenz (als 2A' bezeichnet), die gesamte Sequenz von 2B, 2C, 3A, 3B und einen Teil von 3C (als 3C' bezeichnet) enthielt, wurde in die Sma I Stelle von pUC18 hineinkloniert. Das daraus resultierende Plasmid (pUC18-2C3A) wurde einem Doppelverdau mit Ssp I (Schnittstelle bei Nukleotid 4335) und BamHI (im Polylinker) unterzogen. Das 0.89 kb Fragment, das die viralen Sequenzen enthielt, wurde in die EcoRV und BamHI Stelle des Bluescript(+) Vektors (Stratagene) hineinkloniert. Nach Transfektion von E. coli 5K wurde mit Hilfe eines M13 Helferphagen (VCS-M13, Stratagene) einzelsträngige DNA von Blue(+)-2C3A isoliert. Diese DNA wurde für den Einbau eines Stopcodons nach 2C (GGG -> TAG) verwendet, wie in Figur 1 gezeigt wird. Dabei wurden unter Anwendung von Standardtechniken (Taylor et al., (1985), Nucl. Acids Res. 13, 8765-8785) das Oligonukleotid 5'-ATCAATTGGCTATTGGAATATA-3' (SEQ ID No: 2) herangezogen (Fig. 3).

Ein Klon mit den eingebauten Stopcodons (Blue(+)-2C*3A) wurden nach der Transfektion isoliert und seine Richtigkeit durch direkte DNA Sequenzierung der mutierten Region überprüft (Tabor, S. und Richardson, C.C., (1987), Proc. Natl. Acad. Sci. USA 84, 4767-4771).

Der mutierte DNA-Bereich wurde aus Blue(+)-2C*3A isoliert und wieder in die Wildtyp-Umgebung in pUC18-2C3A eingebracht (Fig. 1). Blue(+)-2C*3A wurde mit BspMI partiell verdaut und das 312 bp lange Fragment, welches die Mutation enthielt, isoliert. pUC18-2C3A wurde vollständig mit demselben Enzym verdaut und das 365 bp lange Fragment wurde ebenfalls isoliert. Ein Aliquot desselben Verdaus wurde mit alkalischer Phosphatase dephosphoryliert. Eine Ligation dieser beiden Fragmente mit verdautem und dephosphoryliertem pUC18-2C3A führte nach Transformation in kompetente E.coli Zellen zu pUC18-2C*3A. Da BspMI ein unterbrochenes Palindrom erkennt, sind die überlappenden Enden immer verschieden; dadurch ist es möglich, drei Fragmente auf diese Art und Weise kontrolliert miteinander zu ligieren.

## B) Einbau der Startcodons vor die 2BC- und 2C-Gene

Das Plasmid pSVL (Pharmacia; Figur 3) wurde mit den Restriktionsenzymen XbaI und BamHI geschnitten und mit alkalischer Phosphatase aus Kälberdarm behandelt. pUC18-2C*3A (Figur 3) wurde einerseits mit den Restriktionsenzymen NdeI und BamHI geschnitten und das 1,6 kb Fragment aus einem Agarosegel eluiert und mit den 5'phosphorylierten Oligonukleotiden EBI 1102/1094 (SEQ ID No: 5) ligiert (Figur 4). Das Oligonukleotidpaar enthielt ein AUG Codon vor dem ersten Codon des 2BC Genes und die zu ergänzenden Codons bis zur NdeI Schnittstelle. Die Sequenz vor dem AUG wurde so gewählt, daß sie die höchstmögliche Übereinstimmung mit der Konsensusequenz von Kozak besaß (Kozak, M., loc. cit.; Figur 20). Das erhaltene Plasmid wurde mit pSVL-2BC bezeichnet; die Integrität der Sequenz am 5' Ende des 2BC Genes wurde durch DNA Sequenzierung bewiesen.

Um das Plasmid pSVL-2C zu konstruieren, wurde analog vorgegangen: Das Plasmid pSVL (Pharmacia: Figur 3) wurde mit den Restriktionsenzymen XbaI und BamHI geschnitten und mit alkalischer Phosphatase aus Kälberdarm behandelt. pUC18-2C*3A (Figur 3) wurde andererseits mit den Restriktionsenzymen SphI und BamHI geschnitten und das 1,3 kb Fragment aus einem Agarosegel eluiert und mit den 5'phosphorylierten Oligonukleotiden EBI 1099/1092 (SEQ ID No: 6) ligiert (Figur 4). Das Oligonukleotidpaar enthielt ein AUG vor dem ersten Codon des 2C Genes und die fehlenden Codons bis zur SphI Schnittstelle. Die Sequenz vor dem AUG Codon wurde so gewählt, daß sie die höchstmögliche Übereinstimmung mit der Konsenssequenz von Kozak besaß (Kozak, M., loc. cit.). Ein Plasmid wurde erhalten, das pSVL-2C genannt wurde; die Integrität der Sequenz am 5' Ende des 2C Genes wurde durch DNA Sequenzierung bewiesen.

Um eine in vitro Transkription durch T7 RNA Polymerase zu ermöglichen, wurde das XbaI/BamHI Fragment von pSVL-2C mit pGEM2, das mit XbaI und BamHI geschnitten und mit alkalischer Phosphatase behandelt wurde, ligiert. Nach Transformation in kompetente E.coli Zellen wurde ein Plasmid (pLink-2C) isoliert, dessen Struktur in Figur 5 und 6 abgebildet ist. Nach der Linearisierung mit dem Restriktionsenzym BamHI entsteht bei der in vitro Transkription mit T7 RNA Polymerase eine RNA, die von der Transkriptionsstelle bis zum AUG Codon, dem Beginn der HRV2 Sequenz, 46 Nukleotide umfaßt, gefolgt von den Nukleotiden 3872 bis 5219 der HRV2 Sequenz.

Analog dazu wurde das XbaI/BamHI Fragment von pSVL-2BC in pBluescript (Stratagene), das mit XbaI und BamHI geschnitten und mit alkalischer Phosphatase behandelt wurde, ligiert. Nach Transformation in kompetente E. coli Zellen wurde ein Plasmid isoliert, genannt pLink-2BC, dessen Struktur in Figur 5 abgebildet ist. Nach der Linearisierung mit dem Restriktionsenzym BamHI entsteht bei der in vitro Transkription mit T7 RNA Polymerase eine RNA, die von der Transkriptionstelle bis zum AUG Codon, dem Beginn der HRV2 Sequenz, 48 Nukleotide umfaßt, gefolgt von den Nukleotiden 3587 bis 5219 der HRV2 Sequenz.

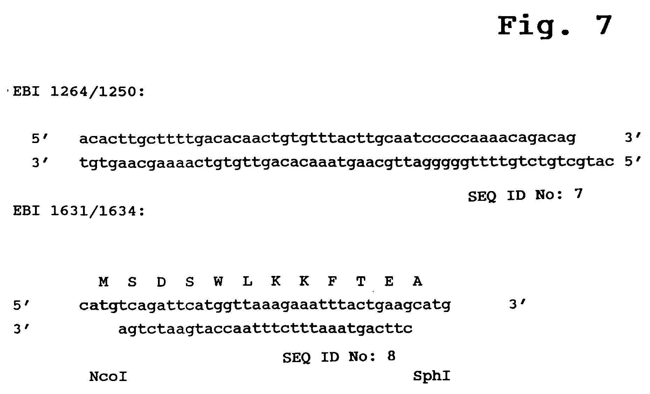

Um das 2C Gen hinter die 5'UTR von HRV2 zu setzen, wurde das Plasmid pHRV2/5'UTR-2C (Figur 6) folgendermaßen hergestellt. Das Plasmid GR6 (Beispiel 6) wurde mit den Restriktionsenzymen NcoI und BamHI geschnitten und mit alkalischer Phosphatase aus Kälberdarm dephosphoryliert. Das Plasmid pLink-2C wurde mit den Restriktionsenzymen SphI und BamHI geschnitten; das 1.3 kb Fragment wurde aus einem Agarosegel eluiert und mit der mit NcoI und BamHI geschnittenen GR6 DNA Präparation und mit den 5' phosphorylierten Oligonukleotiden EBI 1631 und 1634 (SEQ ID No: 7) ligiert (Figur 7; die Oligonukleotide wurden zusammen kurz auf 65°C gebracht und dann langsam auf 4°C gekühlt). Diese Oligonukleotide kodieren für die Aminosäuren, die zwischen dem AUG codon von pLink-2C und der SphI Schnittstelle liegen. Nach Transformation in kompetente E.coli-Zellen wurde ein Plasmid isoliert (pHRV2/5'UTR-2C), dessen Struktur in Figur 6 abgebildet ist. Nach der Linearisierung mit dem Restriktionsenzym BamHI entsteht bei der in vitro Transkription mit T7 RNA Polymerase eine RNA die von der Transkriptionstelle bis zur EcoRV Stelle, dem Beginn der HRV2 Sequenz, 19 Nukleotide umfasst, gefolgt von den Nukleotiden 19 bis 613 und 3872 bis 5219 der HRV2 Sequenz.

Um das 2C Gen hinter die 5'UTR des Kaninchen-$\beta$-Globingens zu setzen, wurde das Plasmid p$\beta$-2C (Figur 6) folgendermaßen hergestellt: Das Plasmid GR6 (Beispiel 6) wurde mit den Restriktionsenzymen EcoRV und BamHI geschnitten, das 3.0 kb Fragment aus einem Agarosegel isoliert und mit alkalischer Phosphatase aus Kälberdarm dephosphoryliert. Das Plasmid pLink-2C wurde mit den Restriktionsenzymen SphI und BamHI geschnitten; das 1.3 kb Fragment wurde aus einem Agarosegel eluiert und mit der mit NcoI und BamHI geschittenen GR6 DNA Präparation und mit den 5' phosphorylierten Oligonukleotidpaaren EBI 1264/1250 (SEQ ID No: 7) und EBI 1631/1634 (SEQ ID No: 8) ligiert (Figur 7; die Oligonukleotide wurden zusammen kurz auf 65°C gebracht und dann langsam auf 4°C gekühlt). Das Oligonukleotidpaar EBI 1264/1250 kodiert für die 5'UTR des Kaninchen $\beta$-Globingens und das Oligonukleotidpaar EBI 1631/1634 für die Aminosäuren, die zwischen dem AUG Codon von pLink-2C und der SphI Schnittstelle liegen. Nach Transformation in kompetente E.coli-Zellen wurde ein Plasmid isoliert (p$\beta$-2C), dessen Struktur in Figur 6 abgebildet ist. Nach der Linearisierung mit dem Restriktionsenzym BamHI entsteht bei der in vitro Transkription mit T7 RNA Polymerase eine RNA, die von der Transkriptionstelle bis zur EcoRV Stelle, dem Beginn der $\beta$-Globin 5'UTR, die 19 Nukleotide umfaßt, gefolgt von den 52 Nukleotiden, der gesamten $\beta$-Globin 5'UTR und den Nukleotiden 3872 bis 5219 der HRV2 Sequenz.

## C) Die in vitro Transkription und Translation

In vitro Transkription dieser Plasmide nach Linearisierung mit BamHI mittels T7 RNA Polymerase wurde wie beschrieben (Duechler et al. (1989), loc. cit.) durchgeführt. Für die Synthese von RNA mit Cap-Struktur wird dem Transkriptionsansatz $^7$mGpppG und GTP zugesetzt. Zum Beispiel kann der von Duechler et al. (1989, loc. cit.) beschriebene Reaktionsansatz 1 mM 7MeGppG und 250 $\mu$m GTP enthalten. Am Ende der Reaktionszeit wurde die DNA durch Zugabe von DNase I (RNAse-frei, Boehringer Mannheim) verdaut, die Proteine durch Phenolextraktion entfernt, und die RNA nach Zugabe von einem Drittel Volumen 8 M Ammoniumacetat mit Ethanol gefällt. Nach nochmaliger Fällung mit Ethanol wurde die RNA anschließend in Wasser aufgenommen und ein Aliquot durch Agarosegelelektrophorese auf Qualität überprüft. Der Laufpuffer enthielt 1% SDS. Für die in vitro Translation wurde jeweils 1 $\mu$g dieser RNAs eingesetzt. Die Reaktion enthielt in 20 $\mu$l 90 mM KCl, 0.3 mM MgCl$_2$, 10 mM Kreatinphosphat, 2 $\mu$l $^{35}$S-Trans-Label (ICN; 10 $\mu$Ci/$\mu$l, 1000 Ci/mmol) und 11,5 $\mu$l Kaninchenreticulocytenlysat, das mit Micrococcal Nuclease (Sigma) behandelt worden war. Das Kaninchenreticulocytenlysat wurde nach der Methode von Jackson, R. und Hunt, T. (Meth. Enzymol. (1983) 96, 50 - 74) durch Behandlung mit Micrococcal Nuclease hergestellt. Nach 1 Stunde bei 30°C wurde die Reaktion durch Zugabe von 1 $\mu$l 200 mM Methionin gestoppt und ein Aliquot (1 $\mu$l) auf einem SDS-Polyacrylamidgel Laemmli, U.K. (1970) Nature 291, 547-553) aufgetragen. Das Gel wurde nach Beendigung des Laufes fluorographiert.

In Figur 8A sind die Translationsprodukte der RNAs, die von den Plasmiden pHRV2/5'UTR-2C, p$\beta$-2C, pLink-2C und pLink-2BC transkribiert wurden, nach einer elektrophoretischen Auftrennung gezeigt. Überraschenderweise wurden von jeder RNA mehrere Proteine translatiert.

Um das unerwartete Phänomen der internen Initiation weiter zu untersuchen, wurden RNAs von den Plasmiden unter Bedingungen transkribiert, die zur Anwesenheit einer 5' Cap-Struktur führen. Die Transkription wurde daher wie oben beschrieben durchgeführt, nur daß das Reaktionsgemisch 1 mM $^7$mGpppG und 250 $\mu$M GTP enthielt. Die Translation wurde wie oben beschrieben durchgeführt.

In Figur 8B sind die Translationsprodukte der mit einer Cap-Struktur versehenen RNAs, die von den Plasmiden pHRV2/5'UTR-2C, pLink-2C und pLink-2BC transkribiert worden waren, nach einer elektrophoretischen Auftrennung gezeigt. Von den RNAs der Plasmide pLink-2C und pLink-2BC wurde hier fast ausschließlich an jenen AUGs initiiert, die in Figur 6 mit schwarzen Pfeilen gekennzeichnet sind; es ist nahezu ausschließlich die 37 kD bzw. die 47 kD Bande zu erkennen. Die Anwesenheit der Cap-Struktur führte zur Zurückdrängung der internen Initiation. Bei den Translationsprodukten der mit einer Cap-Struktur versehenen RNA der Konstruktion pHRV2/5'UTR gibt es kaum Unterschiede zu jenen mit der nicht- gecappten RNA. Die schwachen Banden bei 37 kD und 43 kD deuten auf eine Initiation bei den AUG Codons 449 und 611 hin. Die 5'UTR HRV2 kann daher, auch wenn gecappt, die Eigenschaft der 2C RNA, intern zu initiieren nicht zurückdrängen.

**Beispiel 2: In vitro System zur Detektion des Einflusses einer picornaviralen Proteinase auf die Translation cap-versehener RNAs in einem Kaninchenreticulozytenlysat**

**A) Expression der HRV2 2A Proteinase**

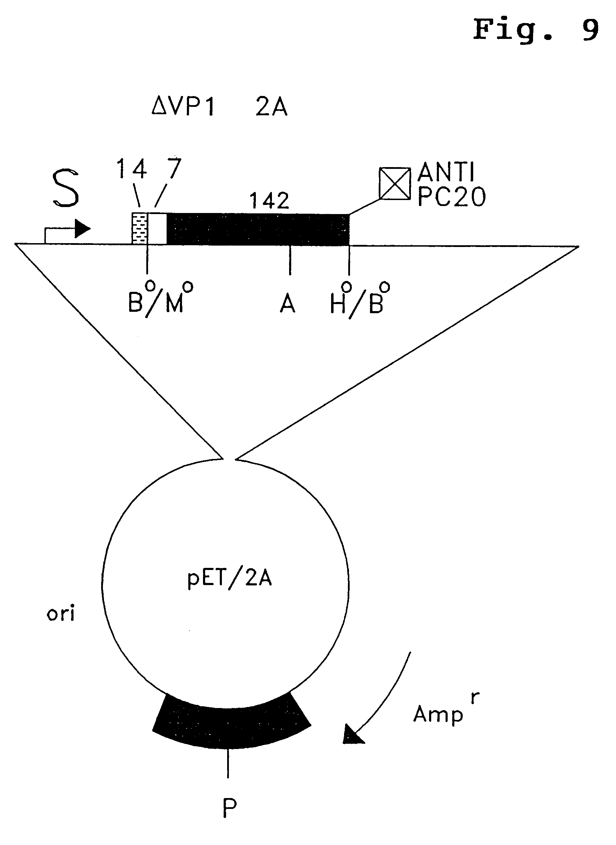

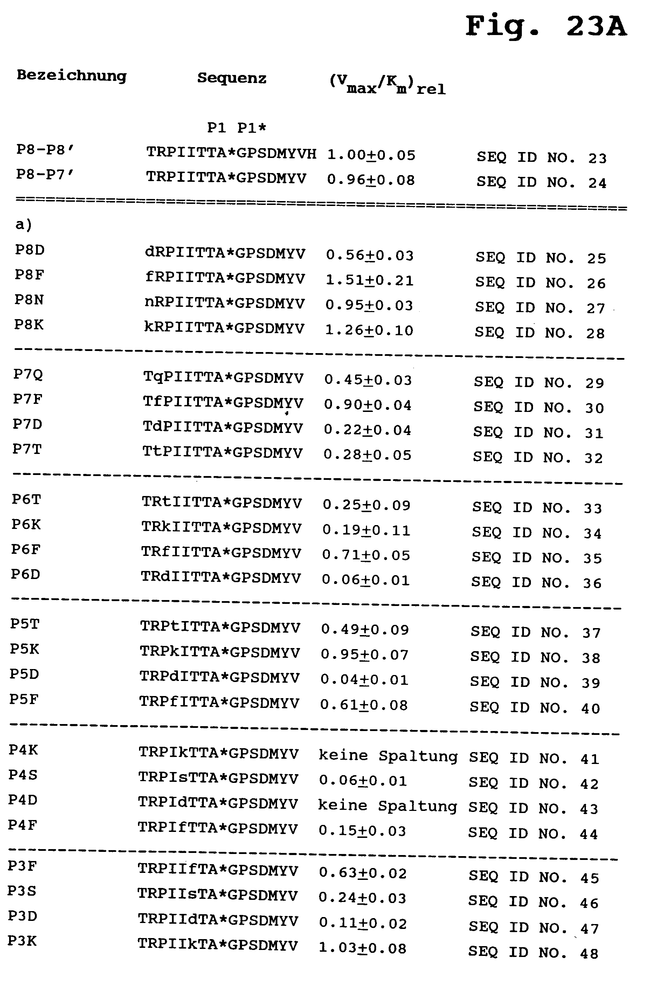

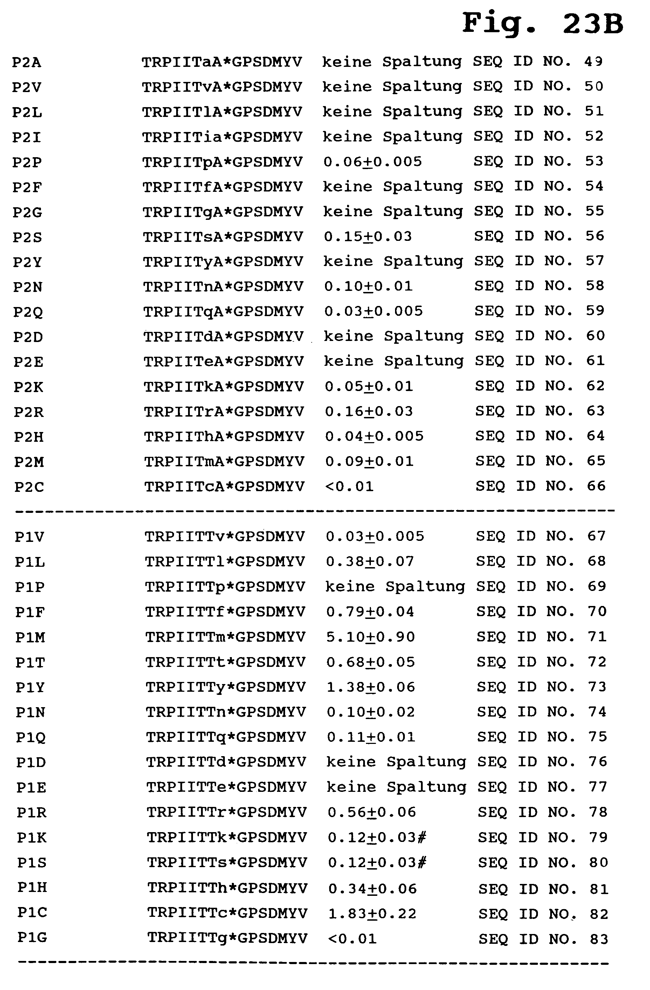

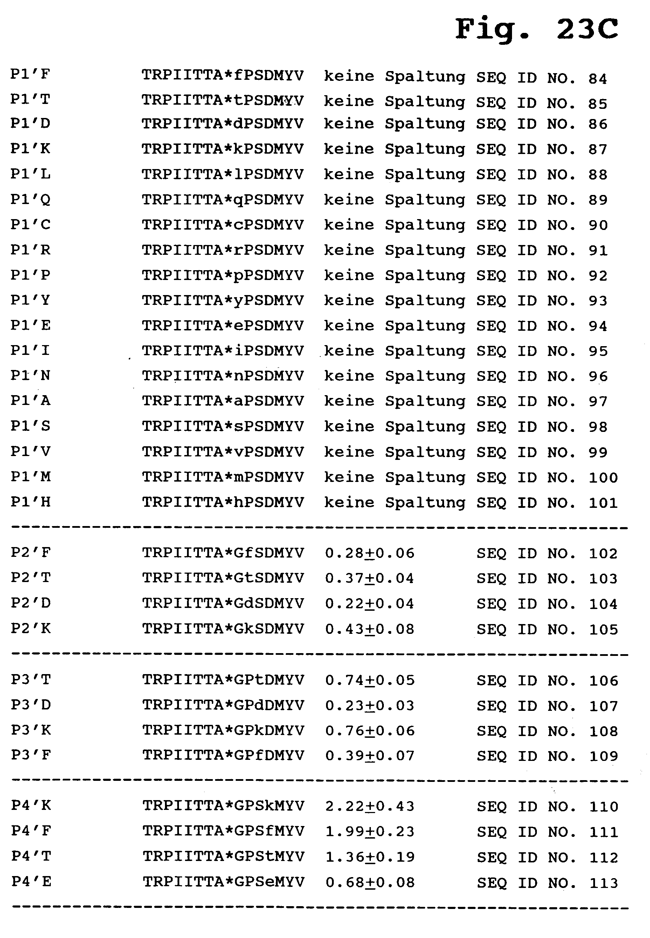

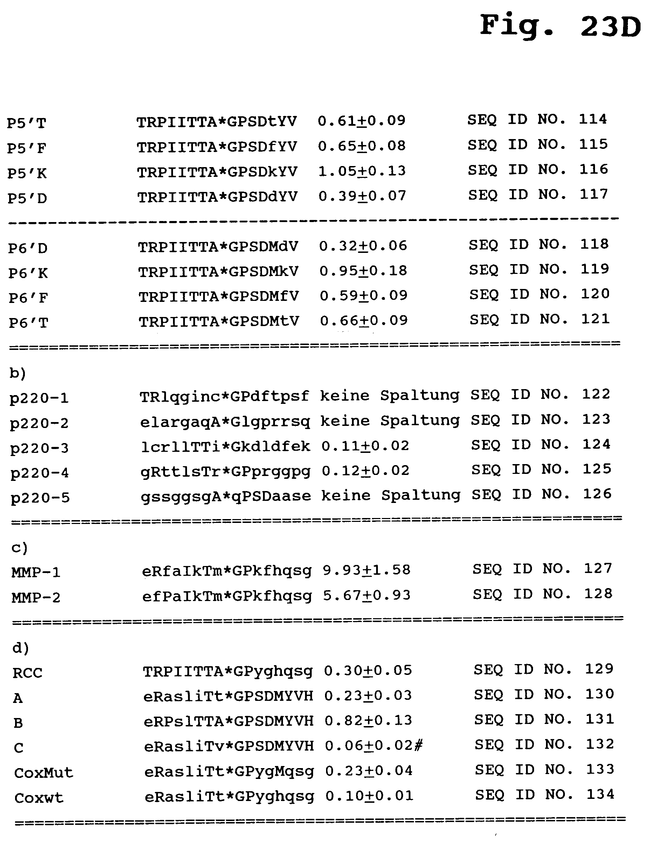

Zur Herstellung der 2A Proteine in E. coli wurde das Plasmid pET3b (Studier, F. et al. (1990) Meth. Enzymol. 185, 60-89; Novagen, Madison, USA) mit BamHI geschnitten und mit alkalischer Phosphatase behandelt. Doppelsträngige DNA des M13-Phagen M13/2A (Liebig et al. (1991) Proc. Natl. Acad. Sci. U.S.A 88, 5979-5983;) wurde mit MluI und HindIII geschnitten, das 448 bp Fragment (HRV2 Nukleotide 3138 bis 3586) aus einem Agarosegel isoliert und mit der BamHI geschnittenen pET3b Präparation ligiert. Nach Transformation in kompetente E.coli-Zellen wurde Plasmid (pET/2A) isoliert, dessen Struktur in Figur 9 abgebildet ist. Dieses Plasmid besitzt den Promoter für die T7 RNA Polymerase, die Translationssignale und die ersten 14 Aminosäuren des Gen 10 Proteins des T7 Phagen gefolgt von den letzten 7 Aminosäuren des Kapsidproteins VP1 von HRV2, allen 142 Aminosäuren der 2A Proteinase von HRV2 und 2 Stoppcodons. Die vollständige VP1- und P2A-Aminosäuresequenz des humanen Rhinovirus Serotyp 2 ist in Figur 23 dargestellt. Nach Transformation dieses Plasmids in den E. coli Stamm BL21(DE3)LysS (Novagen, Madison, USA) und Zugabe von IPTG (Isopropylthiogalactosid) wird dieser Expressionskassette exprimiert, da dieser Stamm das Gen für die T7 RNA Polymerase auf dem Chromosom unter der Kontrolle des lac Operator/Promoters besitzt. Die 2A Proteinase kann sich von der wachsenden Polypeptidkette abspalten; es entsteht daher ein reifes, aktives 2A Molekül vom Molekulargewicht 15 kD.

Hierzu läßt man eine Kultur (50ml) von E. coli BL21(DE3)LysS mit dem Plasmid pET/2A bis zu einer $OD_{590}$ von 0.6 wachsen; der Expressionsblock wird mit 0.2 mM IPTG induziert und die Kultur noch 2 Stunden bei 37°C inkubiert. Die Bakterien werden dann abzentrifugiert, in 0.7 ml Sonizierpuffer (150 mM NaCl, 50 mM Tris-HCl, pH 8.0, 5 mM DTT und 1 mM EDTA) aufgenommen und mit Ultraschall wie von Sommergruber et al. (1989) Virology 169, 68-77) gezeigt, aufgeschlossen. Die Zelltrümmer werden abzentrifugiert; die Proteine im Überstand werden mit 30 % Ammoniumsulfat gefällt und abzentrifugiert. Der 30 % Ammoniumsulfatniederschlag wird in 250 $\mu$l Sonizierpuffer aufgenommen und bei 4°C gelagert.

**B) Translation unter Zusatz der HRV2 2A Proteinase**

In Figur 8B ist die Auswirkung der Zugabe einer 2A Präparation auf die Translation einer gecappten RNA des Plasmides pLink-2C gezeigt. 3 $\mu$l eines solchen Extraktes wurden vor Zugabe der RNA zu einem Kaninchenreticulocytenextrakt zugesetzt und 10 min bei 30°C inkubiert. In Figur 8B, Spur 7, sind die Translationsprodukte mit einem vorbehandelten Kaninchenreticulocytenlysat zu ersehen. Obwohl eine gecappte RNA eingesetzt wurde, beobachtet man ein Translationsmuster einer ungecappten RNA; die Anwesenheit von 2A verändert offensichtlich die Eigenschaften des Kaninchenreticulocytenlysates.

**C) Inhibition der HRV2 2A Proteinase**

Daß die 2A Proteinase für diese Änderung verantwortlich ist, wird vom Ergebnis in Figur 8B, Spur 8 unterstützt. In diesem Experiment wurde die 2A Präparation zuerst mit 0,5 mM Elastatinal versetzt; es konnte gezeigt werden, daß Elastatinal ein IC50 für 2A von 0,05 mM hat (Sommergruber et al. (1992) loc. cit.) und daher ein sehr guter Inhibitor der 2A Aktivität ist. Die Translation einer gecappten RNA läuft in Kaninchenretikulocyten normal ab; die Inhibition von 2A verhindert die Änderung der Translationseigenschaften.

**Beispiel 3: Verfahren zur Reinigung der 2A Proteinase**

Zur Vereinfachung der Reinigung wurde ein Expressionsvektor entwickelt, der im Gegensatz zum Expressionsvektor pET/2A (Bsp. 2, Fig. 9) statt eines 7 Aminosäuren langen Vorläufers von VP1 einen 28 Aminosäuren langen Vorläufer besitzt. Durch diese Änderung wird der Unterschied in der Molekülmasse zwischen prozessiertem und nichtprozessiertem Fusionsprotein größer; dieser Unterschied erleichtert die Reinigung.

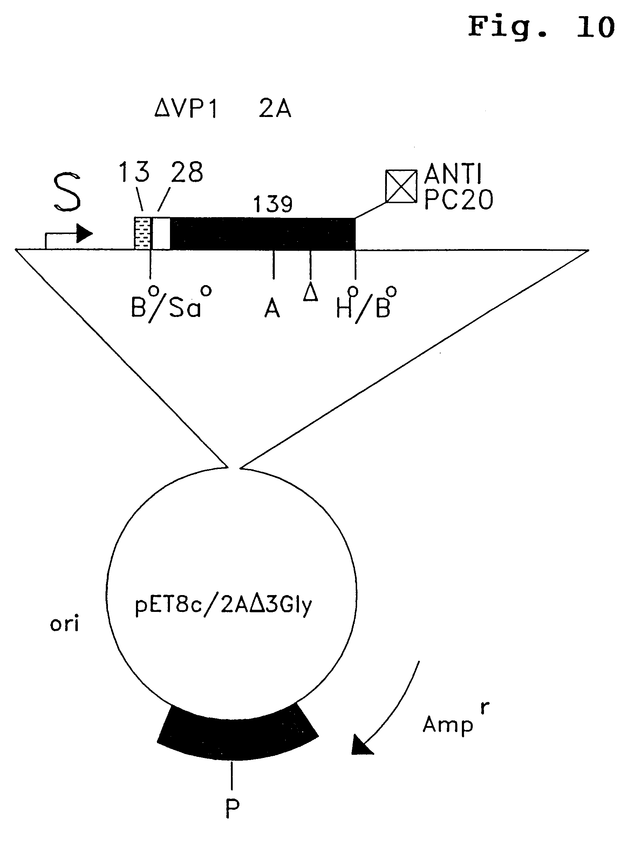

Der Vektor wurde folgendermaßen hergestellt: das Plasmid pET8c (Studier, F. et al., (1990) Meth. Enzymol. 185, 60-89; Novagen, Madison, USA) wurde mit BamHI geschnitten, die überlappenden Enden mit Hilfe des Klenow Fragmentes der E. coli DNA Polymerase I aufgefüllt und mit alkalischer Phosphatase behandelt. Doppelsträngige DNA des M13-Phagen M13/2AΔ3Gly (Liebig et al., (1991) Proc. Nat. Acad. Sci. U.S.A., 88, 5979-5983; WO 92/03559) wurde mit Sall und HindIII geschnitten, das 524 bp Fragment (darunter die HRV2 Nukleotide 3085 bis 3586) aus einem Agarosegel isoliert und mit dem linearisierten pET8c Vektor ligiert. Nach Transformation in E. coli HMS 174 (Novagen, Madison, USA) wurde ein Plasmid (pET8c/2AΔ3Gly) isoliert, dessen Struktur in Figur 10 abgebildet ist. Dieses Plasmid besitzt den T7 RNA Polymerase Promotor, den Translationsstart und die ersten 13 Aminosäuren des Gen 10 Proteins des T7 Phagen, gefolgt von drei Aminosäuren des α-Fragments, den letzten 28 Aminosäuren des Kapsidproteins VP1 von HRV2, den 139 Aminosäuren der enzymatisch nicht aktiven 2A Proteinase von HRV2 und 2 Stoppcodons.

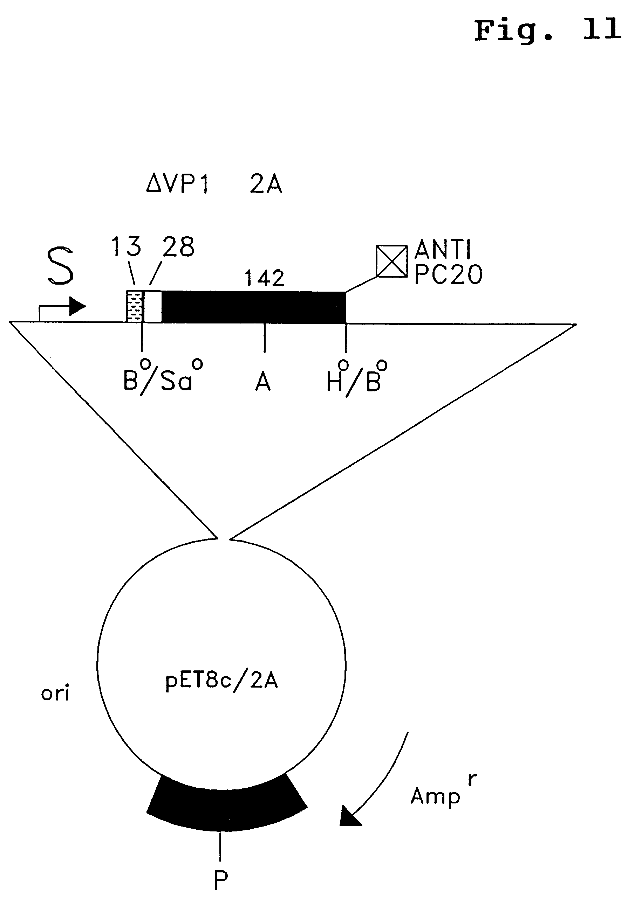

Das 2AΔ3Gly-Gen in pET8c/2AΔ3Gly wurde anschließend durch das Wildtypgen ersetzt. Das Plasmid pET8c/2AΔ3Gly wurde mit den Restriktionsenzymen PstI und ApaI geschnitten und das 3.5 kb Fragment aus einem Agarosegel isoliert. Das Plasmid pET/2A wurde ebenfalls mit PstI und ApaI geschnitten und das 1.0 kb Fragment aus einem Agarosegel isoliert. Nach Ligation dieser beiden Fragmente und Transformation in E. coli HMS 174 wurde ein Plasmid (pET8c/2A) isoliert, dessen Struktur in Figur 11 abgebildet ist. Dieses Plasmid besitzt den Promotor für die T7 RNA Polymerase, den Translationsstart und die ersten 13 Aminosäuren des Gen 10 Proteins des T7 Phagen, gefolgt von drei Aminosäuren des α-Fragments, den letzten 28 Aminosäuren des Kapsidproteins VP1 von HRV2, den 142 Aminosäuren der aktiven 2A Proteinase von HRV2 und 2 Stoppcodons. Nach Transformation des Plasmids in E. coli BL21(DE3)LysS bzw. LysE und Zugabe von IPTG wird dieser Expressionsblock exprimiert, da der Bakterienstamm BL21-(DE3)LysS bzw. -LysE auf dem Chromosom das Gen für die T7 RNA Polymerase unter der Kontrolle des lac Promotor/Operators besitzt. Die 2A Proteinase kann sich von der wachsenden Polypeptidkette abspalten; es entsteht daher ein reifes, aktives 2A Molekül mit einem Molekulargewicht von 15 kD.

### Anzucht

Zur Vorkultur wurden 230 ml LB-Medium (mit 30 mg/l Chloramphenicol und 100 mg/l Ampicillin) mit E. coli BL21(DE3)LysE, die das Plasmid pET8c/2A enthalten, angeimpft und über Nacht bei 34°C inkubiert. Anschließend wurden dreimal je 1 l LB-Medium (wie oben beschrieben) mit je 75 ml Vorkultur angeimpft und 2,5 Stunden bei 34°C und 130 rpm inkubiert. Nach Induktion mit IPTG (Endkonzentration: 0,3 mM) wurde die Bakteriensuspension für weitere vier Stunden bei 34°C und 130 rpm inkubiert. Die optische Dichte betrug vor der Induktion $OD_{595} = 0,55$ und nach vierstündiger Induktion $OD_{595} = 1,34$.

Anschließend wurden die Bakterien sedimentiert (6000 rpm, 10 min, 4°C) und mit 200 ml Waschpuffer (150 mM NaCl, 50 mM Tris-HCl, pH 8,0, 1 mM EDTA) gewaschen. Nach erneuter Zentrifugation wurden die Bakterien in 30 ml Puffer A (Waschpuffer mit 5 mM DTT und 5 % Glycerin) resuspendiert und durch Ultraschallbehandlung dreimal für je 5 bis 10 Sekunden lysiert. Zur Entfernung der Zelltrümmer wurden die lysierten Bakterien zentrifugiert (30 min, 16000 rpm), der Überstand auf 20 % Ammoniumsulfat gebracht und über Nacht bei 7°C belassen. Der Niederschlag wurde abzentrifugiert (30 min, 16000 rpm) und der Überstand auf 40 % Ammoniumsulfat gebracht. Nach drei Stunden bei 7°C wurde die Lösung zentrifugiert (30 min, 10000 rpm) und der Niederschlag in 30 ml Puffer A resuspendiert.

### Säulenchromatographie auf Mono-Q

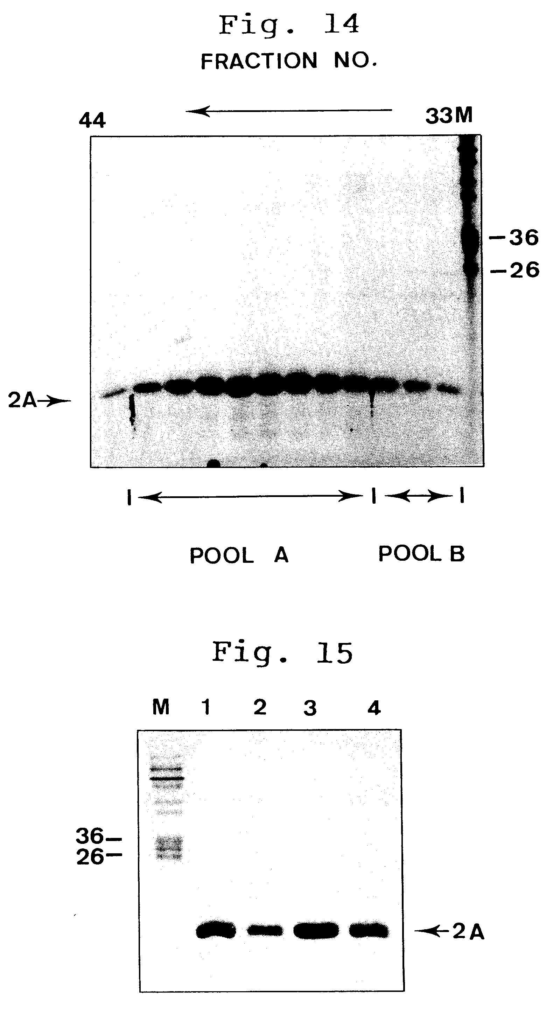

10 ml des in Puffer A resuspendierten Sediments wurden auf eine Mono Q HR 5/5 Säule aufgetragen und die Säule mit 5 ml Puffer A gewaschen. Die an die Mono-Q Säule gebundenen Proteine, u.a. die 2A Proteinase, wurden anschließend durch steigende Zugabe von Puffer B (Puffer B ist Puffer A mit 1 M NaCl statt 150 mM NaCl) von der Säule eluiert. Der dazu verwendete Gradient lautet: 0 % - 30 % Puffer B in einem Volumen von 5 ml, 30 % - 60 % Puffer B in einem Volumen von 30 ml und 60 % - 100 % Puffer B in einem Volumen von 5 ml. Die HRV2 2A Proteinase wurde in dem Bereich von 30 - 60 % Puffer B von der Anionen-Austauschersäule eluiert Dieser Vorgang wurde noch zweimal wiederholt. Auf einem 15 % Polyacrylamidgel wurden die einzelnen Fraktionen mittels Coomassie-Färbung auf ihren 2A Gehalt überprüft (Fig. 13, 14).

### Säulenchromatographie auf Superdex 26/60 (Pharmacia)

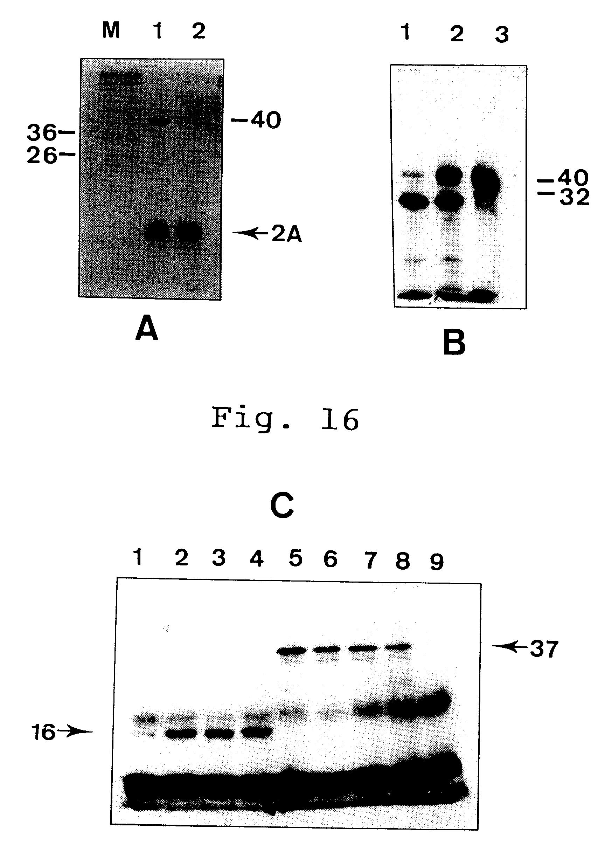

Die Fraktionen, die die Hauptmenge an 2A Protein enthalten, wurden vereinigt, auf eine Gelfiltrationssäule aufgetragen (Superdex 75 prepgrade Hiload 26/60) und mit Puffer C (Puffer C ist Puffer A mit 50 mM NaCl) eluiert. Nach Überprüfung der einzelnen Fraktionen auf 2A-Protein mittels PAGE nach Laemmli (Nature (1970) 227, 680-685) wurden die Fraktionen mit mindestens 95 % 2A-Anteil vereinigt und mittels einer Amicon Centriprep10-Zelle konzentriert (Fig. 14, 16).

Das derart gereinigte 2A Protein ist nun zu mindestens 98 % rein. Die Reinheit und Homogenität der Präparation wurde durch Isoelektrische Fokussierung, Kapillarelektrophorese, Chromatofokussierung auf einer Mono P-Säule und Analyse mittels Gelchromatographie überprüft.

## Aktivitätstest

Die Aktivität der reinen 2A Proteinase kann durch Spaltung eines synthetischen Peptids (Sommergruber et al. (1989) Virology 169, 68-77) mit entsprechender Proteinase 2A-Spaltstelle oder eines in vitro translatierten $^{35}$S markierten VP1/2A Substrates überprüft werden. Eine typische HRV2 2A Proteinase Präparation besitzt eine spezifische Aktivität von 9 Einheiten/mg, wobei eine Einheit die Menge an Protein ist, die 1 $\mu$mol des synthetischen Peptids pro Minute bei 25 °C spaltet. Zur Aufbewahrung wurde die 2A Proteinase in Lagerpuffer (100 mM NaCl, 4 mM Tris-HCl pH 8.0, 5 mM DTT, 0,05 % NaN$_3$) überführt. Dazu wurde das 2A Konzentrat auf eine Waters Protein Pak 125 HPLC-Gelfiltrationssäule jeweils in 200 $\mu$l Aliquots aufgetragen und mit Lagerpuffer eluiert. Die Fraktionen, die das 2A Protein enthielten, wurden vereinigt und mit einer Amicon Centriprep10-Zelle konzentriert.

## Beispiel 4: In vitro System zur Detektion des Einflusses einer picornaviralen Proteinase auf die Translation cap-versehener RNAs in einem HeLa-Zellextrakt

Zur Herstellung eines HeLa-Zellextraktes wurde eine 1 l HeLa-Suspensionkultur (z.B. HeLa S3 (03-157, FLOWLABS) oder HeLa Ohio (03-147, FLOWLABS)), (ca. 4 x 10$^5$ Zellen/ml) in halb-logarithmischer Phase abzentrifugiert (20 min, 1200 rpm), mit isotonischem Puffer gewaschen, durch mehrmaliges "Douncen" (40- bis 50-mal) aufgebrochen und die Zelltrümmer durch Zentrifugation (3 min, 3000 rpm) entfernt. Der Überstand wurde erneut zentrifugiert (20 min, 10000 rpm), das Pellet verworfen und der neue Überstand nochmals zentrifugiert (20 min, 13000 rpm). Der erhaltene Überstand wurde dann bei 4 °C gegen Dialysepuffer (zweimal je 400 ml) dialysiert und nochmals 20 Minuten bei 13000 rpm zentrifugiert (20 min, 13000 rpm). Der Überstand (S 20-Extrakt) wurde portioniert und in flüssigem Stickstoff gelagert.
isotonischer Puffer: 10 mM Hepes, pH 7,9, 140 mM KCl, 1,5 mM MgCl$_2$, 1 mM EDTA, 0,5 mM PMSF.
Dialysepuffer: 0,1 M KCl, 20 % Glycerin, 20 mM Hepes, pH 7,9, 0,2 mM EDTA, 0,5 mM DTT, 0,5 mM PMSF.
Im Gegensatz zu den bekannten Methoden enthält der Dialyse-Puffer keine Magnesiumionen.
1 ml Portionen des S 20-Extraktes wurden dann mit Micrococcal Nuclease (Boehringer Mannheim; Nr. 107921) behandelt, um die endogene RNA abzubauen: Zunächst wurde der Extrakt zentrifugiert (15 min, 4 °C, 13000 rpm) und das Pellet verworfen. Zum Überstand (1 ml) wurden zugegeben:
1,2 $\mu$l 1 M CaCl$_2$
1,5 $\mu$l 1 M MgOAc
16,0 $\mu$l Micrococcal Nuclease (15 U/$\mu$l).
Das Gemisch wurde 15 min. bei 20 °C inkubiert und die Reaktion durch Zugabe von EGTA (Endkonzentration 3 mM) gestoppt.
Nach Zugabe von 1/7 Volumen 80 % Glycerin und kurzem Abzentrifugieren, wurde der so vorbehandelte Extrakt wiederum portioniert und in flüssigem Stickstoff gelagert.
Ein typischer in vitro Translationssatz mit einem so präparierten und behandelten Extrakt lautet:
10 $\mu$l Extrakt
2 $\mu$l $^{35}$S-Met
1 $\mu$l 20 x Energy Mix: 20 mM ATP, 2 mM GTP, 40 mM DTT, 0,2 M Kreatinphosphat, 1 mM Aminosäuren (ohne Methionin)
1 $\mu$l 1,3 M Kaliumacetat
1 $\mu$l 30 mM Magnesiumacetat
1 $\mu$l SHT Mix (48 mM Spermidin; 0,26 M Hepes; 0,48 $\mu$g/ml tRNA)
1 $\mu$l 5 mg/ml Kreatinkinase
2 $\mu$l H$_2$O
1 $\mu$l RNA
Die Reaktionslösung wurde eine Stunde bei 30 °C inkubiert und durch Zugabe von 1 $\mu$l 100 mM Methionin gestoppt.
Die Translation von zwei RNAs (Kaninchen Globin RNA isoliert aus Retikulozyten, d.h. eine vollständig gecappte RNA) und eine in vitro transkribierte, nicht gecappte pHRV2/5'UTR-2C RNA sind in Figur 16 abgebildet. Die 5'-Translation der Globin RNA liefert ein Produkt von 16 kD, das dem Molekulargewicht von Kaninchen β-Globin entspricht. Von der nicht-gecappten pHRV2/5'UTR-2C ist ebenfalls nur eine Bande sichtbar, die einem Molekulargewicht von 37 kD entspricht.

Die Translationssignale von der nicht gecappten RNA von pHRV2/5'UTR-2C werden also richtig erkannt, im Gegensatz zu der Situation in Translationsextrakten aus Kaninchenretikulozyten.

Die Zugabe einer reinen HRV2 2A Proteinase Präparation zur Translation dieser beiden RNAs wurde anschließend untersucht. Die Proteinasepräparation ist in Fig. 16A gezeigt; die Aktivität des Enzyms auf ein in vitro translatiertes, $^{35}$S markiertes VP1/2A Substrat ist ebenfalls in Fig. 16B gezeigt. Zugabe von 2 μg dieses Enzyms führt zu einer Reduktion der Translation der Globin RNA (Fig. 16C, Spur 1). In Spur 3 wurde nur der Puffer getestet; hier wurde die Translation von Globin-RNA nicht beeinflußt. In Spur 4 wurde die 2A Proteinase mit 0,5 mM Elastatinal vorinkubiert; die 2A-Wirkung auf die Translation wird inhibiert.

Die Versuche zeigen, daß die 2A Proteinase zu einer 2 bis 3 fachen Reduktion der Translation einer gecappten RNA führt, nicht aber bei einer RNA mit der rhinoviralen 5'UTR (Figur 16C, Spur 5-8). Das System stellt die in vivo Lage dar, in der die Anwesenheit einer 2A Proteinase zu einem "host-cell shut-off" führt und die Translation wirtseigener RNAs abschaltet. Die Translation der viralen RNAs bleibt von diesem "Block" verschont.

## Beispiel 5: Untersuchung der in vitro Translation von pLink-2C RNAs in der Anwesenheit von Oligonukleotiden

Im Beispiel 2 wird der Unterschied in der Translation in Kaninchenreticulozytenextrakten von pLink-2C RNAs mit und ohne Cap-Struktur klar gezeigt. Es stellt sich die Frage, warum zusätzliche Produkte bei der Translation einer nicht mit Cap-Struktur versehenen RNA entstehen. Man kann sich zwei Hypothesen vorstellen. In der ersten binden die Ribosomen am 5'Ende, initiieren nur zum Teil am ersten AUG, zum Teil am AUG das zum 33 kD Produkt führt, zum Teil am AUG das zum 28 kD führt und so weiter. In der zweiten Hypothese binden die Ribosomen am jeweiligen AUG. Um zwischen diesen Hypothesen zu unterscheiden, wurden folgende Versuche durchgeführt.

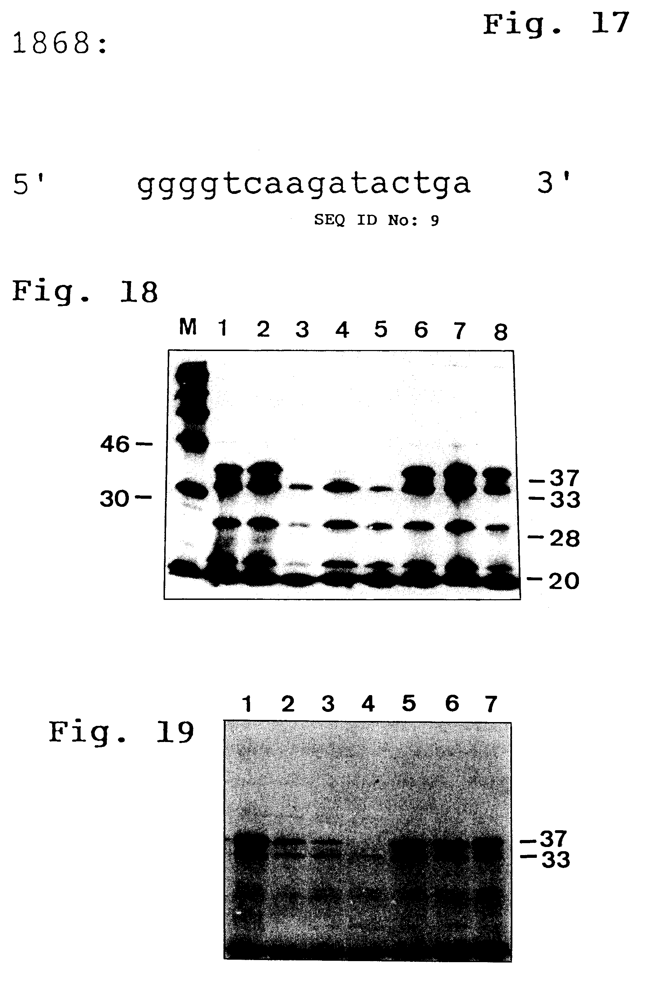

Wie im Beispiel 2 beschrieben wurden in vitro pLink-2C RNAs, mit und ohne Cap-Struktur versehen, transkribiert. Zu diesen RNAs werden steigende Mengen von den Oligonukletiden 1092 (Figur 4, SEQ ID No: 6) und 1828 (Fig. 18) gegeben. Das Oligonukleotid 1092 ist komplementär zur Sequenz um das in Figur 7 mit schwarzem Pfeil gekennzeichnete AUG; die Sequenz des Oligonukleotids 1868 (SEQ ID No: 9) ist von dem Plasmid pSVL (Pharmacia; siehe Beispiel 2) abgeleitet. Das Oligonukleotid sollte daher nicht an die pLink-2C RNAs binden. Die RNA-Oligonukleotidgemische wurden 10 Minuten bei 85°C, 10 Minuten bei 65°C inkubiert und dann langsam auf Raumtemperatur abgekühlt. Anschließend wurden sie wie im Beispiel 2 beschrieben für die in vitro Translation eingesetzt. Das Ergebnis ist in Fig. 19 ersichtlich. Die Zugabe von steigenden Mengen von Oligonukleotid 1092 zu der nicht gecappten pLink-2C RNA führt zu einer Abnahme der Translation der 37 kD Bande, wobei die Mengen der anderen Produkte nicht beeinflußt werden. Die Translation der 37 kD Bande von der mit einer Cap-Struktur versehenen pLink-2C RNA wird ähnlich reduziert; andere Produkte entstehen nicht. Eine geringfügige Translation der 33 kD Bande wurde, unabhängig der Oligonukleotidkonzentration, ebenfalls beobachtet. Sie entsteht durch einen geringfügigen Anteil von nicht gecappter pLink-2C RNA in der Präparation. Die Zugabe des Oligonukleotids 1868 beeinflußt die Translation der RNAs nicht.

Da das Oligonukleotid 1092 komplementär zu der Sequenz um das erste AUG ist, können die Ribosomen in seiner Anwesenheit an dieses AUG nicht binden, und die Translation der 37 kD Bande wird reduziert. Im Falle der nicht gecappten RNA können die Ribosomen weiter an interne AUGs binden und Proteinketten initiieren. Daher müssen die Ribosomen direkt an die jeweiligen AUGs binden können: die zweite Hypothese wird unterstützt! Im Falle der mit einer Cap-Struktur versehenen pLink-2C RNA wird die Translation unterbunden; die Ribosomen scheinen hier an die Cap-Struktur zu binden, können aber nicht initiieren, da die Anwesenheit des Oligonukleotids dies verhindert.

Die Cap-Struktur verhindert die Translation der kleineren Produkte, indem die Ribosomen die Cap-Struktur erkennen.

## Beispiel 6: Herstellung von GR6

Für die Herstellung des Plasmids GR6 (Beispiel 1) wurden drei Plasmide herangezogen, nämlich p773 (HRV2 Nukleotide 562-1012; Skern, T. et al. (1985) Nucl. Acids Res. 13, 2111 - 2126), p19 (HRV2 Nukleotide 19-5100) (Duechler et al. (1989) loc. cit.) und p860. p860 enthält die HRV2 Nukleotide 660 bis 1400 in der PstI Schnittstelle von pUC9; die Orientierung ist so, daß Nukleotid 1400 neben der EcoRI Schnittstelle des Polylinkers liegt.

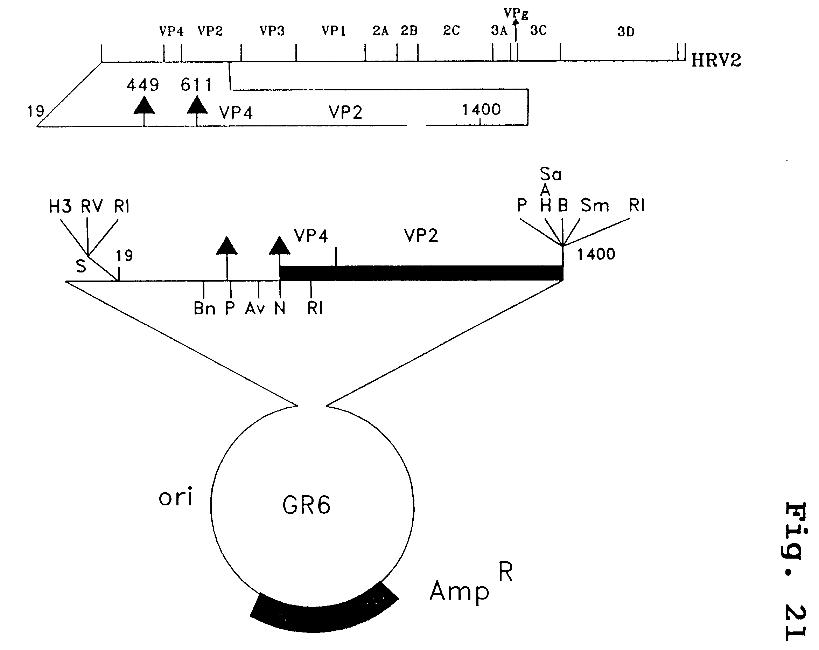

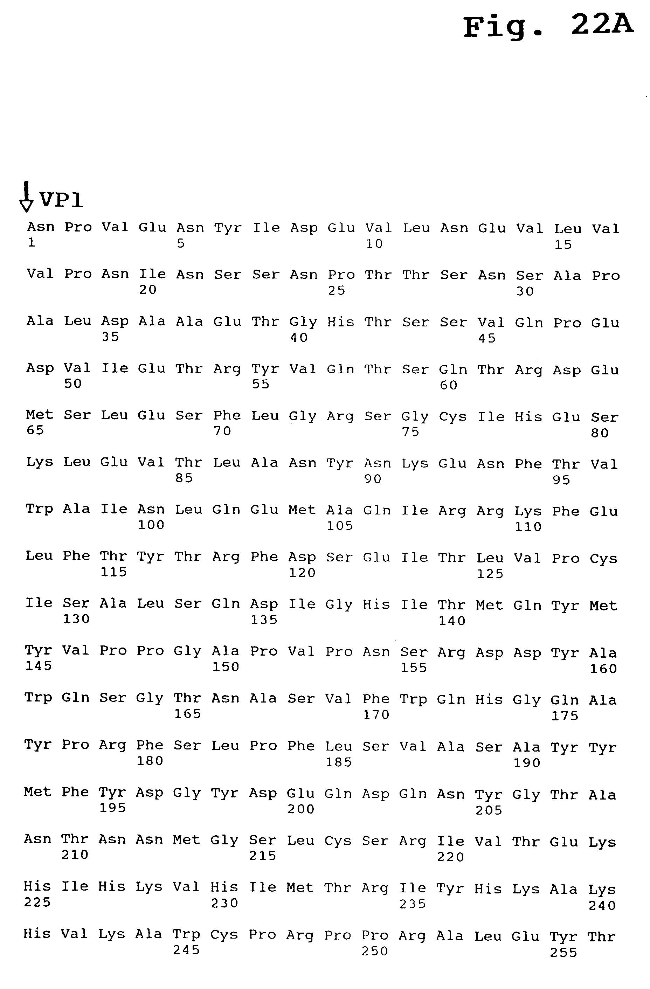

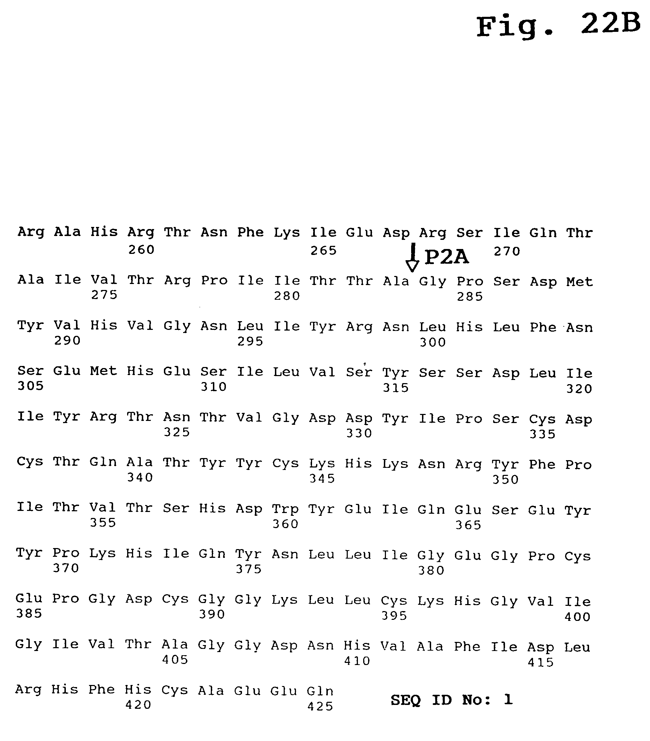

Das Plasmid pGEM2 wurde mit den Restriktionsenzymen HindIII und BamHI geschnitten und mit alkalischer Phosphatase aus Kälberdarm dephosphoryliert; ebenfalls mit HindIII und BamHI wurde das Plasmid p773 geschnitten. Das dadurch freigesetzte HRV2 Insert wurde aus einem Agarosegel eluiert und mit dem mit HindIII/BamHI linearisierten pGEM2 Vektor (Promega) ligiert. Nach Transformation in kompetente E.coli-Zellen wurde das resultierende Plasmid (GR1) isoliert. GR1 wurde dann mit den Restriktionsenzymen HindIII und NcoI und mit alkalischer Phosphatase behandelt; ebenfalls mit HindIII und NcoI wurde das Plasmid p19 geschnitten. Dadurch entsteht ein 361 bp HindIII/NcoI Fragment mit den Nukleotiden 248 bis 609 der HRV2 Genkarte. Dieses Fragment wurde aus einem Agarosegel eluiert und mit dem wie oben beschrieben behandelten GR1 Plasmid ligiert. Das resultierende Plasmid (GR2) wurde mit dem Restriktionsenzym EcoRI geschnitten und mit alkalischer Phosphatase aus Kälberdarm behandelt; ebenso wurde das Plasmid p860 mit EcoRI geschnitten. Das dabei entstehende 680 bp Fragment von der EcoRI Stelle bei Nukleotid 743 der HRV2 Genkarte bis zur EcoRI Stelle des pUC9 Polylinkers (d.h. zu Nukleotid 1400 der HRV2 Genkarte) wurde isoliert und mit dem mit EcoRI geschnittenen, mit alkalischer Phosphatase behandelten GR2 Vektor ligiert. Nach Transformation in kompetente E.coli-Zellen wurde das entsprechende Plasmid (GR5) isoliert. Das Plasmid GR5 wiederum wurde mit dem Restriktionsenzym HindIII geschnitten und mit alkalischer Phosphatase aus Kälberdarm dephosphoryliert; ebenso wurde das Plasmid p19 mit HindIII behandelt. Das gebildete 240 bp Fragment von der HindIII Schnittstelle im Polylinker bis zur HindIII Schnittstelle bei Nukleotid 248 der HRV2 Genkarte (d.h. Nukleotid 19 der HRV2 Genkarte bis 248) wurde mit dem linearisierten GR5 Vektor ligiert. Nach Transformation in kompetente E.coli-Zellen wurde ein Plasmid isoliert, dessen Struktur in Figur 22 abgebildet ist (GR6). Nach der Linearisierung mit dem Restriktionsenzym BamHI entsteht bei der in vitro Transkription mit T7 RNA Polymerase eine RNA die von der Transkriptionstelle bis zur EcoRI Stelle, dem Beginn der HRV2 Sequenz, 28 Nukleotide umfaßt, gefolgt von den Nukleotiden 19 bis 1400 der HRV2 Sequenz (Figur 21).

**Beispiel 7: Ermittlung der Substratspezifität der Proteinase 2A von HRV2 (HRV2 2A) mit Peptidsubstraten**

Aufgrund bekannter Studien mit einer reifen, rekombinanten Proteinase 2A von HRV2 (HRV2 2A) konnte gezeigt werden, daß besonders die Position P2, überraschenderweise nicht aber Position P1, für die Spaltbarkeit verantwortlich ist. Die Verwendung von C- und N-terminal verkürzten Peptidsubstraten weist auf weitere zusätzliche wichtige Positionen in der P-Region ("upstream" von der Spaltstelle) und der P1- und P2-Position hin (Sommergruber, et al. loc. cit.). Um die Substratspezifität bzw. Erkennungssequenz von HRV2 2A genauer zu charakterisieren (insbesondere der P1-Position und der P8-P3-Region), wurden 15-mer Oligopeptide synthetisiert, die jeweils, einen Aminosäureaustausch in den Positionen P8 bis P6' aufweisen. Dabei wurde in den einzelnen Positionen jeweils gegen eine saure, basische, hydrophobe und polare Aminosäure ausgetauscht. Im Fall der Positionen P2, P1 und P1' wurden mehrere Aminosäuresubstitutionen vorgenommen.

Sämtliche Peptide wurden auf einer Milligen (Modell 9050) oder einer Zinsser (SMPS 350) Peptidsynthese-Apparatur nach der Fmoc-Methode (Fluoren-9-ylmethoxy-carbonyl; Merrifield (1963) J. Am. Chem. Soc. 85, 2149-2154) über aktivierte Ester oder HOBt/DIPCDI synthetisiert. Als Festphase für die Peptidsäuren wurde wahlweise ein Polydimethylacrylamid-Harz auf Kieselgurbasis (Pepsyn KA, Milligen/Biosearch), ein TentaGel S AC (Rapp Polymeres), ein Sasrin-Harz oder ein Wang-Harz (Bachem, Schweiz); für Peptidamide ein TentaGel AM (Rapp Polymere) verwendet. Die Fmoc-gekoppelten Aminosäurederivate der freien und der Pentafluorphenylester (Opfp-Ester) wurden von Bachem Ag (Schweiz) und Novabiochem (Schweiz) bezogen. Die Seitenkettenprotektion war wie folgt: Arg(Mtr), Arg(Pmc), Asp(tBu), Cys(Trt), Glu(tBu), His(Boc), Ser(tBu) und Thr(tBu). Die Peptide wurden durch TFA-Behandlung vom Harz gespalten; die Seitenketten durch dreistündige Inkubation in TFA/Thioanisol/Thiocresol (v/v 95:3:2) entschützt. Die komplette Entfernung der Mtr-Gruppe bei Arg benötigte eine einstündige Säurebehandlung bei 50°C. Die Peptide wurden anschließend in Essigsäure gelöst, in Diethylether präzipitiert, zentrifugiert und lyophilisiert. Analytische HPLC Studien (Waters System) wurden in einem linearen Gradienten von H2O/TFA = 100:0,1 und Acetonitril/TFA = 100:0,1 (im folgenden B genannt) auf einer Bakerbond C18 Säule ("wide pore") durchgeführt. Die semipräparative Trennung wurde unter den gleichen Bedingungen auf einer Hibar LiChrosorb RP18 Säule (250/25 Merck) vorgenommen. Die Charakterisierung aller Peptide erfolgte mit Hilfe der "Plasma Desorptions Mass Spectroscopy" (PDMS) auf einem BIO ION BIN K20 Gerät.

Die Substratspezifitätsstudien, wie auch alle anderen Studien in diesem Beispiel, wurden mit einer reifen, rekombinanten HRV2 2A durchgeführt. Mittels Kompetitionsstudien unter Verwendung eines 16-mer Wildtyp-Peptidsubstrats (P8-P8') als Referenzpeptid wurde der jeweilige (Vmax/Km)rel-Wert für das mutierte Peptidsubstrat ermittelt. Das Peptid P8-P8' (TRPIITTA* GPSDMYVH (SEQ ID NO. 23)) entspricht der nativen Spaltstelle von HRV2 2A im Polyprotein (TRPIITTA ist der C-terminale Teil von VP1 und GPSDMYVH der N-Terminus von HRV2 2A). Dieses Peptid wird von der rekombinanten HRV2 2A intermolekular korrekt zwischen Ala und Gly prozessiert (Km-Wert: $(5,4\pm0,02) \times 10^{-4}$ Mol/l; Sommergruber et al., loc cit). Der übliche Testansatz zur Bestimmung der (Vmax/Km)rel Werte war wie folgt: 500 $\mu$l einer wässrigen Lösung von Puffer A (150 mM NaCl, 50 mM TrisHCl, pH 8, 5mM DTT, 5% Glycerin und 1mM EDTA) mit 100 $\mu$M P8-P8' und 100 $\mu$M des jeweiligen mutierten Peptidsubstrats wurden bei 34°C 10 min präinkubiert. Die Kompetitionsreaktion wurde durch Zugabe von 1 $\mu$l einer reinen (ca. 95%) HRV2 2A Stammlösung (2mg/ml in Puffer A + 0,05% $NaN_3$) gestartet. 50 $\mu$l Proben wurden jeweils nach 30, 60, 90, 120 und 150 sek bzw. nach 2, 4, 8, 16 und 32 min entnommen, in ein ULTRAFREE-MC Röhrchen (10,000 NMWL; Fa. Millipore) mit jeweils 250 $\mu$l 0,5 M $HClO_4$ transferriert (Reaktionsstop), gemischt und auf Eis gestellt (mindestens 15 min). Anschließend wurde die Lösung 30 min bei 14K, 4°C in einer SIGMA Eppendorfzentrifuge zentrifugiert. Das Filtrat wurde direkt zur HPLC Analyse eingesetzt. Peptidsubstrate und deren Spaltprodukte wurden entweder auf einem Kurzgradienten (5,5 min; 5,4% B/min; Merck Supersphere C18 Säule, 4x50 mm) oder einem Langgradienten (34,5 min; 0,8% B/min; Bakerbond WP C18 Säule, 4,6x250mm) aufgetrennt. Die Messung der UV Absorption wurde bei 210 nm und 280 nm durchgeführt. Die Spaltprodukte wurden mittels Komigration von Referenzpepetiden und/oder durch Proteinsequenzierung (Hunkapiller und Hood (1983) Science 219, 650-659) identifiziert. Der prozentuelle Anteil der Spaltung für jeden Zeitpunkt wurde durch Vergleich der Flächen der C- und N-terminalen Produktpeaks mit dem des verbliebenen ungespaltenen Peptidsubstrats ermittelt. Zusätzlich wurden die Daten graphisch ausgewertet, um etwaige Abweichungen vom klassischen Kurvenverlauf zu erkennen (z.B. sigmoide Kurve, allosterische Effekte etc.). Bei keinem der in Abb. 23 angeführten mutierten Peptidsubstrate konnte solch ein Effekt beobachtet werden. Für jedes Substrat und dessen Spaltprodukte war die Gesamtfläche der Peaks unabhängig vom Maß der Spaltung (< 5%). Für zwei Peptidsubstrate 1 und 2 ergeben sich in einem Kompetitionsassay nach Pallai et al. (Pallai et al. (1989) J. Biol. Chem. 264, 9738-9741) die (Vmax/Km)rel-Werte:

$$(Vmax/Km)1/(Vmax/Km)2 = log(1-F1)/log(1-F2)$$

wobei F der Anteil des umgesetzten Substrats ist. In allen Kompetitionsstudien waren Substrat- und Produktpeaks entweder auf dem langen oder dem kurzen bzw. auf beiden Gradienten über HPLC-Analyse voneinander trennbar. Alle Peptide, die nach einer Inkubationszeit von 32 min mit HRV2 2A keine Spaltprodukte zeigten, wurden in einem getrennten Ansatz 3 Stunden mit HRV2 2A inkubiert; die Abwesenheit von Spaltprodukten wurde in der Tab. 1 mit "keine Spaltung" angegeben. Alle (Vmax/Km)rel-Werte wurden in drei voneinander unabhängigen Kompetitionsexperimenten ermittelt. In fast allen Fällen lagen die Werte innerhalb der 20% Grenze. Nur bei den in Abb. 23 mit einem (#) versehenen Werten lag die Reproduzierbarkeit der Daten etwas höher als 20 %, da sich die Peaks ihrer Spaltprodukte sowohl auf dem kurzen als auch auf dem langen Gradienten überschnitten.

Von älteren Studien her war bekannt, daß Thr in P2 eine sehr wichtige Rolle bei der Substraterkennung spielt (Sommergruber et al., loc cit). Diese Daten sind zusätzlich ebenfalls in Abb. 23 aufgelistet. Nur einige wenige Aminosäuren können in P2 eingesetzt werden (z.B. Arg, Ser, Asn oder Met), ohne die Effizienz der Spaltung drastisch zu erniedrigen bzw. die Spaltung zu verhindern.

Von geringerer Bedeutung für ein effizientes Prozessieren von Peptidsubstraten ist die Position P1; bis auf wenige Ausnahmen (z.B. Pro, Asp und Glu) werden sämtliche Aminosäuresubstitutionen toleriert. 6 Peptide mit Änderungen in P1 (P1G, P1V, P1N, P1S, P1Q und P1K) zeigten eine schlechte Spalteffizienz (etwa 1/10 und darunter); P1L, P1F, P1T , P1H und P1R dagegen eine 1,5- bis 2,5-fache Reduktion in ihrer Spaltbarkeit. P1Y, P1M und P1C wiederum weisen etwa einen 1,5- bis 5-fachen Anstieg der Spalteffizienz auf. P1Y erscheint deshalb so interessant, da Tyr/Gly die Spaltstelle für alle 3 Poliostämme repräsentiert. Generell dürfte die zu P1 analoge Position S1 im Enzym daher eher eine "offene" Wechselwirkungsstelle darstellen. Nicht erlaubt sind in dieser Position Pro, Asp und Glu.

Die vorliegenden Daten über die Position P1' zeigen eine noch wesentlich höhere Restriktion für diese Position als z.B. bei Position P2. In keinem Fall einer Substitution von Gly konnte eine Spaltung nachgewiesen werden.

Weitere Substitutionen in der P'-Region ("downstream") erbrachten nur mehr im Fall der Position P2 (Pro) eine merkliche Beeinflussung der Spaltbarkeit (Abb. 23: Peptide P2'F, P2'T, P2'D und P2'K) mit einer Reduktion um das 2,5- bis 5-fache. Alle weiteren Substitutionen bis zur Position P6 zeigen nur mehr geringere Effekte. Diese Daten werden von den kürzlich mit kürzeren Wildtyppeptidsubstraten durchgeführten Kompetitionsstudien unterstützt, indem gezeigt werden konnte, daß Deletionen vom C-Terminus des Peptids bis zu P2' keinen dramatischen Effekt auf die Spaltbarkeit ausüben (Sommergruber et al. (1992) loc cit.).

Ganz anders ist die Situation bei N-terminal verkürzten Peptidsubstraten. P6-P8' konnte gegenüber P8-P8' nur mehr ein Drittel so effizient gespalten werden. P5-P8' und P4-P8' Substrate waren sogar 6- bzw. 10-fach in ihrer Spaltbarkeit gegenüber P8-P8' reduziert (Sommergruber, et al., loc cit). Von den in Fig. 23 gezeigten (Vmax/Km)rel Werten der in der P-Region substituierten Peptide zeigen vor allem die P4-Mutanten (P4K, P4S, P4D und P4F) drastisch reduzierte Werte. Durch die Verwendung der von Coxsackie-virus abgeleiteten Spaltpeptide (Peptide A, B, C und CoxMut) konnte außerdem demonstriert werden, daß ein Austausch von Ile gegen Leu in Position P4 nicht für die Reduktion der Spalteffizienz verantwortlich gemacht werden kann (Peptid B mit einem relativen Vmax/Km-Wert von 0,82). Das bedeutet, daß ein konservativer Austausch von Ile gegen Leu keinen nennswerten Einfluß auf die Spaltbarkeit eines Substrates ausübt, jedoch andere Aminosäuren an dieser Stelle nicht akzeptiert werden (siehe Fig. 23).

Einen geringeren, wenn auch noch deutlich erkennbaren Effekt haben die Substitutionen in der Position P6 und P7. Bis auf P7F, P7Q und P6F zeigen die Peptidsubstrate eine 4 bis 5 fach reduzierte Spaltbarkeit (siehe Fig. 23). Umgekehrt aber weist ein Peptidsubstrat (Coxwt B4), welches aus der P-und P'-Region von Coxsackie B4 besteht, einen (Vmax/Km)rel Wert von nur 0,15 gegenüber einer Mutante (CoxMut) mit einem Pro in P6 von 0,82 auf. Mit Ausnahme von Peptidsubstrat C (Val in P1) werden die von Coxsackie B4 abgeleiteten Peptidsubstrate von HRV2 2A mit gleicher Effizienz umgesetzt. Bei dieser Peptidgruppe dürfte das Pro in P6 eine größere Rolle als das Arg in P7 spielen. Zu bemerken wäre jedoch bei den Peptiden A, C und CoxMut, daß die Aminosäure in P1 gegenüber der in Peptid B (weist ein Ala in P1 auf; entspricht dem Wildtyp) eine ungünstige Ausgangssituation für die Spaltung der Peptide A, C und CoxMut entstehen läßt (siehe P1-Mutanten in Fig. 23). Es ist daher nicht auszuschließen, daß der negative Einfluß möglicherweise nicht alleine durch den Austausch von Pro in P6 zustandekommt.

Um nun die Bedeutung der Position P7 und P6 näher zu beleuchten und um den Beweis zu erbringen, daß tatsächlich nur dieses Motiv zur Spaltung eines an allen anderen Positionen statistisch substituierten Peptidsubstrats ausreicht, wurden zwei weitere Peptide MMP1 und MMP2 synthetisiert und ihre (Vmax/Km)-rel Werte bestimmt. MMP1 besitzt in P7 ein Arg, in P4 ein Ile, in P2 ein Thr, in P1' ein Gly und in P2' ein Pro; MMP2 ist gegenüber MMP1 dermaßen verändert, daß kein Arg in P7 jedoch ein Pro in P6 anzutreffen ist. Wie aus Fig. 23 ersichtlich werden beide Peptide um das 10- bzw. 5,5-fache besser gespalten als das Wildttyppeptid P8-P8'. Das Fehlen von Arg in Position P7 in MMP2 reduziert dabei die Spalteffizienz bereits um das 2-fache. Vergleicht man zusätzlich die Spaltregionen anderer Rhino- und Enteroviren, so erkennt man, daß in fast allen Fällen in Position P7 (HRV2, HRV1B, HRV85, HRV14, Cox B1, Cox B4), im Fall von HRV89 und Cox B3 in Position 10, und im Fall von HRV9 und den 3 Poliostämmen (PV1-3) in Position 6, eine stark positive Ladung anzutreffen ist. Prolin dagegen wird nur bei HRV2 in Position P6, bei HRV89 in Position P9, bei HRV1B in Position P8 und den 3 Poliostämmen in Position P10, angetroffen. Möglicherweise besteht in diesem Bereich zwar ein Bedarf für eine stark positive Ladung zwischen P6 bis P10, nicht aber für Prolin. Der Abstand der positiven Ladung zur Spaltstelle könnte dabei weniger von Bedeutung sein; es wäre z.B. denkbar, daß die Region zwischen Spaltstelle und positiver Ladung sich dem jeweiligen 2A-Enzym durch Bildung einer "loop"-Struktur anpaßt.

Substitutionen in den Positionen P5 (mit Ausnahme von P5D) und P8 zeigen keinen signifikanten Effekt; im Fall der P8-Substitutionen sogar eine Stimulierung (P8F und P8K) bzw. Wildtypeigenschaft bei P8N.

Generell war zu beobachten, daß eine Asp-Substitution immer zu einer Erniedrigung der Spalteffizienz führte, während die Lys-Substitutionen einen eher stimulierenden bzw. einen geringeren negativen Effekt hervorriefen; wie z.B. die (Vmax/Km)rel-Werte von P8D und P8K (0,56 und 1,26), P6D und P6K (0,06 und 0,19), P5D und P5K (< 0,01 und 0,95), P3D und P3K (0,11 und 0,79), P5 D und P5 K (0,39 und 1,05) usw. Dies deutet darauf hin, daß im Substrat eingebrachte negative Ladungen bei der Wechselwirkung mit dem Enzym stören, positive dagegen meistens einen geringen Substitutionseffekt aufweisen, in vielen Fällen sogar zu einer Stimulierung der Spaltbarkeit führen können (z.B. P8K, P4 K und P5 K).

Basierend auf diesen Daten und den Ergebnissen der Deletionsstudien in Peptidsubstraten (Sommergruber, et al., loc. cit.) kann man wie folgt eine potentielle Erkennungssequenz für HRV2 2A in einem Peptidsubstrat beschreiben:

```
P7(Arg)-P6(X)-P5(X)-P4(Ile/Leu)-P3(X)-P2(Thr)-P1(X)  *
P1'(Gly)-P2'(Pro)
```

**Beispiel 8: Ermittlung potentieller Spaltmotive in p220-Molekül**

Das in Beispiel 7 ermittelte Sequenzmotiv wurde dazu verwendet, um nach potentiellen Spaltstellen im p220-Molekül, dem potentiellen zellulären Ziel der Proteinase 2A Aktivität, zu suchen. (Kräusslich et al. (1987) J. Virol. 61, 2711-2718). Da die Aminosäuresequenz des humanen p220 Proteins (eIF-4$_\gamma$) veröffentlicht wurde (Yan et al. 1992, J.Biol. Chem. 267, 23226) war es möglich, nach potentiellen Spaltregionen innerhalb des p220 Moleküls zu suchen.

Als Such-Motiv wurde:

Arg-X-X-Ile/Leu-X-Thr-X-Gly-Pro

vorgegeben und mit Hilfe von Computerprogrammen (MacMolly/U. Schöneberg et al., Softgene GesmbH und DNA Protean/DNA Star Incorp.) mit verschiedenen Stringenzen nach potentiellen Spaltstellen gesucht. Zusätzlich wurden alle bis jetzt bekannten Sequenzen der VP1-2A Spaltstellenregionen von Entero- und Rhinoviren ebenfalls für dieses Suchprofil eingesetzt (Übersicht siehe: Palmenberg, A., 1989, in "Molecular Aspects of Picornavcirus Infection and Detection"; American Society for Microbiology, Washington, D.C., 211-241; eds. Semler und Ehrenfeld). Dabei wurden 5 potentielle Regionen ermittelt, die zumindest teilweise der Anforderung der Substratsspezifität genügen (siehe Abb. 23 Peptidsubstrate p220-1 bis p220-5; SEQ ID NO. 122-126).

Wie oben beschrieben wurden 16-mer Oligopeptide synthetisiert und im Kompetitionsassay eingesetzt. Überraschenderweise zeigte sich, daß die Peptidsubstrate p220-4 und p220-3 von HRV2 2A prozessiert werden können, was bedeutet, daß zumindest potentiell angreifbare Spaltstellen im p220 Molekül existieren und die Möglichkeit einer direkten Proteolyse von p220 während des "host cell shut-off" nicht auszuschließen ist. Die Position der Peptidsequenzen im p220-Molekül ist für p220-1: 457-472, p220-2: 493-508, p220-3: 737-752, p220-4: 476-491 und p220-5: 896-911 (Yan, R. et al. (1992) J. Biol. Chem. 267, 23226-23231).

Die Bestimmung des Km-Wertes für das von p220 abgeleitete Peptidsubstrat p220-4 erfolgte wie oben für die Kompetitionsexperimente beschrieben, nur wurde die Reaktion bei 15°C und mit verschiedenen Peptidsubstratkonzentrationen (0,25 - 4 mM) durchgeführt. Die Zeitpunkte der Probenentnahme im linearen Bereich (Spaltung <10 %) richteten sich bei den einzelnen Ansätzen nach der Konzentration des Peptidsubstrats. Die Ermittlung des Km-Wertes erfolgte graphisch nach Lineweaver-Burk (Lineweaver und Burk, (1934) J. Am. Chem. Soc. 56, 658) wobei jeweils von 3 unabhängigen Experimenten eine Regressionsgerade errechnet wurde. Der Km-Wert für p220-4 liegt mit $1,71 \pm 0,3 \times 10^{-3}$ Mol/l innerhalb der zu erwartenden Größenordnung (im Vergleich mit dem Km-Wert für das Wildtyppeptidsubstrat P8-P8':$5,4\pm0,02 \times 10^{-4}$ Mol/l).

Es kann damit gezeigt werden, daß im p220 Molekül durchaus potentielle Spaltstellen für einen direkten Angriff von HRV2 2A zur Verfügung stehen.

Sowohl p220-3 als auch p220-4 wirken als kompetitive Inhibitoren und sind in der Lage, den "host cell shut-off" zu inhibieren.

**LEGENDEN ZU DEN FIGUREN**

**Figur 1** zeigt den Herstellungsweg des Plasmids pUC18-2C*3A.

**Figur 2** zeigt die für die Einführung eines Stoppcodons hinter dem 2C Gen verwendeten Oligonukleotide. Gezeigt sind auch die Sequenzen von diesem Bereich vor und nach der Mutagenese; das geänderte Codon ist durch Fettdruck hervorgehoben.

**Figur 3** zeigt den Herstellungsweg der Plasmide pSVL-2BC und pSVL-2C.

**Figur 4** zeigt die für die Herstellung von pSVL-2BC und pSVL-2C verwendeten Oligonukleotide; die AUGs sind durch Fettdruck hervorgehoben.

**Figur 5** zeigt die Plasmide pLink-2BC und pLink-2C. Der schwarze Pfeil markiert jeweils das synthetische AUG, das vor den beiden Genen eingeführt wurde. Die offenen Pfeile markieren AUGs bei denen vermutlich eine interne Initiation stattfindet. S zeigt den Promoter der T7 RNA Polymerase. Der geschlossene Kreis zeigt die Position des durch gezielte Mutagenese eingeführten Stopcodons. Abkürzung: Ba, BamHI.

**Figur 6** zeigt die Konstruktionen pHRV2/5'UTR-2C, p$\beta$-2C, pLink-2C und pLink-2BC. Der offene Rhombus stellt die 5'UTR des Kaninchen $\beta$-Globingens dar. Die übrigen Symbole sind mit denen in Figur 5 identisch.

**Figur 7** zeigt die Oligonukleotide die für die Herstellung der Konstruktionen von pHRV2/5'UTR-2C und p$\beta$-2C verwendet wurden; das AUG ist durch Fettdruck hervorgehoben.

**Figur 8A** zeigt das Fluorogramm eines 12,5%-igen Proteingeles mit Translationsprodukten von ungecappten RNAs von den vier Plasmiden pHRV2/5'UTR-2C (Spur 1), p$\beta$-2C (Spur 2), pLink-2C (Spur 3) und pLink-2BC (Spur 4). In Spur 5 ist das Ergebnis einer Translation ohne Zugabe von RNA dargestellt. Die Zahlen links (Einheit: Kilodalton) weisen auf die Translationsprodukte von den RNAs von den Plasmiden pHRV2/5'UTR-2C, pβ-2C, and pLink-2C. Die Zahlen rechts (Einheit: Kilodalton) weisen auf die zusätzlichen Translationsprodukte von der RNA des Plasmids pLink-2BC.

**Figur 8B** zeigt das Fluorogramm eines 12,5%-igen Proteingeles mit Translationsprodukten von RNAs von den folgenden Plasmiden:

Spur 1: RNA von Plasmid pHRV2/5'UTR-2C, ohne Cap-Struktur.

Spur 2: RNA vom Plasmid pHRV2/5'UTR-2C, mit Cap-Struktur.

Spur 3: RNA vom Plasmid pLink-2BC, ohne Cap-Struktur.

Spur 4: RNA von Plasmid pLink-2BC, mit Cap-Struktur.

Spur 5: RNA von Plasmid pLink-2C, mit Cap-Struktur.

Spur 6: RNA von Plasmid pLink-2C, ohne Cap-Struktur.

Spur 7: RNA von Plasmid pLink-2C, mit Cap-Struktur.

Spur 8: RNA vom Plasmid pLink-2C, mit Cap-Struktur.

In Spur 7 wurde der Kaninchenreticulocytenextrakt mit einer 2A Proteinase Präparation vorbehandelt, wie im Beispiel 3 beschrieben wird. In Spur 8 wurde der Kaninchenreticulocytenextrakt wie in Spur 7 vorbehandelt; in diesem Fall wurde eine 2A Präparation verwendet, die mit 0.5 mM Elastatinal versetzt worden war.

Die Zahlen (Einheit: Kilodalton) weisen auf die jeweiligen Translationsprodukte der verschiedenen RNAs.

**Figur 9** zeigt die Konstruktion pET/2A. Der mit waagrechten Strichen gefüllte Block stellt die 14 Aminosäuren des T7 Gen 10 Proteins dar, der offene Block die letzten 7 Aminosäuren des HRV2 VP1 Gens und der gerade, schwarze Block die 142 Aminosäuren des 2A Gens. Die Bindungsstelle für das PC20 Antiserum ist durch das mit einem Kreuz gefüllte Viereck dargestellt. S zeigt den Promotor der T7 RNA Polymerase. Die folgenden Restriktionsschnittstellen sind angegeben: A, Apal; B, BamHI; H, HindIII; M, MluI; P, PstI. Ein Kreis bei einer Restriktionsschnittstelle zeigt an, daß diese Spaltstelle während der Herstellung verlorengegangen ist.

**Figur 10** zeigt die Konstruktion pET8c/2AΔ3Gly. Der mit waagrechten Strichen gefüllte Block stellt die 13 Aminosäuren des T7 Gen 10 Proteins dar, der sich anschließende Streifen die 3 Aminosäuren des α-Fragmentes, der offene Block die letzten 28 Aminosäuren des HRV2 VP1 Gens und der gerade, schwarze Block die 139 der 142 Aminosäuren des 2A Gens. Die Position der Deletion der drei Glycinreste ist durch ein Delta gezeichnet. Die Bindungsstelle für das PC20 Antiserum ist durch das mit einem Kreuz gefüllte Viereck dargestellt. S zeigt den Promotor der T7 RNA Polymerase. Die folgenden Restriktionsschnittstellen sind angegeben: A, Apal; B, BamHI; H, HindIII; P, PstI; S, SalI. Ein Kreis bei einer Restriktionsstelle zeigt an, daß diese während der Herstellung verlorengegangen ist.

**Figur 11** zeigt die Konstruktion pET8c/2A. Der mit waagrechten Strichen gefüllte Block stellt die 13 Aminosäuren des T7 Gen 10 Proteins dar, der sich anschließende Streifen die 3 Aminosäuren des α-Fragmentes, der offene Block die C-terminalen 28 Aminosäuren des HRV2 VP1 Gens und der gerade, schwarze Block die 142 Aminosäuren des 2A Gens. Die Bindungsstelle für das PC20 Antiserum ist durch das mit einem Kreuz gefüllte Viereck dargestellt. S zeigt den Promotor der T7 RNA Polymerase. Die folgenden Restriktionsschnittstellen sind angegeben: A, Apal; P, PstI.

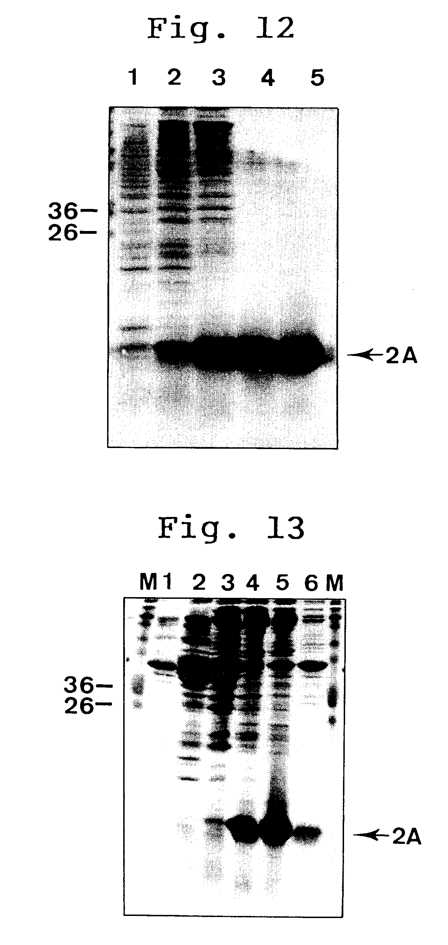

**Figur 12** zeigt ein mit Coomassie-Blue gefärbtes 15 % Proteingel mit den einzelnen Reinigungsstufen der 2A-Reinigung. In jeder Spur wurden 0,13 OD$_{280}$ Einheiten aufgetragen.

Spur 1: Proteingehalt des löslichen E. coli Rohextraktes.

Spur 2: Proteingehalt des in Puffer A aufgenommen 40 % Ammoniumsulfatniederschlages.

Spur 3: Proteingehalt der vereinigten Mono-Q Fraktionen.

Spur 4: Proteingehalt der vereinigten und konzentrierten Superdex Fraktionen (pool A).

Spur 5: Proteingehalt der vereinigten und konzentrierten Superdex Fraktionen (Pool A).

In Spur 5 wurde die Probe mit einem Laemmli-Probenpuffer ohne β-Mercaptoethanol und ohne vorheriges Erhitzen aufgetragen.

Der Pfeil zeigt das 2A Protein. Die Zahlen links (Einheit: Kilodalton) weisen auf die Position ausgewählter Markerproteine.

**Figur 13** zeigt ein mit Coomassie-Blue gefärbtes 15 % Proteingel von Fraktionen der Mono-Q Säule. Es wurden die Fraktionen aufgetragen, die zwischen 350 mM und 500 mM NaCl eluierten.

Der Pfeil zeigt das 2A Protein. Die Zahlen links (Einheit: Kilodalton) weisen auf die Position ausgewählter Markerproteine.

Figur 14 zeigt ein mit Coomassie-Blue gefärbtes 15 % Proteingel von Fraktionen der Superdex Säule. Es wurden die Fraktionen 33 bis 44 aufgetragen. Fraktionen 36 bis 43 wurden vereinigt und als Pool A bezeichnet; Fraktionen 33 bis 35 wurden ebenfalls vereinigt und als Pool B bezeichnet.

Der Pfeil zeigt das 2A Protein. Die Zahlen rechts (Einheit: Kilodalton) weisen auf die Position ausgewählter Markerproteine.

**Figur 15** zeigt ein mit Coomassie-Blue gefärbtes 15 % Proteingel von vier verschiedenen 2A Präparationen (Pool A Konzentrate), die in Lagerpuffer aufbewahrt wurden.

Es wurden folgende Mengen aufgetragen:

Spur 1: 6,3 µg (Gesamtmenge 1,04 mg, Konzentration 2,1 mg/ml).

Spur 2: 2,74 µg (Gesamtmenge 0,44 mg, Konzentration 0,88 mg/ml).

Spur 3: 9,84 µg (Gesamtmenge 1,64 mg, Konzentration 3,28 mg/ml).

Spur 4: 5,52 µg (Gesamtmenge 0,92 mg, Konzentration 1,84 mg/ml).

Der Pfeil zeigt das 2A Protein. Die Zahlen links (Einheit: Kilodalton) weisen auf die Position ausgewählter Markerproteine.

**Figur 16A** zeigt ein mit Coomassie-Blue gefärbtes 15 % Proteingel.

Spur M: Markerproteine

Spur 1: 5 µg von 2A Pool B (Figur 21), zu dem 1.25 µg Kreatinkinase als Markerprotein versetzt worden waren.

Spur 2: 5 µg von 2A Pool B.

**Figur 16B** zeigt das Fluorogramm eines 12,5 % Proteingeles mit dem Translationsprodukt des ApaI geschnittenen Plasmids pβHRV2/sub nach Inkubation mit 2A Proteinase.

Spur 1: Inkubation (30 min, 30 °C) mit 10 µg 2A (Pool B).

Spur 2: Inkubation (30 min, 30 °C) mit 1 µg 2A (Pool B).

Spur 3: Inkubation ohne 2A Proteinase.

Die markierte 40 kD Bande ist das Translationsprodukt von pβHRV2/sub; die mit 32 kD markierte Bande entsteht durch die proteolytische Aktivität der 2A Proteinase.

**Figur 16C** zeigt das Fluorogramm eines 12,5 % Polyacrylamidgeles mit Translationsprodukten folgender RNAs:

Spuren 1 - 4: 2 µg RNA aus Kaninchenretikulocyten.

Spuren 5 - 8: ca 1 µg einer in vitro transkribierten RNA (ohne Cap-Struktur) vom Plasmid pHRV2/5'UTR-2C.

Spur 9: Keine Zugabe von RNA.

In den Spuren 1 und 5 wurde der HeLa Translationsextrakt mit 2 µg aus 2A Pool B wie im Beispiel 5 beschrieben vorbehandelt. In den Spuren den 2 und 6 wurde der HeLa Translationsextrakt nicht vorbehandelt. In den Spuren 3 und 7 wurde der HeLa Translationsextrakt mit 1 µl Puffer C behandelt. In den Spuren 4 und 8 wurde der HeLa Translationsextrakt wie in den Spuren 1 und 5 behandelt; in diesem Fall wurde die 2A Proteinase jedoch zuvor mit 0.5 mM Elastatinal inhibiert.

Der linke Pfeil weist auf das 16 kD Translationsprodukt der Kaninchenretikulocyten; der rechte Pfeil weist auf das 37 kD Translationsprodukt der RNA vom Plasmid pHRV21/5'UTR-2C.

**Figur 17** zeigt die Sequenz des Oligonukleotides 1868; sie entspricht den Nukleotiden 1694 bis 1717 von SV40.

**Figur 18** zeigt das Fluorogramm eines 12,5 % Proteingeles mit Translationsprodukten von pLink-2C RNA (circa 2 pmol) ohne Cap-Struktur in Anwesenheit folgender Oligonukleotide:

Spur 1: ohne Oligonukleotid.

Spur 2: 0,2 pmol 1092

Spur 3: 2 pmol 1092

Spur 4: 20 pmol 1092

Spur 5: 200 pmol 1092

Spur 6: 0,2 pmol 1868

Spur 7: 2 pmol 1868

Spur 8: 20 pmol 1868

In der Spur M sind $C^{14}$-markierte Proteingrößenmarker (Firma Amersham, Produkt Nr. CFA 626) aufgetragen; die Zahlen links weisen auf ihre Molekulargewichte. Die Zahlen rechts (Einheit: Kilodalton) weisen auf die jeweiligen Translationsprodukte der verschiedenen RNAs.

**Figur 19** zeigt das Fluorogramm eines 12,5 % Proteingeles mit Translationsprodukten von pLink-2C RNA (circa 2 pmol) mit Cap-Struktur in Anwesenheit folgender Oligonukleotide:

Spur 1: 0,2 pmol 1092

Spur 2: 2 pmol 1092

Spur 3: 20 pmol 1092

Spur 4: 200 pmol 1092

Spur 5: 0,2 pmol 1868

Spur 6: 2 pmol 1868

Spur 7: 20 pmol 1868

Die Zahlen rechts (Einheit: Kilodalton) weisen auf die 37 kD und 33 kD Translationsprodukte.

**Figur 20** zeigt die tabellarische Auflistung der Nukleotidsequenzen in der Umgebung der AUGs der 2C und 2BC-Konstruktionen (A). Das Oligonukleotid, daß für die Konstruktion von pLink-2C benutzt wurde, ist unterstrichen. Für alle internen Translationsprodukte sind zwei AUG Startcodons möglich - außer für das 20 kD Produkt. Teil (B) zeigt die AUGs innerhalb der HRV2 2C und 2B Sequenz, die als Translationsstart-Codons dienen könnten.

**Figur 21** zeigt eine schematische Darstellung des HRV2 Genomes und die Konstruktion GR6. Die geschlossenen Pfeile zeigen die ATG Kodons bei 449 und 611. Der schwarze Block stellt die kodierende Region von HRV2 dar. S zeigt den Promoter der T7 RNA Polymerase. Die folgenden Restriktionstellen sind angegeben: A, AccI; Av, AvaII; B, BamHI; Bn, BanII; H, HincII; H3, HindIII; N, NcoI; P, PstI; RI, Eco RI; RV, EcoRV; Sa, SalI; Sm, SmaI.

**Figur 22** zeigt die Aminosäuresequenz der VP1/P2A-Proteine nach Skern et al. (1985) Nucl. Acids Res. 13, 2111-2126 und Sommergruber et al. (1989) Virology 169, 68-77.

**Figur 23** zeigt die relative Spaltbarkeit ausgedrückt in (Vmax/Km)rel-Werten von synthetischen Peptidsubstraten:

a) mit einzelnen Aminosäurensubstitutionen in den Positionen P8 bis P6' in einem 15-mer Wildtypsubstrat,

b) welche von der p220 Sequenz als potentielle Spaltregion abgeleitet wurden,

c) mit einer artifiziellen Aminosäurensequenz, die das potentielle Erkennungsmotiv für HRV2 2A enthalten,

d) die von der Coxsackievirus B4-Spaltregion für 2A abgeleitet wurden.

\* gibt die Spaltstelle innerhalb der Peptidsubstrate an

\# weist auf Schwankungen in der Reproduzierbarkeit von mehr als ± 20 % hin (siehe Text).

**EP 0 564 801 A1**

SEQUENZPROTOKOLL

(1) ALGEMEINE INFORMATION:

    (i) ANMELDER:
      (A) NAME: Boehringer Ingelheim International GmbH
      (B) STRASSE: Binger Str.
      (C) ORT: Ingelheim
      (D) BUNDESLAND: Rheinland-Pfalz
      (E) LAND: Deutschland
      (F) POSTLEITZAHL: W-6507
      (G) TELEPHON: 06132-772003
      (H) TELEFAX: 01632-774377
      (I) TELEX: 418791-0 bid

    (ii) ANMELDETITEL: VERFAHREN ZUR ANALYSE DES "HOST CELL SHUT-OFFS"

   (iii) ANZAHL DER SEQUENZEN: 22

    (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)

    (v) GEGENWÄRTIGE ANMELDEDATEN:
      ANMELDENUMMER: ...........

(2) INFORMATION ZU SEQ ID NO: 1:

    (i) SEQUENZ CHARAKTERISTIKA:
      (A) LÄNGE: 425 Aminosäuren
      (B) ART: Aminosäure
      (C) STRANGFORM: Einzel
      (D) TOPOLOGIE: linear

    (ii) ART DES MOLEKÜLS: Protein

    (vi) URSPRÜNLICHE HERKUNFT:
      (B) STAMM: HUMAN RHINOVIRUS TYPE 2

    (xi) SEQUENZBESCHREIBUNG: SEQ ID NO: 1:

```
Asn Pro Val Glu Asn Tyr Ile Asp Glu Val Leu Asn Glu Val Leu Val
1               5                   10                  15

Val Pro Asn Ile Asn Ser Ser Asn Pro Thr Thr Ser Asn Ser Ala Pro
            20                  25                  30

Ala Leu Asp Ala Ala Glu Thr Gly His Thr Ser Ser Val Gln Pro Glu
        35                  40                  45

Asp Val Ile Glu Thr Arg Tyr Val Gln Thr Ser Gln Thr Arg Asp Glu
        50                  55                  60

Met Ser Leu Glu Ser Phe Leu Gly Arg Ser Gly Cys Ile His Glu Ser
65                  70                  75                  80

Lys Leu Glu Val Thr Leu Ala Asn Tyr Asn Lys Glu Asn Phe Thr Val
                85                  90                  95
```

```
Trp Ala Ile Asn Leu Gln Glu Met Ala Gln Ile Arg Arg Lys Phe Glu
            100             105             110

Leu Phe Thr Tyr Thr Arg Phe Asp Ser Glu Ile Thr Leu Val Pro Cys
        115             120             125

Ile Ser Ala Leu Ser Gln Asp Ile Gly His Ile Thr Met Gln Tyr Met
    130             135             140

Tyr Val Pro Pro Gly Ala Pro Val Pro Asn Ser Arg Asp Asp Tyr Ala
145             150             155             160

Trp Gln Ser Gly Thr Asn Ala Ser Val Phe Trp Gln His Gly Gln Ala
            165             170             175

Tyr Pro Arg Phe Ser Leu Pro Phe Leu Ser Val Ala Ser Ala Tyr Tyr
        180             185             190

Met Phe Tyr Asp Gly Tyr Asp Glu Gln Asp Gln Asn Tyr Gly Thr Ala
        195             200             205

Asn Thr Asn Asn Met Gly Ser Leu Cys Ser Arg Ile Val Thr Glu Lys
    210             215             220

His Ile His Lys Val His Ile Met Thr Arg Ile Tyr His Lys Ala Lys
225             230             235             240

His Val Lys Ala Trp Cys Pro Arg Pro Pro Arg Ala Leu Glu Tyr Thr
            245             250             255

Arg Ala His Arg Thr Asn Phe Lys Ile Glu Asp Arg Ser Ile Gln Thr
        260             265             270

Ala Ile Val Thr Arg Pro Ile Ile Thr Thr Ala Gly Pro Ser Asp Met
        275             280             285

Tyr Val His Val Gly Asn Leu Ile Tyr Arg Asn Leu His Leu Phe Asn
    290             295             300

Ser Glu Met His Glu Ser Ile Leu Val Ser Tyr Ser Ser Asp Leu Ile
305             310             315             320

Ile Tyr Arg Thr Asn Thr Val Gly Asp Asp Tyr Ile Pro Ser Cys Asp
            325             330             335

Cys Thr Gln Ala Thr Tyr Tyr Cys Lys His Lys Asn Arg Tyr Phe Pro
            340             345             350

Ile Thr Val Thr Ser His Asp Trp Tyr Glu Ile Gln Glu Ser Glu Tyr
        355             360             365

Tyr Pro Lys His Ile Gln Tyr Asn Leu Leu Ile Gly Glu Gly Pro Cys
    370             375             380

Glu Pro Gly Asp Cys Gly Gly Lys Leu Leu Cys Lys His Gly Val Ile
385             390             395             400

Gly Ile Val Thr Ala Gly Gly Asp Asn His Val Ala Phe Ile Asp Leu
            405             410             415

Arg His Phe His Cys Ala Glu Glu Gln
        420             425
```

(2) INFORMATION ZU SEQ ID NO: 2:

26

     (i) SEQUENZ CHARAKTERISTIKA:
        (A) LÄNGE: 22 Basenpaare
        (B) ART: Nukleinsäure
        (C) STRANGFORM: Einzel
        (D) TOPOLOGIE: linear

    (ii) ART DES MOLEKÜLS: cDNS

    (xi) SEQUENZBESCHREIBUNG: SEQ ID NO: 2:

ATCAATTGGC TATTGGAATA TA            22

(2) INFORMATION ZU SEQ ID NO: 3:

     (i) SEQUENZ CHARAKTERISTIKA:
        (A) LÄNGE: 51 Basenpaare
        (B) ART: Nukleinsäure
        (C) STRANGFORM: Doppel
        (D) TOPOLOGIE: linear

    (ii) ART DES MOLEKÜLS: cDNS

    (xi) SEQUENZBESCHREIBUNG: SEQ ID NO: 3:

GTTGATGTCA TGACAGCTAT ATTCCAAGGG CCAATTGATA TGAAAAACCC A    51

(2) INFORMATION ZU SEQ ID NO: 4:

     (i) SEQUENZ CHARAKTERISTIKA:
        (A) LÄNGE: 51 Basenpaare
        (B) ART: Nukleinsäure
        (C) STRANGFORM: Doppel
        (D) TOPOLOGIE: linear

    (ii) ART DES MOLEKÜLS: cDNS

    (xi) SEQUENZBESCHREIBUNG: SEQ ID NO: 4:

GTTGATGTCA TGACAGCTAT ATTCCAATAG CCAATTGATA TGAAAAACCC A    51

(2) INFORMATION ZU SEQ ID NO: 5:

     (i) SEQUENZ CHARAKTERISTIKA:
        (A) LÄNGE: 33 Basenpaare
        (B) ART: Nukleinsäure
        (C) STRANGFORM: Doppel
        (D) TOPOLOGIE: linear

    (ii) ART DES MOLEKÜLS: cDNS

    (xi) SEQUENZBESCHREIBUNG: SEQ ID NO: 5:

CTAGACCACC ATGGGGGTTA CAGATTATAT ACA    33

(2) INFORMATION ZU SEQ ID NO: 6:

```
    (i) SEQUENZ CHARAKTERISTIKA:
        (A) LÄNGE: 48 Basenpaare
        (B) ART: Nukleinsäure
        (C) STRANGFORM: Doppel
        (D) TOPOLOGIE: linear

   (ii) ART DES MOLEKÜLS: cDNS
```

```
   (xi) SEQUENZBESCHREIBUNG: SEQ ID NO: 6:

CTAGACCACC ATGTCAGATT CATGGTTAAA GAAATTTACT GAAGCATG                    48
```

(2) INFORMATION ZU SEQ ID NO: 7:

```
    (i) SEQUENZ CHARAKTERISTIKA:
        (A) LÄNGE: 52 Basenpaare
        (B) ART: Nukleinsäure
        (C) STRANGFORM: Doppel
        (D) TOPOLOGIE: linear

   (ii) ART DES MOLEKÜLS: cDNS
```

```
   (xi) SEQUENZBESCHREIBUNG: SEQ ID NO: 7:

ACACTTGCTT TTGACACAAC TGTGTTTACT TGCAATCCCC CAAAACAGAC AG              52
```

(2) INFORMATION ZU SEQ ID NO: 8:

```
    (i) SEQUENZ CHARAKTERISTIKA:
        (A) LÄNGE: 39 Basenpaare
        (B) ART: Nukleinsäure
        (C) STRANGFORM: Doppel
        (D) TOPOLOGIE: linear

   (ii) ART DES MOLEKÜLS: cDNS
```

```
   (xi) SEQUENZBESCHREIBUNG: SEQ ID NO: 8:

CATGTCAGAT TCATGGTTAA AGAAATTTAC TGAAGCATG                             39
```

(2) INFORMATION ZU SEQ ID NO: 9:

```
    (i) SEQUENZ CHARAKTERISTIKA:
        (A) LÄNGE: 16 Basenpaare
        (B) ART: Nukleinsäure
        (C) STRANGFORM: Einzel
        (D) TOPOLOGIE: linear

   (ii) ART DES MOLEKÜLS: cDNS
```

```
   (xi) SEQUENZBESCHREIBUNG: SEQ ID NO: 9:

GGGGTCAAGA TACTGA                                                      16
```

(2) INFORMATION ZU SEQ ID NO: 10:

       (i) SEQUENZ CHARAKTERISTIKA:
            (A) LÄNGE: 12 Basenpaare
            (B) ART: Nukleinsäure
            (C) STRANGFORM: Einzel
            (D) TOPOLOGIE: linear

       (ii) ART DES MOLEKÜLS: mRNS


       (xi) SEQUENZBESCHREIBUNG: SEQ ID NO: 10:

ACCACCAUGU CA                                                    12

(2) INFORMATION ZU SEQ ID NO: 11:

       (i) SEQUENZ CHARAKTERISTIKA:
            (A) LÄNGE: 12 Basenpaare
            (B) ART: Nukleinsäure
            (C) STRANGFORM: Einzel
            (D) TOPOLOGIE: linear

       (ii) ART DES MOLEKÜLS: mRNS


       (xi) SEQUENZBESCHREIBUNG: SEQ ID NO: 11:

GACAGCAUGU CA                                                    12

(2) INFORMATION ZU SEQ ID NO: 12:

       (i) SEQUENZ CHARAKTERISTIKA:
            (A) LÄNGE: 12 Basenpaare
            (B) ART: Nukleinsäure
            (C) STRANGFORM: Einzel
            (D) TOPOLOGIE: linear

       (ii) ART DES MOLEKÜLS: mRNS


       (xi) SEQUENZBESCHREIBUNG: SEQ ID NO: 12:

GGCACCAUGU CA                                                    12

(2) INFORMATION ZU SEQ ID NO: 13:

       (i) SEQUENZ CHARAKTERISTIKA:
            (A) LÄNGE: 12 Basenpaare
            (B) ART: Nukleinsäure
            (C) STRANGFORM: Einzel
            (D) TOPOLOGIE: linear

       (ii) ART DES MOLEKÜLS: mRNS


       (xi) SEQUENZBESCHREIBUNG: SEQ ID NO: 13:

CCCUGAAUGU GG                                                    12

(2) INFORMATION ZU SEQ ID NO: 14:

        (i) SEQUENZ CHARAKTERISTIKA:
            (A) LÄNGE: 12 Basenpaare
            (B) ART: Nukleinsäure
            (C) STRANGFORM: Einzel
            (D) TOPOLOGIE: linear

        (ii) ART DES MOLEKÜLS: mRNS


        (xi) SEQUENZBESCHREIBUNG: SEQ ID NO: 14:

ACCACCAUGG GG                                                    12

(2) INFORMATION ZU SEQ ID NO: 15:

        (i) SEQUENZ CHARAKTERISTIKA:
            (A) LÄNGE: 9 Basenpaare
            (B) ART: Nukleinsäure
            (C) STRANGFORM: Einzel
            (D) TOPOLOGIE: linear

        (ii) ART DES MOLEKÜLS: mRNS


        (xi) SEQUENZBESCHREIBUNG: SEQ ID NO: 15:    ´

CCRCCAUGG                                                        9

(2) INFORMATION ZU SEQ ID NO: 16:

        (i) SEQUENZ CHARAKTERISTIKA:
            (A) LÄNGE: 12 Basenpaare
            (B) ART: Nukleinsäure
            (C) STRANGFORM: Einzel
            (D) TOPOLOGIE: linear

        (ii) ART DES MOLEKÜLS: mRNS


        (xi) SEQUENZBESCHREIBUNG: SEQ ID NO: 16:

GAAUGGAUGA AG                                                    12

(2) INFORMATION ZU SEQ ID NO: 17:

        (i) SEQUENZ CHARAKTERISTIKA:
            (A) LÄNGE: 12 Basenpaare
            (B) ART: Nukleinsäure
            (C) STRANGFORM: Einzel
            (D) TOPOLOGIE: linear

        (ii) ART DES MOLEKÜLS: mRNS


        (xi) SEQUENZBESCHREIBUNG: SEQ ID NO: 17:

AAGUCGAUGC UC                                                    12

(2) INFORMATION ZU SEQ ID NO: 18:

(i) SEQUENZ CHARAKTERISTIKA:
    (A) LÄNGE: 12 Basenpaare
    (B) ART: Nukleinsäure
    (C) STRANGFORM: Einzel
    (D) TOPOLOGIE: linear

(ii) ART DES MOLEKÜLS: mRNS


(xi) SEQUENZBESCHREIBUNG: SEQ ID NO: 18:

GCUGACAUGA AA                                              12

(2) INFORMATION ZU SEQ ID NO: 19:

    (i) SEQUENZ CHARAKTERISTIKA:
        (A) LÄNGE: 12 Basenpaare
        (B) ART: Nukleinsäure
        (C) STRANGFORM: Einzel
        (D) TOPOLOGIE: linear

    (ii) ART DES MOLEKÜLS: mRNS


(xi) SEQUENZBESCHREIBUNG: SEQ ID NO: 19:

AUUAAAAUGG AA                                              12

(2) INFORMATION ZU SEQ ID NO: 20:

    (i) SEQUENZ CHARAKTERISTIKA:
        (A) LÄNGE: 12 Basenpaare
        (B) ART: Nukleinsäure
        (C) STRANGFORM: Einzel
        (D) TOPOLOGIE: linear

    (ii) ART DES MOLEKÜLS: mRNS


(xi) SEQUENZBESCHREIBUNG: SEQ ID NO: 20:

GCCAAAAUGA UA                                              12

(2) INFORMATION ZU SEQ ID NO: 21:

    (i) SEQUENZ CHARAKTERISTIKA:
        (A) LÄNGE: 12 Basenpaare
        (B) ART: Nukleinsäure
        (C) STRANGFORM: Einzel
        (D) TOPOLOGIE: linear

    (ii) ART DES MOLEKÜLS: mRNS


(xi) SEQUENZBESCHREIBUNG: SEQ ID NO: 21:

AAAUGGAUGU UG                                              12

(2) INFORMATION ZU SEQ ID NO: 22:

```
(i) SEQUENZ CHARAKTERISTIKA:
    (A) LÄNGE: 12 Basenpaare
    (B) ART: Nukleinsäure
    (C) STRANGFORM: Einzel
    (D) TOPOLOGIE: linear

(ii) ART DES MOLEKÜLS: mRNS


(xi) SEQUENZBESCHREIBUNG: SEQ ID NO: 22:
```

UCAGCAAUGG UC                                    12

```
(2) INFORMATION ZU SEQ ID NO: 23:

    (i) SEQUENZ CHARAKTERISTIKA:
        (A) LÄNGE: 16 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 23

    Thr Arg Pro Ile Ile Thr Thr Ala Gly Pro Ser Asp Met Tyr Val His
    1                   5                   10                  15


(2) INFORMATION ZU SEQ ID NO: 24:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 24:

    Thr Arg Pro Ile Ile Thr Thr Ala Gly Pro Ser Asp Met Tyr Val
    1                   5                   10                  15

(2) INFORMATION ZU SEQ ID NO: 25:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 25:
```

```
Asp Arg Pro Ile Ile Thr Thr Ala Gly Pro Ser Asp Met Tyr Val
1                   5                   10                  15
```

(2) INFORMATION ZU SEQ ID NO: 26:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 26:

```
Phe Arg Pro Ile Ile Thr Thr Ala Gly Pro Ser Asp Met Tyr Val
1                   5                   10                  15
```

(2) INFORMATION ZU SEQ ID NO: 27:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 27:

```
Asn Arg Pro Ile Ile Thr Thr Ala Gly Pro Ser Asp Met Tyr Val
1                   5                   10                  15
```

(2) INFORMATION ZU SEQ ID NO: 28:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 28:

```
Lys Arg Pro Ile Ile Thr Thr Ala Gly Pro Ser Asp Met Tyr Val
1                   5                   10                  15
```

(2) INFORMATION ZU SEQ ID NO: 29:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 29:

Thr Gln Pro Ile Ile Thr Thr Ala Gly Pro Ser Asp Met Tyr Val
1               5               10              15

(2) INFORMATION ZU SEQ ID NO: 30:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 30:

Thr Phe Pro Ile Ile Thr Thr Ala Gly Pro Ser Asp Met Tyr Val
1               5               10              15

(2) INFORMATION ZU SEQ ID NO: 31:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 31:

Thr Asp Pro Ile Ile Thr Thr Ala Gly Pro Ser Asp Met Tyr Val
1               5               10              15

(2) INFORMATION ZU SEQ ID NO: 32:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 32:

Thr Thr Pro Ile Ile Thr Thr Ala Gly Pro Ser Asp Met Tyr Val
1               5               10              15

(2) INFORMATION ZU SEQ ID NO: 33:

    (i) SEQUENZ CHARAKTERISTIKA:
        (A) LÄNGE: 15 Aminosäuren
        (B) ART: Aminosäure
        (C) STRANGFORM: Einzel

(D) TOPOLOGIE: linear

(ii) ART DES MOLEKÜLS: Peptid

(xi) SEQUENZBESCHREIBUNG: SEQ ID NO: 33:

Thr Arg Thr Ile Ile Thr Thr Ala Gly Pro Ser Asp Met Tyr Val
1               5                   10                  15

(2) INFORMATION ZU SEQ ID NO: 34:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 34:

Thr Arg Lys Ile Ile Thr Thr Ala Gly Pro Ser Asp Met Tyr Val
1               5                   10                  15

(2) INFORMATION ZU SEQ ID NO: 35:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 35:

Thr Arg Phe Ile Ile Thr Thr Ala Gly Pro Ser Asp Met Tyr Val
1               5                   10                  15

(2) INFORMATION ZU SEQ ID NO: 36:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 36:

Thr Arg Asp Ile Ile Thr Thr Ala Gly Pro Ser Asp Met Tyr Val
1               5                   10                  15

(2) INFORMATION ZU SEQ ID NO: 37:

```
(i)  SEQUENZ CHARAKTERISTIKA:
     (A)  LÄNGE: 16 Aminosäuren
     (B)  ART: Aminosäure
     (C)  STRANGFORM: Einzel
     (D)  TOPOLOGIE: linear

(ii) ART DES MOLEKÜLS: Peptid


(xi) SEQUENZBESCHREIBUNG: SEQ ID NO: 37:

Thr Arg Pro Thr Ile Thr Thr Ala Gly Pro Ser Asp Met Tyr Val
1               5               10                  15
```

(2) INFORMATION ZU SEQ ID NO: 38:

```
(i)  SEQUENZ CHARAKTERISTIKA:
     (A)  LÄNGE: 15 Aminosäuren
     (B)  ART: Aminosäure
     (C)  STRANGFORM: Einzel
     (D)  TOPOLOGIE: linear

(ii) ART DES MOLEKÜLS: Peptid


(xi) SEQUENZBESCHREIBUNG: SEQ ID NO: 38:

Thr Arg Pro Lys Ile Thr Thr Ala Gly Pro Ser Asp Met Tyr Val
1               5               10                  15
```

(2) INFORMATION ZU SEQ ID NO: 39:

```
(i)  SEQUENZ CHARAKTERISTIKA:
     (A)  LÄNGE: 15 Aminosäuren
     (B)  ART: Aminosäure
     (C)  STRANGFORM: Einzel
     (D)  TOPOLOGIE: linear

(ii) ART DES MOLEKÜLS: Peptid


(xi) SEQUENZBESCHREIBUNG: SEQ ID NO: 39:

Thr Arg Pro Asp Ile Thr Thr Ala Gly Pro Ser Asp Met Tyr Val
1               5               10                  15
```

(2) INFORMATION ZU SEQ ID NO: 40:

```
(i)  SEQUENZ CHARAKTERISTIKA:
     (A)  LÄNGE: 15 Aminosäuren
     (B)  ART: Aminosäure
     (C)  STRANGFORM: Einzel
     (D)  TOPOLOGIE: linear

(ii) ART DES MOLEKÜLS: Peptid


(xi) SEQUENZBESCHREIBUNG: SEQ ID NO: 40:
```

```
Thr Arg Pro Phe Ile Thr Thr Ala Gly Pro Ser Asp Met Tyr Val
1               5                   10                  15
```

(2)  INFORMATION ZU SEQ ID NO: 41:

     (i)  SEQUENZ CHARAKTERISTIKA:
          (A) LÄNGE: 15 Aminosäuren
          (B) ART: Aminosäure
          (C) STRANGFORM: Einzel
          (D) TOPOLOGIE: linear

     (ii) ART DES MOLEKÜLS: Peptid


     (xi) SEQUENZBESCHREIBUNG: SEQ ID NO: 41:

```
Thr Arg Pro Ile Lys Thr Thr Ala Gly Pro Ser Asp Met Tyr Val
1               5                   10                  15
```

(2)  INFORMATION ZU SEQ ID NO: 42:

     (i)  SEQUENZ CHARAKTERISTIKA:
          (A) LÄNGE: 15 Aminosäuren
          (B) ART: Aminosäure
          (C) STRANGFORM: Einzel
          (D) TOPOLOGIE: linear

     (ii) ART DES MOLEKÜLS: Peptid


     (xi) SEQUENZBESCHREIBUNG: SEQ ID NO: 42:

```
Thr Arg Pro Ile Ser Thr Thr Ala Gly Pro Ser Asp Met Tyr Val
1               5                   10                  15
```

(2)  INFORMATION ZU SEQ ID NO: 43:

     (i)  SEQUENZ CHARAKTERISTIKA:
          (A) LÄNGE: 15 Aminosäuren
          (B) ART: Aminosäure
          (C) STRANGFORM: Einzel
          (D) TOPOLOGIE: linear

     (ii) ART DES MOLEKÜLS: Peptid


     (xi) SEQUENZBESCHREIBUNG: SEQ ID NO: 43:

```
Thr Arg Pro Ile Asp Thr Thr Ala Gly Pro Ser Asp Met Tyr Val
1               5                   10                  15
```

(2)  INFORMATION ZU SEQ ID NO: 44:

     (i)  SEQUENZ CHARAKTERISTIKA:
          (A) LÄNGE: 15 Aminosäuren
          (B) ART: Aminosäure
          (C) STRANGFORM: Einzel
          (D) TOPOLOGIE: linear

     (ii) ART DES MOLEKÜLS: Peptid

(xi) SEQUENZBESCHREIBUNG: SEQ ID NO: 44:

```
Thr Arg Pro Ile Phe Thr Thr Ala Gly Pro Ser Asp Met Tyr Val
1               5                   10                  15
```

(2) INFORMATION ZU SEQ ID NO: 45:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 45:

```
Thr Arg Pro Ile Ile Phe Thr Ala Gly Pro Ser Asp Met Tyr Val
1               5                   10                  15
```

(2) INFORMATION ZU SEQ ID NO: 46:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 46:

```
Thr Arg Pro Ile Ile Ser Thr Ala Gly Pro Ser Asp Met Tyr Val
1               5                   10                  15
```

(2) INFORMATION ZU SEQ ID NO: 47:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 47:

```
Thr Arg Pro Ile Ile Asp Thr Ala Gly Pro Ser Asp Met Tyr Val
1               5                   10                  15
```

(2) INFORMATION ZU SEQ ID NO: 48:

    (i) SEQUENZ CHARAKTERISTIKA:
        (A) LÄNGE: 15 Aminosäuren
        (B) ART: Aminosäure
        (C) STRANGFORM: Einzel

(D) TOPOLOGIE: linear

(ii) ART DES MOLEKÜLS: Peptid

(xi) SEQUENZBESCHREIBUNG: SEQ ID NO: 48:

Thr Arg Pro Ile Ile Lys Thr Ala Gly Pro Ser Asp Met Tyr Val
1               5                   10                  15

(2) INFORMATION ZU SEQ ID NO: 49:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 49:

Thr Arg Pro Ile Ile Thr Ala Ala Gly Pro Ser Asp Met Tyr Val
1               5                   10                  15

(2) INFORMATION ZU SEQ ID NO: 50:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 50:

Thr Arg Pro Ile Ile Thr Val Ala Gly Pro Ser Asp Met Tyr Val
1               5                   10                  15

(2) INFORMATION ZU SEQ ID NO: 51:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 51:

Thr Arg Pro Ile Ile Thr Leu Ala Gly Pro Ser Asp Met Tyr Val
1               5                   10                  15

(2) INFORMATION ZU SEQ ID NO: 52:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 52:

Thr Arg Pro Ile Ile Thr Ile Ala Gly Pro Ser Asp Met Tyr Val
1           5              10             15

(2) INFORMATION ZU SEQ ID NO: 53:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 53:

Thr Arg Pro Ile Ile Thr Pro Ala Gly Pro Ser Asp Met Tyr Val
1           5              10             15

(2) INFORMATION ZU SEQ ID NO: 54:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 54:

Thr Arg Pro Ile Ile Thr Phe Ala Gly Pro Ser Asp Met Tyr Val
1           5              10             15

(2) INFORMATION ZU SEQ ID NO: 55:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 55:

Thr Arg Pro Ile Ile Thr Gly Ala Gly Pro Ser Asp Met Tyr Val

      1              5                 10               15

(2) INFORMATION ZU SEQ ID NO: 56:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 56:

Thr Arg Pro Ile Ile Thr Ser Ala Gly Pro Ser Asp Met Tyr Val
1            5                10            15

(2) INFORMATION ZU SEQ ID NO: 57:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 57:

Thr Arg Pro Ile Ile Thr Tyr Ala Gly Pro Ser Asp Met Tyr Val
1            5                10            15

(2) INFORMATION ZU SEQ ID NO: 58:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 58:

Thr Arg Pro Ile Ile Thr Asn Ala Gly Pro Ser Asp Met Tyr Val
1            5                10            15

(2) INFORMATION ZU SEQ ID NO: 59:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 59:

Thr Arg Pro Ile Ile Thr Gln Ala Gly Pro Ser Asp Met Tyr Val
1              5                  10                 15

(2) INFORMATION ZU SEQ ID NO: 60:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 60:

Thr Arg Pro Ile Ile Thr Asp Ala Gly Pro Ser Asp Met Tyr Val
1              5                  10                 15

(2) INFORMATION ZU SEQ ID NO: 61:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 61:

Thr Arg Pro Ile Ile Thr Glu Ala Gly Pro Ser Asp Met Tyr Val
1              5                  10                 15

(2) INFORMATION ZU SEQ ID NO: 62:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 62:

Thr Arg Pro Ile Ile Thr Lys Ala Gly Pro Ser Asp Met Tyr Val
1              5                  10                 15

(2) INFORMATION ZU SEQ ID NO: 63:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

(ii) ART DES MOLEKÜLS: Peptid

(xi) SEQUENZBESCHREIBUNG: SEQ ID NO: 63:

Thr Arg Pro Ile Ile Thr Arg Ala Gly Pro Ser Asp Met Tyr Val
1               5               10                  15

(2) INFORMATION ZU SEQ ID NO: 64:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 64:

Thr Arg Pro Ile Ile Thr His Ala Gly Pro Ser Asp Met Tyr Val
1               5               10                  15

(2) INFORMATION ZU SEQ ID NO: 65:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 65:

Thr Arg Pro Ile Ile Thr Met Ala Gly Pro Ser Asp Met Tyr Val
1               5               10                  15

(2) INFORMATION ZU SEQ ID NO: 66:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 66:

Thr Arg Pro Ile Ile Thr Cys Ala Gly Pro Ser Asp Met Tyr Val
1               5               10                  15

(2) INFORMATION ZU SEQ ID NO: 67:

    (i) SEQUENZ CHARAKTERISTIKA:

```
                (A) LÄNGE: 15 Aminosäuren
                (B) ART: Aminosäure
                (C) STRANGFORM: Einzel
                (D) TOPOLOGIE: linear

        (ii) ART DES MOLEKÜLS: Peptid


        (xi) SEQUENZBESCHREIBUNG: SEQ ID NO: 67:

        Thr Arg Pro Ile Ile Thr Thr Val Gly Pro Ser Asp Met Tyr Val
        1               5                   10                  15

    (2) INFORMATION ZU SEQ ID NO: 68:

        (i) SEQUENZ CHARAKTERISTIKA:
                (A) LÄNGE: 15 Aminosäuren
                (B) ART: Aminosäure
                (C) STRANGFORM: Einzel
                (D) TOPOLOGIE: linear

        (ii) ART DES MOLEKÜLS: Peptid


        (xi) SEQUENZBESCHREIBUNG: SEQ ID NO: 68:

        Thr Arg Pro Ile Ile Thr Thr Leu Gly Pro Ser Asp Met Tyr Val
        1               5                   10                  15

    (2) INFORMATION ZU SEQ ID NO: 69:

        (i) SEQUENZ CHARAKTERISTIKA:
                (A) LÄNGE: 15 Aminosäuren
                (B) ART: Aminosäure
                (C) STRANGFORM: Einzel
                (D) TOPOLOGIE: linear

        (ii) ART DES MOLEKÜLS: Peptid


        (xi) SEQUENZBESCHREIBUNG: SEQ ID NO: 69:

        Thr Arg Pro Ile Ile Thr Thr Pro Gly Pro Ser Asp Met Tyr Val
        1               5                   10                  15

    (2) INFORMATION ZU SEQ ID NO: 70:

        (i) SEQUENZ CHARAKTERISTIKA:
                (A) LÄNGE: 15 Aminosäuren
                (B) ART: Aminosäure
                (C) STRANGFORM: Einzel
                (D) TOPOLOGIE: linear

        (ii) ART DES MOLEKÜLS: Peptid


        (xi) SEQUENZBESCHREIBUNG: SEQ ID NO: 70:

        Thr Arg Pro Ile Ile Thr Thr Phe Gly Pro Ser Asp Met Tyr Val
        1               5                   10                  15
```

44

(2) INFORMATION ZU SEQ ID NO: 71:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 71:

Thr Arg Pro Ile Ile Thr Thr Met Gly Pro Ser Asp Met Tyr Val
1               5               10              15

(2) INFORMATION ZU SEQ ID NO: 72:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 72:

Thr Arg Pro Ile Ile Thr Thr Thr Gly Pro Ser Asp Met Tyr Val
1               5               10              15

(2) INFORMATION ZU SEQ ID NO: 73:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

    (xi) SEQUENZBESCHREIBUNG: SEQ ID NO: 73:

Thr Arg Pro Ile Ile Thr Thr Tyr Gly Pro Ser Asp Met Tyr Val
1               5               10              15

(2) INFORMATION ZU SEQ ID NO: 74:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 74:

Thr Arg Pro Ile Ile Thr Thr Asn Gly Pro Ser Asp Met Tyr Val
1               5               10              15

(2) INFORMATION ZU SEQ ID NO: 75:

    (i) SEQUENZ CHARAKTERISTIKA:
       (A) LÄNGE: 15 Aminosäuren
       (B) ART: Aminosäure
       (C) STRANGFORM: Einzel
       (D) TOPOLOGIE: linear

    (ii) ART DES MOLEKÜLS: Peptid

(xi) SEQUENZBESCHREIBUNG: SEQ ID NO: 75:

Thr Arg Pro Ile Ile Thr Thr Gln Gly Pro Ser Asp Met Tyr Val
1               5               10              15

(2) INFORMATION ZU SEQ ID NO: 76:

    (i) SEQUENZ CHARAKTERISTIKA:
       (A) LÄNGE: 15 Aminosäuren
       (B) ART: Aminosäure
       (C) STRANGFORM: Einzel
       (D) TOPOLOGIE: linear

    (ii) ART DES MOLEKÜLS: Peptid

(xi) SEQUENZBESCHREIBUNG: SEQ ID NO: 76:

Thr Arg Pro Ile Ile Thr Thr Asp Gly Pro Ser Asp Met Tyr Val
1               5               10              15

(2) INFORMATION ZU SEQ ID NO: 77:

    (i) SEQUENZ CHARAKTERISTIKA:
       (A) LÄNGE: 15 Aminosäuren
       (B) ART: Aminosäure
       (C) STRANGFORM: Einzel
       (D) TOPOLOGIE: linear

    (ii) ART DES MOLEKÜLS: Peptid

(xi) SEQUENZBESCHREIBUNG: SEQ ID NO: 77:

Thr Arg Pro Ile Ile Thr Thr Glu Gly Pro Ser Asp Met Tyr Val
1               5               10              15

(2) INFORMATION ZU SEQ ID NO: 78:

    (i) SEQUENZ CHARAKTERISTIKA:
       (A) LÄNGE: 15 Aminosäuren
       (B) ART: Aminosäure
       (C) STRANGFORM: Einzel
       (D) TOPOLOGIE: linear

    (ii) ART DES MOLEKÜLS: Peptid

(xi) SEQUENZBESCHREIBUNG: SEQ ID NO: 78:

Thr Arg Pro Ile Ile Thr Thr Arg Gly Pro Ser Asp Met Tyr Val
1               5               10              15

(2) INFORMATION ZU SEQ ID NO: 79:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 79:

Thr Arg Pro Ile Ile Thr Thr Lys Gly Pro Ser Asp Met Tyr Val
1               5               10              15

(2) INFORMATION ZU SEQ ID NO: 80:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 80:

Thr Arg Pro Ile Ile Thr Thr Ser Gly Pro Ser Asp Met Tyr Val
1               5               10              15

(2) INFORMATION ZU SEQ ID NO: 81:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 81:

Thr Arg Pro Ile Ile Thr Thr His Gly Pro Ser Asp Met Tyr Val
1               5               10              15

(2) INFORMATION ZU SEQ ID NO: 82:

    (i) SEQUENZ CHARAKTERISTIKA:
        (A) LÄNGE: 14 Aminosäuren
        (B) ART: Aminosäure

```
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 82:

    Thr Arg Pro Ile Ile Thr Thr Cys Gly Pro Ser Asp Met Tyr Val
    1               5               10                  15

(2) INFORMATION ZU SEQ ID NO: 83:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 83:

    Thr Arg Pro Ile Ile Thr Thr Gly Gly Pro Ser Asp Met Tyr Val
    1               5           ·   10                  15

(2) INFORMATION ZU SEQ ID NO: 84:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


                    ·
    (xi) SEQUENZBESCHREIBUNG: SEQ ID NO: 84:

    Thr Arg Pro Ile Ile Thr Thr Ala Phe Pro Ser Asp Met Tyr Val
    1               5               10                  15

(2) INFORMATION ZU SEQ ID NO: 85:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 85:

    Thr Arg Pro Ile Ile Thr Thr Ala Thr Pro Ser Asp Met Tyr Val
    1               5               10                  15

(2) INFORMATION ZU SEQ ID NO: 86:
```

```
    (i)  SEQUENZ CHARAKTERISTIKA:
         (A)  LÄNGE: 15 Aminosäuren
         (B)  ART: Aminosäure
         (C)  STRANGFORM: Einzel
         (D)  TOPOLOGIE: linear

   (ii)  ART DES MOLEKÜLS: Peptid


   (xi)  SEQUENZBESCHREIBUNG: SEQ ID NO: 86:

   Thr Arg Pro Ile Ile Thr Thr Ala Asp Pro Ser Asp Met Tyr Val
   1               5                   10                  15

(2) INFORMATION ZU SEQ ID NO: 87:

    (i)  SEQUENZ CHARAKTERISTIKA:
         (A)  LÄNGE: 15 Aminosäuren
         (B)  ART: Aminosäure
         (C)  STRANGFORM: Einzel
         (D)  TOPOLOGIE: linear

   (ii)  ART DES MOLEKÜLS: Peptid


   (xi)  SEQUENZBESCHREIBUNG: SEQ ID NO: 87:

   Thr Arg Pro Ile Ile Thr Thr Ala Lys Pro Ser Asp Met Tyr Val
   1               5                   10                  15

(2) INFORMATION ZU SEQ ID NO: 88:

    (i)  SEQUENZ CHARAKTERISTIKA:
         (A)  LÄNGE: 15 Aminosäuren
         (B)  ART: Aminosäure
         (C)  STRANGFORM: Einzel
         (D)  TOPOLOGIE: linear

   (ii)  ART DES MOLEKÜLS: Peptid


   (xi)  SEQUENZBESCHREIBUNG: SEQ ID NO: 88:

   Thr Arg Pro Ile Ile Thr Thr Ala Leu Pro Ser Asp Met Tyr Val
   1               5                   10                  15

(2) INFORMATION ZU SEQ ID NO: 89:

    (i)  SEQUENZ CHARAKTERISTIKA:
         (A)  LÄNGE: 15 Aminosäuren
         (B)  ART: Aminosäure
         (C)  STRANGFORM: Einzel
         (D)  TOPOLOGIE: linear

   (ii)  ART DES MOLEKÜLS: Peptid


   (xi)  SEQUENZBESCHREIBUNG: SEQ ID NO: 89:
```

```
Thr Arg Pro Ile Ile Thr Thr Ala Gln Pro Ser Asp Met Tyr Val
1               5                   10                  15
```

(2) INFORMATION ZU SEQ ID NO: 90:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 90:

```
Thr Arg Pro Ile Ile Thr Thr Ala Cys Pro Ser Asp Met Tyr Val
1               5                   10                  15
```

(2) INFORMATION ZU SEQ ID NO: 91:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 91:

```
Thr Arg Pro Ile Ile Thr Thr Ala Arg Pro Ser Asp Met Tyr Val
1               5                   10                  15
```

(2) INFORMATION ZU SEQ ID NO: 92:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 92:

```
Thr Arg Pro Ile Ile Thr Thr Ala Pro Pro Ser Asp Met Tyr Val
1               5                   10                  15
```

(2) INFORMATION ZU SEQ ID NO: 93:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 93:

Thr Arg Pro Ile Ile Thr Thr Ala Tyr Pro Ser Asp Met Tyr Val
1               5               10                  15

(2) INFORMATION ZU SEQ ID NO: 94:

   (i) SEQUENZ CHARAKTERISTIKA:
      (A) LÄNGE: 15 Aminosäuren
      (B) ART: Aminosäure
      (C) STRANGFORM: Einzel
      (D) TOPOLOGIE: linear

   (ii) ART DES MOLEKÜLS: Peptid

(xi) SEQUENZBESCHREIBUNG: SEQ ID NO: 94:

Thr Arg Pro Ile Ile Thr Thr Ala Glu Pro Ser Asp Met Tyr Val
1               5               10                  15

(2) INFORMATION ZU SEQ ID NO: 95:

   (i) SEQUENZ CHARAKTERISTIKA:
      (A) LÄNGE: 15 Aminosäuren
      (B) ART: Aminosäure
      (C) STRANGFORM: Einzel
      (D) TOPOLOGIE: linear

   (ii) ART DES MOLEKÜLS: Peptid

(xi) SEQUENZBESCHREIBUNG: SEQ ID NO: 95:

Thr Arg Pro Ile Ile Thr Thr Ala Ile Pro Ser Asp Met Tyr Val
1               5               10                  15

(2) INFORMATION ZU SEQ ID NO: 96:

   (i) SEQUENZ CHARAKTERISTIKA:
      (A) LÄNGE: 15 Aminosäuren
      (B) ART: Aminosäure
      (C) STRANGFORM: Einzel
      (D) TOPOLOGIE: linear

   (ii) ART DES MOLEKÜLS: Peptid

(xi) SEQUENZBESCHREIBUNG: SEQ ID NO: 96:

Thr Arg Pro Ile Ile Thr Thr Ala Asn Pro Ser Asp Met Tyr Val
1               5               10                  15

(2) INFORMATION ZU SEQ ID NO: 97:

   (i) SEQUENZ CHARAKTERISTIKA:
      (A) LÄNGE: 15 Aminosäuren
      (B) ART: Aminosäure
      (C) STRANGFORM: Einzel

(D) TOPOLOGIE: linear

(ii) ART DES MOLEKÜLS: Peptid

(xi) SEQUENZBESCHREIBUNG: SEQ ID NO: 97:

Thr Arg Pro Ile Ile Thr Thr Ala Ala Pro Ser Asp Met Tyr Val
1               5                   10                  15

(2) INFORMATION ZU SEQ ID NO: 98:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 98:

Thr Arg Pro Ile Ile Thr Thr Ala Ser Pro Ser Asp Met Tyr Val
1               5                   10                  15

(2) INFORMATION ZU SEQ ID NO: 99:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 99:

Thr Arg Pro Ile Ile Thr Thr Ala Val Pro Ser Asp Met Tyr Val
1               5                   10                  15

(2) INFORMATION ZU SEQ ID NO: 100:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 100:

Thr Arg Pro Ile Ile Thr Thr Ala Met Pro Ser Asp Met Tyr Val
1               5                   10                  15

(2) INFORMATION ZU SEQ ID NO: 101:

         (i) SEQUENZ CHARAKTERISTIKA:
             (A) LÄNGE: 15 Aminosäuren
             (B) ART: Aminosäure
             (C) STRANGFORM: Einzel
             (D) TOPOLOGIE: linear

        (ii) ART DES MOLEKÜLS: Peptid


        (xi) SEQUENZBESCHREIBUNG: SEQ ID NO: 101:

        Thr Arg Pro Ile Ile Thr Thr Ala His Pro Ser Asp Met Tyr Val
        1               5                   10                  15

   (2) INFORMATION ZU SEQ ID NO: 102:

         (i) SEQUENZ CHARAKTERISTIKA:
             (A) LÄNGE: 15 Aminosäuren
             (B) ART: Aminosäure
             (C) STRANGFORM: Einzel
             (D) TOPOLOGIE: linear

        (ii) ART DES MOLEKÜLS: Peptid


        (xi) SEQUENZBESCHREIBUNG: SEQ ID NO: 102:

        Thr Arg Pro Ile Ile Thr Thr Ala Gly Phe Ser Asp Met Tyr Val
        1               5                   10                  15

   (2) INFORMATION ZU SEQ ID NO: 103:

         (i) SEQUENZ CHARAKTERISTIKA:
             (A) LÄNGE: 15 Aminosäuren
             (B) ART: Aminosäure
             (C) STRANGFORM: Einzel
             (D) TOPOLOGIE: linear

        (ii) ART DES MOLEKÜLS: Peptid


        (xi) SEQUENZBESCHREIBUNG: SEQ ID NO: 103:

        Thr Arg Pro Ile Ile Thr Thr Ala Gly Thr Ser Asp Met Tyr Val
        1               5                   10                  15

   (2) INFORMATION ZU SEQ ID NO: 104:

         (i) SEQUENZ CHARAKTERISTIKA:
             (A) LÄNGE: 15 Aminosäuren
             (B) ART: Aminosäure
             (C) STRANGFORM: Einzel
             (D) TOPOLOGIE: linear

        (ii) ART DES MOLEKÜLS: Peptid


        (xi) SEQUENZBESCHREIBUNG: SEQ ID NO: 104:

        Thr Arg Pro Ile Ile Thr Thr Ala Gly Asp Ser Asp Met Tyr Val

```
              1                5                     10                    15
```

(2)  INFORMATION ZU SEQ ID NO: 105:

    (i)  SEQUENZ CHARAKTERISTIKA:
        (A)  LÄNGE: 15 Aminosäuren
        (B)  ART: Aminosäure
        (C)  STRANGFORM: Einzel
        (D)  TOPOLOGIE: linear

    (ii)  ART DES MOLEKÜLS: Peptid

    (xi)  SEQUENZBESCHREIBUNG: SEQ ID NO: 105:

```
Thr Arg Pro Ile Ile Thr Thr Ala Gly Lys Ser Asp Met Tyr Val
1               5                   10                  15
```

(2)  INFORMATION ZU SEQ ID NO: 106:

    (i)  SEQUENZ CHARAKTERISTIKA:
        (A)  LÄNGE: 15 Aminosäuren
        (B)  ART: Aminosäure
        (C)  STRANGFORM: Einzel
        (D)  TOPOLOGIE: linear

    (ii)  ART DES MOLEKÜLS: Peptid

    (xi)  SEQUENZBESCHREIBUNG: SEQ ID NO: 106:

```
Thr Arg Pro Ile Ile Thr Thr Ala Gly Pro Thr Asp Met Tyr Val
1               5                   10                  15
```

(2)  INFORMATION ZU SEQ ID NO: 107:

    (i)  SEQUENZ CHARAKTERISTIKA:
        (A)  LÄNGE: 15 Aminosäuren
        (B)  ART: Aminosäure
        (C)  STRANGFORM: Einzel
        (D)  TOPOLOGIE: linear

    (ii)  ART DES MOLEKÜLS: Peptid

    (xi)  SEQUENZBESCHREIBUNG: SEQ ID NO: 107:

```
Thr Arg Pro Ile Ile Thr Thr Ala Gly Pro Asp Asp Met Tyr Val
1               5                   10                  15
```

(2)  INFORMATION ZU SEQ ID NO: 108:

    (i)  SEQUENZ CHARAKTERISTIKA:
        (A)  LÄNGE: 15 Aminosäuren
        (B)  ART: Aminosäure
        (C)  STRANGFORM: Einzel
        (D)  TOPOLOGIE: linear

    (ii)  ART DES MOLEKÜLS: Peptid

(xi) SEQUENZBESCHREIBUNG: SEQ ID NO: 108:

Thr Arg Pro Ile Ile Thr Thr Ala Gly Pro Lys Asp Met Tyr Val
1               5                   10                  15

(2) INFORMATION ZU SEQ ID NO: 109:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 109:

Thr Arg Pro Ile Ile Thr Thr Ala Gly Pro Phe Asp Met Tyr Val
1               5                   10                  15

(2) INFORMATION ZU SEQ ID NO: 110:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 110:

Thr Arg Pro Ile Ile Thr Thr Ala Gly Pro Phe Asp Met Tyr Val
1               5                   10                  15

(2) INFORMATION ZU SEQ ID NO: 111:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 111:

Thr Arg Pro Ile Ile Thr Thr Ala Gly Pro Ser Phe Met Tyr Val
1               5                   10                  15

(2) INFORMATION ZU SEQ ID NO: 112:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

(ii) ART DES MOLEKÜLS: Peptid

(xi) SEQUENZBESCHREIBUNG: SEQ ID NO: 112:

```
Thr Arg Pro Ile Ile Thr Thr Ala Gly Pro Ser Thr Met Tyr Val
1               5                   10                  15
```

(2) INFORMATION ZU SEQ ID NO: 113:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NOD: 113:

```
Thr Arg Pro Ile Ile Thr Thr Ala Gly Pro Ser Glu Met Tyr Val
1               5                   10                  15
```

(2) INFORMATION ZU SEQ ID NO: 114:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 114:

```
Thr Arg Pro Ile Ile Thr Thr Ala Gly Pro Ser Asp Thr Tyr Val
1               5                   10                  15
```

(2) INFORMATION ZU SEQ ID NO: 115:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 115:

```
Thr Arg Pro Ile Ile Thr Thr Ala Gly Pro Ser Asp Phe Tyr Val
1               5                   10                  15
```

(2) INFORMATION ZU SEQ ID NO: 116:

    (i) SEQUENZ CHARAKTERISTIKA:

        (A) LÄNGE: 15 Aminosäuren
        (B) ART: Aminosäure
        (C) STRANGFORM: Einzel
        (D) TOPOLOGIE: linear

   (ii) ART DES MOLEKÜLS: Peptid


   (xi) SEQUENZBESCHREIBUNG: SEQ ID NO: 116:

Thr Arg Pro Ile Ile Thr Thr Ala Gly Pro Ser Asp Lys Tyr Val
1          5               10             15

(2) INFORMATION ZU SEQ ID NO: 117:

   (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

   (ii) ART DES MOLEKÜLS: Peptid


   (xi) SEQUENZBESCHREIBUNG: SEQ ID NO: 117:

Thr Arg Pro Ile Ile Thr Thr Ala Gly Pro Ser Asp Asp Tyr Val
1          5               10             15

(2) INFORMATION ZU SEQ ID NO: 118:

   (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

   (ii) ART DES MOLEKÜLS: Peptid


   (xi) SEQUENZBESCHREIBUNG: SEQ ID NO: 118:

Thr Arg Pro Ile Ile Thr Thr Ala Gly Pro Ser Asp Met Asp Val
1          5               10             15

(2) INFORMATION ZU SEQ ID NO: 119:

   (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

   (ii) ART DES MOLEKÜLS: Peptid


   (xi) SEQUENZBESCHREIBUNG: SEQ ID NO: 119:

Thr Arg Pro Ile Ile Thr Thr Ala Gly Pro Ser Asp Met Lys Val
1          5               10             15

(2) INFORMATION ZU SEQ ID NO: 120:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 120:

```
Thr Arg Pro Ile Ile Thr Thr Ala Gly Pro Ser Asp Met Phe Val
1               5               10              15
```

(2) INFORMATION ZU SEQ ID NO: 121:

    (i) SEQUENZ CHARAKTERISTIKA:
        (A) LÄNGE: 15 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 121:

```
Thr Arg Pro Ile Ile Thr Thr Ala Gly Pro Ser Asp Met Thr Val
1               5               10              15
```

(2) INFORMATION ZU SEQ ID NO: 122:

    (i) SEQUENZ CHARAKTERISTIKA:
        (A) LÄNGE: 16 Aminosäuren
        (B) ART: Aminosäure
        (C) STRANGFORM: Einzel
        (D) TOPOLOGIE: linear

    (ii) ART DES MOLEKÜLS: Peptid


    (xi) SEQUENZBESCHREIBUNG: SEQ ID NO: 122:

```
Thr Arg Leu Gln Gly Ile Asn Cys Gly Pro Asp Phe Thr Pro Ser Phe
1               5               10              15
```

(2) INFORMATION ZU SEQ ID NO: 123:

    (i) SEQUENZ CHARAKTERISTIKA:
        (A) LÄNGE: 16 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 123:

Glu Leu Ala Arg Gly Ala Gln Ala Gly Leu Gly Pro Arg Arg Ser Gln
1            5                10              15

(2) INFORMATION ZU SEQ ID NO: 124:

    (i) SEQUENZ CHARAKTERISTIKA:
        (A) LÄNGE: 16 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 124:

Leu Cys Arg Leu Leu Thr Thr Ile Gly Lys Asp Leu Asp Phe Glu Lys
1            5                10              15

(2) INFORMATION ZU SEQ ID NO: 125:

    (i) SEQUENZ CHARAKTERISTIKA:
        (A) LÄNGE: 16 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 125:

Gly Arg Thr Thr Leu Ser Thr Arg Gly Pro Pro Arg Gly Gly Pro Gly
1            5                10              15

(2) INFORMATION ZU SEQ ID NO: 126:

    (i) SEQUENZ CHARAKTERISTIKA:
        (A) LÄNGE: 16 Aminosäuren
        (B) ART: Aminosäure
        (C) STRANGFORM: Einzel
        (D) TOPOLOGIE: linear

    (ii) ART DES MOLEKÜLS: Peptid

(xi) SEQUENZBESCHREIBUNG: SEQ ID NO: 126:

Gly Ser Ser Gly Gly Ser Gly Ala Gln Pro Ser Asp Ala Ala Ser Glu
1            5                10              15

(2) INFORMATION ZU SEQ ID NO: 127:

    (i) SEQUENZ CHARAKTERISTIKA:

```
        (A)  LÄNGE: 17 Aminosäuren
        (B)  ART: Aminosäure
        (C)  STRANGFORM: Einzel
        (D)  TOPOLOGIE: linear

  (ii)  ART DES MOLEKÜLS: Peptid


  (xi)  SEQUENZBESCHREIBUNG: SEQ ID NO: 127:

  Glu Arg Phe Ala Ile Lys Thr Met Gly Pro Lys Phe His Gln Ser
  1               5               10              15

  Gly


(2)  INFORMATION ZU SEQ ID NO: 128:

      (i)  SEQUENZ CHARAKTERISTIKA:
           (A)  LÄNGE: 16 Aminosäuren
           (B)  ART: Aminosäure
           (C)  STRANGFORM: Einzel
           (D)  TOPOLOGIE: linear

      (ii)  ART DES MOLEKÜLS: Peptid



      (xi)  SEQUENZBESCHREIBUNG: SEQ ID NO: 128:

      Glu Phe Pro Ala Ile Lys Thr Met Gly Pro Lys Phe His Gln Ser Gly
      1               5               10              15


(2)  INFORMATION ZU SEQ ID NO: 129:

      (i)  SEQUENZ CHARAKTERISTIKA:
           (A)  LÄNGE: 16 Aminosäuren
           (B)  ART: Aminosäure
           (C)  STRANGFORM: Einzel
           (D)  TOPOLOGIE: linear

      (ii)  ART DES MOLEKÜLS: Peptid



      (xi)  SEQUENZBESCHREIBUNG: SEQ ID NO: 129:

      Thr Arg Pro Ile Ile Thr Thr Ala Gly Pro Tyr Gly His Gln Ser Gly
      1               5               10              15


(2)  INFORMATION ZU SEQ ID NO: 130:

      (i)  SEQUENZ CHARAKTERISTIKA:
           (A)  LÄNGE: 16 Aminosäuren
           (B)  ART: Aminosäure
           (C)  STRANGFORM: Einzel
           (D)  TOPOLOGIE: linear

      (ii)  ART DES MOLEKÜLS: Peptid
```

(xi) SEQUENZBESCHREIBUNG: SEQ ID NO: 130:

Glu Arg Ala Ser Leu Ile Thr Thr Gly Pro Ser Asp Met Tyr Val His
1               5                   10                  15

(2) INFORMATION ZU SEQ ID NO: 131:

  (i) SEQUENZ CHARAKTERISTIKA:
    (A) LÄNGE: 16 Aminosäuren
    (B) ART: Aminosäure
    (C) STRANGFORM: Einzel
    (D) TOPOLOGIE: linear

  (ii) ART DES MOLEKÜLS: Peptid

(xi) SEQUENZBESCHREIBUNG: SEQ ID NO: 131:

Glu Arg Pro Ser Leu Thr Thr Ala Gly Pro Ser Asp Met Tyr Val His
1               5                   10                  15

(2) INFORMATION ZU SEQ ID NO: 132:

  (i) SEQUENZ CHARAKTERISTIKA:
    (A) LÄNGE: 16 Aminosäuren
    (B) ART: Aminosäure
    (C) STRANGFORM: Einzel
    (D) TOPOLOGIE: linear

  (ii) ART DES MOLEKÜLS: Peptid

(xi) SEQUENZBESCHREIBUNG: SEQ ID NO: 132:

Glu Arg Ala Ser Leu Ile Thr Val Gly Pro Ser Asp Met Tyr Val His
1               5                   10                  15

(2) INFORMATION ZU SEQ ID NO: 133:

  (i) SEQUENZ CHARAKTERISTIKA:
    (A) LÄNGE: 16 Aminosäuren
    (B) ART: Aminosäure
    (C) STRANGFORM: Einzel
    (D) TOPOLOGIE: linear

  (ii) ART DES MOLEKÜLS: Peptid

(xi) SEQUENZBESCHREIBUNG: SEQ ID NO: 133:

Glu Arg Ala Ser Leu Ile Thr Thr Gly Pro Tyr Gly Met Gln Ser Gly
1               5                   10                  15

(2) INFORMATION ZU SEQ ID NO: 134:

  (i) SEQUENZ CHARAKTERISTIKA:

```
(A) LÄNGE: 16 Aminosäuren
(B) ART: Aminosäure
(C) STRANGFORM: Einzel
(D) TOPOLOGIE: linear

(ii) ART DES MOLEKÜLS: Peptid


(xi) SEQUENZBESCHREIBUNG: SEQ ID NO: 134:

Glu Arg Ala Ser Leu Ile Thr Thr Gly Pro Tyr Gly His Gln Ser Gly
 1               5                   10                  15
```

## Patentansprüche

**1.** Verfahren zur Analyse des durch Picornaviren verursachten "host cell shut-offs", dadurch gekennzeichnet, daß

a) eine RNA abgeleitet von einem Gen mit mindestens einem internen Translationsstart mit einer Cap-Struktur versehen wird,

b) in einer Translationsmischung, die mit einer picornaviralen Proteinase vorinkubiert wurde, in vitro translatiert wird und

c) die Translationsprodukte analysiert werden.

**2.** Verfahren nach Anspruch 1, dadurch gekennzeichnet, daß die in vitro translatierten Proteine markiert sind.

**3.** Verfahren gemäß Anspruch 1, dadurch gekennzeichnet, daß die erhaltenen Proteine mit Translationsprodukten der RNA ohne Cap-Struktur und/oder ohne Vorinkubation der Translationsmischung mit einer picornaviralen Proteinase verglichen werden.

**4.** Verfahren nach Anspruch 1, dadurch gekennzeichnet, daß die RNA durch in vitro Transkription eines picornaviralen 2C Gens, bevorzugt eines rhinoviralen 2C Gens, erhalten wird.

**5.** Verfahren nach Anspruch 1, dadurch gekennzeichnet, daß die RNA durch in vitro Transkription der Plasmide pHRV2/5'UTR-2C, pβ-2C, pLink-2C oder pLink-2BC erhalten wird.

**6.** Verfahren nach Anspruch 1, dadurch gekennzeichnet, daß für das Verfahren eine lösliche, mature picornavirale 2A Proteinase verwendet wird.

**7.** Verfahren nach Anspruch 1, dadurch gekennzeichnet, daß zur Vorinkubation der Translationsmischung die rhinovirale 2A Proteinase, insbesondere die HRV2 2A Proteinase, in Form eines angereicherten Rohextraktes oder in reiner Form verwendet wird.

**8.** Verfahren nach Anspruch 1, dadurch gekennzeichnet, daß die Translationsmischung aus einem Reticulozytenlysat oder einem HeLa-Zellextrakt oder fraktionierten Anteilen dieser Translationssysteme besteht.

**9.** Verfahren nach Anspruch 1 zur Analyse des durch Rhinoviren verursachten "host cell shut-offs", dadurch gekennzeichnet, daß

a) das HRV2 2C-Gen mit Start- und Stopcodon ausgestattet, in vitro transkribiert und das Transkript mit einer Cap-Struktur versehen wird und

b) in einer Translationsmischung, die mit einer rhinoviralen 2A Proteinase vorinkubiert ist, translatiert und

c) die translatierten Proteine mit markierten Translationsprodukten der RNA ohne Cap-Struktur mittels Elektrophorese und Autoradiographie verglichen werden.

10. Verwendung des Verfahrens nach Anspruch 1 zum Nachweis und zur Isolierung der zellulären und der viralen Proteine, die am "host cell shut-off" beteiligt sind.

11. Verwendung des Verfahrens nach Anspruch 1 zur Isolierung von Inhibitoren des "host cell shut-offs".

12. Verwendung des Verfahrens nach Anspruch 1 zur Isolierung von Inhibitoren der rhinoviralen Proteinasen.

13. Plasmide pHRV2/5'UTR-2C, pLink-2C, pLink-2BC, pSVL-2BC, pSVL-2C und p$\beta$-2C.

14. Verfahren zur rekombinanten Herstellung einer löslichen, maturen, picornaviralen 2A Proteinase, dadurch gekennzeichnet, daß ein Genfragment für das C-terminale Ende des VP1 Proteins und darauf folgend das vollständige Gen für die 2A Proteinase in den Expressionsvektor pET8c oder pET3b kloniert zur Expression gebracht wird.

15. Verfahren nach Anspruch 14, dadurch gekennzeichnet, daß die 2A Proteinase in dem E. coli Stamm BL21(DE3)LysS bzw. -LysE als Fusionsprotein exprimiert und durch autokatalytische Aktivität die mature 2A Proteinase freigesetzt wird.

16. Verfahren nach Anspruch 14, dadurch gekennzeichnet, daß für das C-terminale Ende des VP1 Proteins und das vollständige Gen für die 2A Proteinase eine DNA-Sequenz codierend für die Aminosäuresequenz SEQ ID No. 1 verwendet wird.

17. Plasmide pET8c/2A und pET/2A.

18. Verfahren zur Reinigung einer löslichen, maturen rhinoviralen Proteinase 2A, dadurch gekennzeichnet, daß
    a) die Proteinase in einem heterologen Wirt exprimiert,
    b) die die Proteinase enthaltenden Zellen lysiert,
    c) die Proteinase aus dem Zellüberstand durch fraktionierte Ammoniumsulfatfällung konzentriert und
    d) durch Ionenaustausch- und Gelchromatographie gereinigt wird.

19. Inhibitor der rhinoviralen HRV2 2A Proteinase, abgeleitet aus der natürlichen Spaltungssequenz TRPIITTAGPSPMYVH (SEQ ID NO. 23), dadurch gekennzeichnet, daß er mindestens 10, höchstens 16 Aminosäuren umfaßt und das Aminosäuremotiv R-X-X-I/L-X-T-X-G-P enthält, wobei X eine beliebige Aminosäure ist.

20. Inhibitor gemäß Anspruch 19, dadurch gekennzeichnet, daß Position P1 Methionin ist.

21. Inhibitor gemäß Anspruch 19, dadurch gekennzeichnet, daß es P8F (SEQ ID NO. 26), P8K (SEQ ID NO. 28), P3K (SEQ ID NO. 48), P1C (SEQ ID NO. 82), P4'K (SEQ ID NO. 110), P4'F (SEQ ID NO. 111, P4'T (SEQ ID NO. 112), P5'K (SEQ ID NO. 116) oder MMP-2 (SEQ ID NO. 127) ist.

22. Inhibitor der rhinoviralen HRV2 2A Proteinase, abgeleitet aus der p220 Sequenz, dadurch gekennzeichnet, daß er das Aminosäuremotiv R-X-X-I/L-X-T-X-G-P enthält, wobei X eine beliebige Aminosäure ist.

23. Inhibitor der rhinoviralen HRV2 2A Proteinase, abgeleitet aus der p220 Sequenz, dadurch gekennzeichnet, daß er p220-3 (SEQ ID NO. 124) oder p220-4 (SEQ ID NO. 125) ist.

24. Verwendung eines Inhibitors gemäß der Ansprüchen 19-23 zur Herstellung eines Arzneimittels zur Inhibierung der HRV 2A Proteinase.

25. Mittel enthaltend einen Inhibitor gemäß den Ansprüchen 19-23, gegebenenfalls mit Hilfs- und/oder Trägerstoffen und/oder Stabilisatoren, zur Inhibierung der HRV 2A Proteinase.

26. Verfahren zur Herstellung der Peptide gemäß den Ansprüchen 19-23, dadurch gekennzeichnet, daß sie nach der Fmoc-Strategie mit aktivierten Estern oder über HOBt/DIPCDI-Aktivierung sowie nach Analogieverfahren hergestellt werden.

# Fig. 1A

pUC18-2C3A

BspM-l    BspM-l

BspM-l

BspM-l

BspM-l

BspM-l

S → pUC18-2C*3A

stop

durch Fragment aus Blue(+)-2C*3A ersetzt

Fig. 1B

EP 0 564 801 A1

# Fig. 2

```
                2C                          3A


                                 v
        V   D   V   M   T   A   I   F   Q   G   P   I   D   M   K   N   P
  ....gttgatgtcatgacagctatattccaagggccaattgatatgaaaaaccca...
      caactacagtactgtcaatataaggttcccggttaactatacttttgggt

                                              SEQ ID No: 3
```

Mutagenese mittels Oligonukleotid
EBI 1031

```
                    3' atataaggttatcggttaacta 5'

                              SEQ ID No: 2

                2C                          3A



        V   D   V   M   T   A   I   F   Q   *
  ....gttgatgtcatgacagctatattccaatagccaattgatatgaaaaaccca...
      caactacagtactgtcaatataaggttatcgttaactatacttttgggt

                              SEQ ID No: 4
```

Fig. 3

# Fig. 4

EBI 1102/1094:

```
                    M   G   V   T   D   Y   I   H
5'      ctagaccaccatggggggttacagattatataca          3'
3'          tggtggtaccccccattgtctaatatatgtat         5'
                      SEQ ID No: 5
        XbaI                                    NdeI
```

EBI 1099/1092:

```
                    M   S   D   S   W   L  .K   K   F   T   E   A   C
5'      ctagaccaccatgtcagattcatggttaaagaaatttactgaagcatg     3'
3'          tggtggtacagtctaagtaccaatttctttaaatgacttc          5'
                              SEQ ID No: 6
        XbaI                                              SphI
```

# Fig. 5

EP 0 564 801 A1

**A**

VP4 VP2    VP3    VP1    2A    2B    2C    VPg    3A    3C    3D

HRV2

|200bp|

2C

S|                                                                    Ba
19

611  3872                                                    4837  pHRV2/5'UTR-2C

S|                                                                    Ba
3872                                                          4837  p ß-2C

S|                                                                    Ba
3872                                                          4837  pLink-2C

2B                                                               2C

S|                                                                    Ba
3587                                                          4837  pLink-2BC

70

**Fig. 6**

# Fig. 7

EBI 1264/1250:

```
  5'    acacttgcttttgacacaactgtgtttacttgcaatcccccaaaacagacag     3'
  3'    tgtgaacgaaaactgtgttgacacaaatgaacgttaggggggttttgtctgtcgtac 5'
```

                                                                SEQ ID No: 7

EBI 1631/1634:

```
        M   S   D   S   W   L   K   K   F   T   E   A
  5'      catgtcagattcatggttaaagaaatttactgaagcatg         3'
  3'        agtctaagtaccaatttctttaaatgacttc
```

                          SEQ ID No: 8
      NcoI                                    SphI

# Fig. 8

Fig. 9

Fig. 10

Fig. 11

# Fig. 12

# Fig. 13

# Fig. 14

FRACTION NO.

44 ⟵⟶ 33M

—36
—26

2A→

I⟵⟶I⟵⟶I

POOL A          POOL B

# Fig. 15

M   1   2   3   4

36—
26—

←2A

Fig. 16

1868:

## Fig. 17

5'      gggggtcaagatactga     3'

SEQ ID No: 9

## Fig. 18

M  1  2  3  4  5  6  7  8

46 —

30 —

—37
—33
—28
—20

## Fig. 19

1  2  3  4  5  6  7

—37
—33

# Fig. 20

**A**

| Konstruktion | Sequenz | Molekulargewicht des Translations-produktes (kD) |
|---|---|---|
| pLink-2C | accaccAUGuca (SEQ ID No: 10) | 37 |
| pß-2C | gacagcAUGuca (SEQ ID No: 13) | 37 |
| pHRV2/5'UTR-2C AUG 611 | ggcaccAUGuca (SEQ ID No: 12) | 37 |
| AUG 449 | cccugaAUGugg | 43 |
| 2BC pLink-2BC | accaccAUGggg (SEQ ID No: 14) | 47 |
| Kozak Konsensus | ccaccAUGg g (SEQ ID No: 15) | |

**B**

| Molekulargewicht (kD) | Sequenz | Position |
|---|---|---|
| 2C 33 | gaauggAUGaag (SEQ ID No: 16) | 3965 |
| | aagucgAUGcuc (SEQ ID No: 17) | 3974 |
| 28 | gcugacAUGaaa (SEQ ID No: 18) | 4070 |
| | auuaaaAUGgaa (SEQ ID No: 19) | 4094 |
| 20 | gccaaaAUGaua (SEQ ID No: 20) | 4289 |
| 2BC 43 | aaauggAUGuug (SEQ ID No: 21) | 3710 |
| | ucagcaAUGguc (SEQ ID No: 22) | 3731 |

Fig. 21

# Fig. 22A

↓VP1

```
Asn Pro Val Glu Asn Tyr Ile Asp Glu Val Leu Asn Glu Val Leu Val
1               5                   10                  15

Val Pro Asn Ile Asn Ser Ser Asn Pro Thr Thr Ser Asn Ser Ala Pro
            20                  25                  30

Ala Leu Asp Ala Ala Glu Thr Gly His Thr Ser Ser Val Gln Pro Glu
            35                  40                  45

Asp Val Ile Glu Thr Arg Tyr Val Gln Thr Ser Gln Thr Arg Asp Glu
            50                  55                  60

Met Ser Leu Glu Ser Phe Leu Gly Arg Ser Gly Cys Ile His Glu Ser
65                  70                  75                  80

Lys Leu Glu Val Thr Leu Ala Asn Tyr Asn Lys Glu Asn Phe Thr Val
                85                  90                  95

Trp Ala Ile Asn Leu Gln Glu Met Ala Gln Ile Arg Arg Lys Phe Glu
                100                 105                 110

Leu Phe Thr Tyr Thr Arg Phe Asp Ser Glu Ile Thr Leu Val Pro Cys
            115                 120                 125

Ile Ser Ala Leu Ser Gln Asp Ile Gly His Ile Thr Met Gln Tyr Met
            130                 135                 140

Tyr Val Pro Pro Gly Ala Pro Val Pro Asn Ser Arg Asp Asp Tyr Ala
145                 150                 155                 160

Trp Gln Ser Gly Thr Asn Ala Ser Val Phe Trp Gln His Gly Gln Ala
                165                 170                 175

Tyr Pro Arg Phe Ser Leu Pro Phe Leu Ser Val Ala Ser Ala Tyr Tyr
            180                 185                 190

Met Phe Tyr Asp Gly Tyr Asp Glu Gln Asp Gln Asn Tyr Gly Thr Ala
            195                 200                 205

Asn Thr Asn Asn Met Gly Ser Leu Cys Ser Arg Ile Val Thr Glu Lys
    210                 215                 220

His Ile His Lys Val His Ile Met Thr Arg Ile Tyr His Lys Ala Lys
225                 230                 235                 240

His Val Lys Ala Trp Cys Pro Arg Pro Arg Ala Leu Glu Tyr Thr
                245                 250                 255
```

# Fig. 22B

```
Arg Ala His Arg Thr Asn Phe Lys Ile Glu Asp Arg Ser Ile Gln Thr
          260                 265        ↓ P2A      270

Ala Ile Val Thr Arg Pro Ile Ile Thr Thr Ala Gly Pro Ser Asp Met
          275             280         285

Tyr Val His Val Gly Asn Leu Ile Tyr Arg Asn Leu His Leu Phe Asn
          290             295             300

Ser Glu Met His Glu Ser Ile Leu Val Ser Tyr Ser Ser Asp Leu Ile
305                 310             315                 320

Ile Tyr Arg Thr Asn Thr Val Gly Asp Asp Tyr Ile Pro Ser Cys Asp
              325             330             335

Cys Thr Gln Ala Thr Tyr Tyr Cys Lys His Lys Asn Arg Tyr Phe Pro
              340             345             350

Ile Thr Val Thr Ser His Asp Trp Tyr Glu Ile Gln Glu Ser Glu Tyr
          355             360             365

Tyr Pro Lys His Ile Gln Tyr Asn Leu Leu Ile Gly Glu Gly Pro Cys
          370             375             380

Glu Pro Gly Asp Cys Gly Gly Lys Leu Leu Cys Lys His Gly Val Ile
385             390             395                 400

Gly Ile Val Thr Ala Gly Gly Asp Asn His Val Ala Phe Ile Asp Leu
              405             410             415

Arg His Phe His Cys Ala Glu Glu Gln          SEQ ID No: 1
              420             425
```

# Fig. 23A

| Bezeichnung | Sequenz | $(V_{max}/K_m)_{rel}$ | |
|---|---|---|---|
| | P1 P1* | | |
| P8-P8' | TRPIITTA*GPSDMYVH | 1.00±0.05 | SEQ ID NO. 23 |
| P8-P7' | TRPIITTA*GPSDMYV | 0.96±0.08 | SEQ ID NO. 24 |

==================================================================

a)

| P8D | dRPIITTA*GPSDMYV | 0.56±0.03 | SEQ ID NO. 25 |
|---|---|---|---|
| P8F | fRPIITTA*GPSDMYV | 1.51±0.21 | SEQ ID NO. 26 |
| P8N | nRPIITTA*GPSDMYV | 0.95±0.03 | SEQ ID NO. 27 |
| P8K | kRPIITTA*GPSDMYV | 1.26±0.10 | SEQ ID NO. 28 |

------------------------------------------------------------------

| P7Q | TqPIITTA*GPSDMYV | 0.45±0.03 | SEQ ID NO. 29 |
|---|---|---|---|
| P7F | TfPIITTA*GPSDMYV | 0.90±0.04 | SEQ ID NO. 30 |
| P7D | TdPIITTA*GPSDMYV | 0.22±0.04 | SEQ ID NO. 31 |
| P7T | TtPIITTA*GPSDMYV | 0.28±0.05 | SEQ ID NO. 32 |

------------------------------------------------------------------

| P6T | TRtIITTA*GPSDMYV | 0.25±0.09 | SEQ ID NO. 33 |
|---|---|---|---|
| P6K | TRkIITTA*GPSDMYV | 0.19±0.11 | SEQ ID NO. 34 |
| P6F | TRfIITTA*GPSDMYV | 0.71±0.05 | SEQ ID NO. 35 |
| P6D | TRdIITTA*GPSDMYV | 0.06±0.01 | SEQ ID NO. 36 |

------------------------------------------------------------------

| P5T | TRPtITTA*GPSDMYV | 0.49±0.09 | SEQ ID NO. 37 |
|---|---|---|---|
| P5K | TRPkITTA*GPSDMYV | 0.95±0.07 | SEQ ID NO. 38 |
| P5D | TRPdITTA*GPSDMYV | 0.04±0.01 | SEQ ID NO. 39 |
| P5F | TRPfITTA*GPSDMYV | 0.61±0.08 | SEQ ID NO. 40 |

------------------------------------------------------------------

| P4K | TRPIkTTA*GPSDMYV | keine Spaltung | SEQ ID NO. 41 |
|---|---|---|---|
| P4S | TRPIsTTA*GPSDMYV | 0.06±0.01 | SEQ ID NO. 42 |
| P4D | TRPIdTTA*GPSDMYV | keine Spaltung | SEQ ID NO. 43 |
| P4F | TRPIfTTA*GPSDMYV | 0.15±0.03 | SEQ ID NO. 44 |

------------------------------------------------------------------

| P3F | TRPIIfTA*GPSDMYV | 0.63±0.02 | SEQ ID NO. 45 |
|---|---|---|---|
| P3S | TRPIIsTA*GPSDMYV | 0.24±0.03 | SEQ ID NO. 46 |
| P3D | TRPIIdTA*GPSDMYV | 0.11±0.02 | SEQ ID NO. 47 |
| P3K | TRPIIkTA*GPSDMYV | 1.03±0.08 | SEQ ID NO. 48 |

# Fig. 23B

| P2A | TRPIITaA*GPSDMYV | keine Spaltung | SEQ ID NO. 49 |
|-----|------------------|----------------|---------------|
| P2V | TRPIITvA*GPSDMYV | keine Spaltung | SEQ ID NO. 50 |
| P2L | TRPIITlA*GPSDMYV | keine Spaltung | SEQ ID NO. 51 |
| P2I | TRPIITia*GPSDMYV | keine Spaltung | SEQ ID NO. 52 |
| P2P | TRPIITpA*GPSDMYV | 0.06±0.005 | SEQ ID NO. 53 |
| P2F | TRPIITfA*GPSDMYV | keine Spaltung | SEQ ID NO. 54 |
| P2G | TRPIITgA*GPSDMYV | keine Spaltung | SEQ ID NO. 55 |
| P2S | TRPIITsA*GPSDMYV | 0.15±0.03 | SEQ ID NO. 56 |
| P2Y | TRPIITyA*GPSDMYV | keine Spaltung | SEQ ID NO. 57 |
| P2N | TRPIITnA*GPSDMYV | 0.10±0.01 | SEQ ID NO. 58 |
| P2Q | TRPIITqA*GPSDMYV | 0.03±0.005 | SEQ ID NO. 59 |
| P2D | TRPIITdA*GPSDMYV | keine Spaltung | SEQ ID NO. 60 |
| P2E | TRPIITeA*GPSDMYV | keine Spaltung | SEQ ID NO. 61 |
| P2K | TRPIITkA*GPSDMYV | 0.05±0.01 | SEQ ID NO. 62 |
| P2R | TRPIITrA*GPSDMYV | 0.16±0.03 | SEQ ID NO. 63 |
| P2H | TRPIIThA*GPSDMYV | 0.04±0.005 | SEQ ID NO. 64 |
| P2M | TRPIITmA*GPSDMYV | 0.09±0.01 | SEQ ID NO. 65 |
| P2C | TRPIITcA*GPSDMYV | <0.01 | SEQ ID NO. 66 |

---

| P1V | TRPIITTv*GPSDMYV | 0.03±0.005 | SEQ ID NO. 67 |
|-----|------------------|------------|---------------|
| P1L | TRPIITTl*GPSDMYV | 0.38±0.07 | SEQ ID NO. 68 |
| P1P | TRPIITTp*GPSDMYV | keine Spaltung | SEQ ID NO. 69 |
| P1F | TRPIITTf*GPSDMYV | 0.79±0.04 | SEQ ID NO. 70 |
| P1M | TRPIITTm*GPSDMYV | 5.10±0.90 | SEQ ID NO. 71 |
| P1T | TRPIITTt*GPSDMYV | 0.68±0.05 | SEQ ID NO. 72 |
| P1Y | TRPIITTy*GPSDMYV | 1.38±0.06 | SEQ ID NO. 73 |
| P1N | TRPIITTn*GPSDMYV | 0.10±0.02 | SEQ ID NO. 74 |
| P1Q | TRPIITTq*GPSDMYV | 0.11±0.01 | SEQ ID NO. 75 |
| P1D | TRPIITTd*GPSDMYV | keine Spaltung | SEQ ID NO. 76 |
| P1E | TRPIITTe*GPSDMYV | keine Spaltung | SEQ ID NO. 77 |
| P1R | TRPIITTr*GPSDMYV | 0.56±0.06 | SEQ ID NO. 78 |
| P1K | TRPIITTk*GPSDMYV | 0.12±0.03# | SEQ ID NO. 79 |
| P1S | TRPIITTs*GPSDMYV | 0.12±0.03# | SEQ ID NO. 80 |
| P1H | TRPIITTh*GPSDMYV | 0.34±0.06 | SEQ ID NO. 81 |
| P1C | TRPIITTc*GPSDMYV | 1.83±0.22 | SEQ ID NO. 82 |
| P1G | TRPIITTg*GPSDMYV | <0.01 | SEQ ID NO. 83 |

---

# Fig. 23C

```
P1'F        TRPIITTA*fPSDMYV    keine Spaltung SEQ ID NO. 84
P1'T        TRPIITTA*tPSDMYV    keine Spaltung SEQ ID NO. 85
P1'D        TRPIITTA*dPSDMYV    keine Spaltung SEQ ID NO. 86
P1'K        TRPIITTA*kPSDMYV    keine Spaltung SEQ ID NO. 87
P1'L        TRPIITTA*lPSDMYV    keine Spaltung SEQ ID NO. 88
P1'Q        TRPIITTA*qPSDMYV    keine Spaltung SEQ ID NO. 89
P1'C        TRPIITTA*cPSDMYV    keine Spaltung SEQ ID NO. 90
P1'R        TRPIITTA*rPSDMYV    keine Spaltung SEQ ID NO. 91
P1'P        TRPIITTA*pPSDMYV    keine Spaltung SEQ ID NO. 92
P1'Y        TRPIITTA*yPSDMYV    keine Spaltung SEQ ID NO. 93
P1'E        TRPIITTA*ePSDMYV    keine Spaltung SEQ ID NO. 94
P1'I        TRPIITTA*iPSDMYV    keine Spaltung SEQ ID NO. 95
P1'N        TRPIITTA*nPSDMYV    keine Spaltung SEQ ID NO. 96
P1'A        TRPIITTA*aPSDMYV    keine Spaltung SEQ ID NO. 97
P1'S        TRPIITTA*sPSDMYV    keine Spaltung SEQ ID NO. 98
P1'V        TRPIITTA*vPSDMYV    keine Spaltung SEQ ID NO. 99
P1'M        TRPIITTA*mPSDMYV    keine Spaltung SEQ ID NO. 100
P1'H        TRPIITTA*hPSDMYV    keine Spaltung SEQ ID NO. 101
------------------------------------------------------------
P2'F        TRPIITTA*GfSDMYV    0.28±0.06      SEQ ID NO. 102
P2'T        TRPIITTA*GtSDMYV    0.37±0.04      SEQ ID NO. 103
P2'D        TRPIITTA*GdSDMYV    0.22±0.04      SEQ ID NO. 104
P2'K        TRPIITTA*GkSDMYV    0.43±0.08      SEQ ID NO. 105
------------------------------------------------------------
P3'T        TRPIITTA*GPtDMYV    0.74±0.05      SEQ ID NO. 106
P3'D        TRPIITTA*GPdDMYV    0.23±0.03      SEQ ID NO. 107
P3'K        TRPIITTA*GPkDMYV    0.76±0.06      SEQ ID NO. 108
P3'F        TRPIITTA*GPfDMYV    0.39±0.07      SEQ ID NO. 109
------------------------------------------------------------
P4'K        TRPIITTA*GPSkMYV    2.22±0.43      SEQ ID NO. 110
P4'F        TRPIITTA*GPSfMYV    1.99±0.23      SEQ ID NO. 111
P4'T        TRPIITTA*GPStMYV    1.36±0.19      SEQ ID NO. 112
P4'E        TRPIITTA*GPSeMYV    0.68±0.08      SEQ ID NO. 113
------------------------------------------------------------
```

# Fig. 23D

| P5'T | TRPIITTA*GPSDtYV | 0.61±0.09 | SEQ ID NO. 114 |
|------|------------------|-----------|-----------------|
| P5'F | TRPIITTA*GPSDfYV | 0.65±0.08 | SEQ ID NO. 115 |
| P5'K | TRPIITTA*GPSDkYV | 1.05±0.13 | SEQ ID NO. 116 |
| P5'D | TRPIITTA*GPSDdYV | 0.39±0.07 | SEQ ID NO. 117 |

------------------------------------------------------------

| P6'D | TRPIITTA*GPSDMdV | 0.32±0.06 | SEQ ID NO. 118 |
|------|------------------|-----------|-----------------|
| P6'K | TRPIITTA*GPSDMkV | 0.95±0.18 | SEQ ID NO. 119 |
| P6'F | TRPIITTA*GPSDMfV | 0.59±0.09 | SEQ ID NO. 120 |
| P6'T | TRPIITTA*GPSDMtV | 0.66±0.09 | SEQ ID NO. 121 |

============================================================

b)

| p220-1 | TRlqginc*GPdftpsf | keine Spaltung | SEQ ID NO. 122 |
|--------|-------------------|----------------|-----------------|
| p220-2 | elargaqA*Glgprrsq | keine Spaltung | SEQ ID NO. 123 |
| p220-3 | lcrllTTi*Gkdldfek | 0.11±0.02 | SEQ ID NO. 124 |
| p220-4 | gRttlsTr*GPprggpg | 0.12±0.02 | SEQ ID NO. 125 |
| p220-5 | gssggsgA*qPSDaase | keine Spaltung | SEQ ID NO. 126 |

============================================================

c)

| MMP-1 | eRfaIkTm*GPkfhqsg | 9.93±1.58 | SEQ ID NO. 127 |
|-------|------------------|-----------|-----------------|
| MMP-2 | efPaIkTm*GPkfhqsg | 5.67±0.93 | SEQ ID NO. 128 |

============================================================

d)

| RCC | TRPIITTA*GPyghqsg | 0.30±0.05 | SEQ ID NO. 129 |
|-----|-------------------|-----------|-----------------|
| A | eRasliTt*GPSDMYVH | 0.23±0.03 | SEQ ID NO. 130 |
| B | eRPslTTA*GPSDMYVH | 0.82±0.13 | SEQ ID NO. 131 |
| C | eRasliTv*GPSDMYVH | 0.06±0.02# | SEQ ID NO. 132 |
| CoxMut | eRasliTt*GPygMqsg | 0.23±0.04 | SEQ ID NO. 133 |
| Coxwt | eRasliTt*GPyghqsg | 0.10±0.01 | SEQ ID NO. 134 |

============================================================

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | WO-A-9 001 061 (BOEHRINGER INGELHEIM GMBH) <br> * das ganze Dokument * <br> --- | 14-16 | C12Q1/70 <br> C07K15/00 <br> C12N9/50 |
| D,A | VIROLOGY <br> Bd. 169, 1989, <br> Seiten 68 - 77 <br> W. SOMMERGRUBER ET AL. 'Polypeptide 2A of human rhinovirus type 2: identification as a protease and characterization by mutational analysis' <br> * das ganze Dokument * <br> --- | 14-17, 19-23 | |
| D,A | VIROLOGY <br> Bd. 181, 1991, <br> Seiten 46 - 54 <br> T. SKERN ET AL. 'SUBSTRATE REQUIREMENTS OF A HUMAN RHINOVIRAL 2A PROTEINASE' <br> --- | - | |
| D,A | PROCEEDINGS OF THE NATIONAL ACADEMY OF SCIENCES OF USA <br> Bd. 87, Dezember 1990, WASHINGTON US <br> Seiten 9529 - 9533 <br> E. E. WYCKOFF ET AL. 'Eukaryotic initiation factor 3 is requiered for poliovirus 2A protease induced cleavageof the p220 component of eukaryotic initiation factor 4F' <br> --- | - | **RECHERCHIERTE SACHGEBIETE (Int. Cl.5 )** <br><br> C12Q <br> C07K <br> C12N |
| D,A | NATURE <br> Bd. 334, Juli 1987, LONDON GB <br> Seiten 320 - 325 <br> J. PELLETIER ET AL. 'Internal initiation of translation of eukaryotic mRNA directed by a sequence derived from poliovirus RNA' <br><br> ----- | - | |

Der vorliegende Recherchenbericht wurde für alle Patentansprüche erstellt

| Recherchenort | Abschlußdatum der Recherche | Prüfer |
|---|---|---|
| DEN HAAG | 16 JUNI 1993 | MOLINA GALAN E. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

EPO FORM 1503 03.82 (P0403)